# FASCICULE DE BREVET EUROPEEN

(11) **EP 0 877 758 B1**
(45) Date de publication et mention de la délivrance du brevet: **08.04.2009**
(21) Numéro de dépôt: 97904481.5
(22) Date de dépôt: 03.02.1997
(51) Int. Cl.: C07K 14/47, C12N 15/12, C12Q 1/68, A61K 39/395, G01N 33/68

(54) **PROTEINE PURIFIEE SR-p70**
GEREINIGTES SR-P70 PROTEIN
PURIFIED SR-p70 PROTEIN

(30) Priorité: 02.02.1996 FR 9601309
(43) Date de publication de la demande: 18.11.1998
(73) Titulaire: Sanofi-Aventis, 75013 Paris (FR)
(72) Inventeur: CAPUT, Daniel, F-31290 Avignonet-Lauragais (FR); FERRARA, Pascual, F-31290 Avignonet-Lauragais (FR); KAGHAD, Ahmed, Mourad, F-31450 Montgiscard (FR)
(74) Mandataire: Bernasconi, Jean Raymond
(86) Numéro de dépôt international: PCT/FR1997/000214
(87) Numéro de publication internationale: WO 1997/028186

(56) Documents cités:
- EP-A- 0 377 295
- WO-A-94/01563
- WO-A-94/08241
- FR-A- 2 692 594
- SCIENCE, vol. 237, 1987, pages 1620-1624, XP000604718 BODRUG: "Molecular analysis of a constitutional X-autosome translocation in a female with muscular dystrophy" & DATABASE STRAND ref. EMHUM hsrtmd1, AN: L08092 6 Avril 1993 & J. MOL. BIOL., vol. 232, no. 1, 1993, pages 314-321, XP000604618 MC NAUGHTON ET AL.: "A cluster of transposon-like repetitive sequences in intron 7 of the human dystrophin gene"
- NUCLEIC ACID RESEARCH, vol. 16, no. 23, 1988, page 1183 XP002014924 SOUSSI ET AL.: "Nucleotide sequence of a cDNA encoding the chicken p53 nuclear protein" & DATABASE STRAND ref. Swissprot: P53-chick : AN : P10360 1 Mars 1989
- BIOCHEM. BIOPHYS. RES. COMMUN., vol. 194, no. 2, 30 Juillet 1993, pages 698-705, XP002014925 IWASE ET AL.: "Identification of protein-tyrosine kinase genes preferentially expressed in embryo stomach and gastric cancer" & DATABASE STRAND ref. EMHUM1: Hserk1p, AN: D37827 16 Août 1994
- DATABASE STRAND ref. EN960713, AN: Z75711 XP002014926 & NATURE, vol. 368, 1994, pages 32-38, WILSON ET AL.: "2.2. Mb of contiguous nucleotide sequence from chromosome III of C. elegans"
- MOL. CELL. BIOL., vol. 6, no. 9, Septembre 1986, pages 3232-3239, XP000604639 ARAI ET AL.: "Immunologically distinct p53 molecules generated by alternative splicing" & DATABASE STRAND ref. Pir2: S38822 13 Janvier 1995
- NATURE GENETICS, vol. 6, no. 4, Avril 1994, pages 357-362, XP000604628 NEUMANN ET AL.: "Multifactorial inheritance of neural tube defects : localization of the major gene and recognition of modifiers in ct mutant genes"
- DATABASE EMBL ID: CEF26F12, AC= U55373, XP002032930 & NATURE, vol. 368, 1994, pages 32-38, WILSON ET AL.: "2.2 Mb of contiguous nucleotide sequence from chromosome III of C. elegans"
- DATABASE EMBL ID=AC= S77819, 29 Septembre 1995 XP002032931 & CANCER LETTERS, vol. 92, no. 2, 8 Juin 1995, pages 181-186, XP000674693 KRAEGEL ET AL.: "Sequence analysis of canine p53 in the region of exons 3-8"
- DATABASE EMBL ID: SIP53, AC=M75145, 24 Août 1991 XP002032932 & GENE, vol. 112, 1992, pages 241-245, DE FROMENTEL ET AL.: "Rainbow trout p53: cDNA cloning and biochemical characterization"
- DATABASE EMBL ID: BTP53, AC= X81704, 14 Décembre 1994 XP002032933 & DNA SEQ., vol. 5, no. 4, 1995, pages 261-264, XP000674685 DESQUIEDT ET AL.: "Nucleotide sequence of bovine p53 tumor-suppressor cDNA"
- NUCLEIC ACIDS RES., vol. 20, no. 8, 1992, pages 1879-1882, XP002032929 GRYAZNOV ET AL.: "Selective O-phosphitilation with nucleoside phosphoramidite reagents"
- INT. J. RADIAT. BIOL. RELAT. STUD. PHYS., CHEM. MED., vol. 51, no. 3, 1987, pages 429-439, XP000674638 TEOULE ET AL.: "Gamma-irradiation of homodeoxyoligonucleotides 32P-labelled at one end : computer simulation of the chain length distribution of the radioactive fragments"

### LISTE DE SEQUENCES

(1) INFORMATION GENERALE:
   (i) DEPOSANT:
      (A) NOM: sanofi
      (B) RUE: 32-34 rue Marbeuf
      (C) VILLE: PARIS
      (E) PAYS: FRANCE
      (F) CODE POSTAL: 75008
      (G) TELEPHONE: 01 53 77 40 00
      (H) TELECOPIE: 01 53 77 41 33
   (ii) TITRE DE L' INVENTION: SR-p70
   (iii) NOMBRE DE SEQUENCES: 40
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 2874 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (A) ORGANISME: Cebus apella
   (ix) CARACTERISTIQUE ADDITIONELLE:
      (A) NOM/CLE: CDS
      (B) EMPLACEMENT: 156..2066
   (xi) DESCRIPTION DE LA SEQUENCE: SEQ ID NO: 1:
   TGCCTCCCCG CCCGCGCACC CGCCCCGAGG CCTGTGCTCC TGCGAAGGGG ACGCAGCGAA      60
   GCCGGGGCCC GCGCCAGGCC GGCCGGGACG GACGCCGATG CCCGGAGCTG CGACGGCTGC     120
   AGAGCGAGCT GCCCTCGGAG GCCGGTGTGA GGAAG ATG GCC CAG TCC ACC ACC        173
                                          Met Ala Gln Ser Thr Thr
                                           1               5
   ACC TCC CCC GAT GGG GGC ACC ACG TTT GAG CAC CTC TGG AGC TCT CTG       221
   Thr Ser Pro Asp Gly Gly Thr Thr Phe Glu His Leu Trp Ser Ser Leu
               10                  15                  20
   GAA CCA GAC AGC ACC TAC TTC GAC CTT CCC CAG TCA AGC CGG GGG AAT       269
   Glu Pro Asp Ser Thr Tyr Phe Asp Leu Pro Gln Ser Ser Arg Gly Asn
           25                  30                  35
   AAT GAG GTG GTG GGT GGC ACG GAT TCC AGC ATG GAC GTC TTC CAC CTA       317
   Asn Glu Val Val Gly Gly Thr Asp Ser Ser Met Asp Val Phe His Leu
       40                  45                  50
   GAG GGC ATG ACC ACA TCT GTC ATG GCC CAG TTC AAT TTG CTG AGC AGC       365
   Glu Gly Met Thr Thr Ser Val Met Ala Gln Phe Asn Leu Leu Ser Ser
   55                  60                  65                  70
   ACC ATG GAC CAG ATG AGC AGC CGC GCT GCC TCG GCC AGC CCG TAC ACC       413
   Thr Met Asp Gln Met Ser Ser Arg Ala Ala Ser Ala Ser Pro Tyr Thr
                   75                  80                  85
   CCG GAG CAC GCC GCC AGC GTG CCC ACC CAT TCA CCC TAC GCA CAG CCC      461
   Pro Glu His Ala Ala Ser Val Pro Thr His Ser Pro Tyr Ala Gln Pro
                    90                  95                 100
   AGC TCC ACC TTC GAC ACC ATG TCG CCC GCG CCT GTC ATC CCC TCC AAC      509
   Ser Ser Thr Phe Asp Thr Met Ser Pro Ala Pro Val Ile Pro Ser Asn
           105                 110                 115
   ACC GAC TAT CCC GGA CCC CAC CAC TTC GAG GTC ACT TTC CAG CAG TCC      557
   Thr Asp Tyr Pro Gly Pro His His Phe Glu Val Thr Phe Gln Gln Ser
       120                 125                 130
   AGC ACG GCC AAG TCA GCC ACC TGG ACG TAC TCC CCA CTC TTG AAG AAA      605
   Ser Thr Ala Lys Ser Ala Thr Trp Thr Tyr Ser Pro Leu Leu Lys Lys
   135                 140                 145                 150
   CTC TAC TGC CAG ATC GCC AAG ACA TGC CCC ATC CAG ATC AAG GTG TCC      653
   Leu Tyr Cys Gln Ile Ala Lys Thr Cys Pro Ile Gln Ile Lys Val Ser
                   155                 160                 165
   GCC CCA CCG CCC CCG GGC ACC GCC ATC CGG GCC ATG CCT GTC TAC AAG      701
   Ala Pro Pro Pro Pro Gly Thr Ala Ile Arg Ala Met Pro Val Tyr Lys
               170                 175                 180
   AAG GCG GAG CAC GTG ACC GAC ATC GTG AAG CGC TGC CCC AAC CAC GAG      749
   Lys Ala Glu His Val Thr Asp Ile Val Lys Arg Cys Pro Asn His Glu
           185                 190                 195
   CTC GGG AGG GAC TTC AAC GAA GGA CAG TCT GCC CCA GCC AGC CAC CTC      797
   Leu Gly Arg Asp Phe Asn Glu Gly Gln Ser Ala Pro Ala Ser His Leu
       200                 205                 210
   ATC CGT GTG GAA GGC AAT AAT CTC TCG CAG TAT GTG GAC GAC CCT GTC      845
   Ile Arg Val Glu Gly Asn Asn Leu Ser Gln Tyr Val Asp Asp Pro Val
   215                 220                 225                 230
   ACC GGC AGG CAG AGC GTC GTG GTG CCC TAT GAG CCA CCA CAG GTG GGG      893
   Thr Gly Arg Gln Ser Val Val Val Pro Tyr Glu Pro Pro Gln Val Gly
                   235                 240                 245
   ACA GAA TTC ACC ACC ATC CTG TAC AAC TTC ATG TGT AAC AGC AGC TGT      941
   Thr Glu Phe Thr Thr Ile Leu Tyr Asn Phe Met Cys Asn Ser Ser Cys
               250                 255                 260
   GTG GGG GGC ATG AAC CGA CGG CCC ATC CTC ATC ATC ATC ACC CTG GAG      989
   Val Gly Gly Met Asn Arg Arg Pro Ile Leu Ile Ile Ile Thr Leu Glu
           265                 270                 275
   ACG CGG GAT GGG CAG GTG CTG GGC CGC CGG TCC TTC GAG GGC CGC ATC     1037
   Thr Arg Asp Gly Gln Val Leu Gly Arg Arg Ser Phe Glu Gly Arg Ile
       280                 285                 290
   TGC GCC TGT CCT GGC CGC GAC CGA AAA GCC GAT GAG GAC CAC TAC CGG     1085
   Cys Ala Cys Pro Gly Arg Asp Arg Lys Ala Asp Glu Asp His Tyr Arg
   295                 300                 305                 310
   GAG CAG CAG GCC TTG AAT GAG AGC TCC GCC AAG AAC GGG GCT GCC AGC     1133
   Glu Gln Gln Ala Leu Asn Glu Ser Ser Ala Lys Asn Gly Ala Ala Ser
                   315                 320                 325
   AAG CGC GCC TTC AAG CAG AGT CCC CCT GCC GTC CCC GCC CTG GGC CCG     1181
   Lys Arg Ala Phe Lys Gln Ser Pro Pro Ala Val Pro Ala Leu Gly Pro
               330                 335                 340
   GGT GTG AAG AAG CGG CGG CAC GGA GAC GAG GAC ACG TAC TAC CTG CAG     1229
   Gly Val Lys Lys Arg Arg His Gly Asp Glu Asp Thr Tyr Tyr Leu Gln
           345                 350                 355
   GTG CGA GGC CGC GAG AAC TTC GAG ATC CTG ATG AAG CTG AAG GAG AGC     1277
   Val Arg Gly Arg Glu Asn Phe Glu Ile Leu Met Lys Leu Lys Glu Ser
       360                 365                 370
   CTG GAG CTG ATG GAG TTG GTG CCG CAG CCG CTG GTA GAC TCC TAT CGG        1325
   Leu Glu Leu Met Glu Leu Val Pro Gln Pro Leu Val Asp Ser Tyr Arg
   375                 380                 385                 390
   CAG CAG CAG CAG CTC CTA CAG AGG CCG AGT CAC CTA CAG CCC CCA TCC        1373
   Gln Gln Gln Gln Leu Leu Gln Arg Pro Ser His Leu Gln Pro Pro Ser
                   395                 400                 405
   TAC GGG CCG GTC CTC TCG CCC ATG AAC AAG GTG CAC GGG GGC GTG AAC        1421
   Tyr Gly Pro Val Leu Ser Pro Met Asn Lys Val His Gly Gly Val Asn
               410                 415                 420
   AAG CTG CCC TCC GTC AAC CAG CTG GTG GGC CAG CCT CCC CCG CAC AGC        1469
   Lys Leu Pro Ser Val Asn Gln Leu Val Gly Gln Pro Pro Pro His Ser
           425                 430                 435
   TCG GCA GCT ACA CCC AAC CTG GGA CCT GTG GGC TCT GGG ATG CTC AAC        1517
   Ser Ala Ala Thr Pro Asn Leu Gly Pro Val Gly Ser Gly Met Leu Asn
       440                 445                 450
   AAC CAC GGC CAC GCA GTG CCA GCC AAC AGC GAG ATG ACC AGC AGC CAC        1565
   Asn His Gly His Ala Val Pro Ala Asn Ser Glu Met Thr Ser Ser His
   455                 460                 465                 470
   GGC ACC CAG TCC ATG GTC TCG GGG TCC CAC TGC ACT CCG CCA CCC CCC        1613
   Gly Thr Gln Ser Met Val Ser Gly Ser His Cys Thr Pro Pro Pro Pro
                   475                 480                 485
   TAC CAC GCC GAC CCC AGC CTC GTC AGT TTT TTA ACA GGA TTG GGG TGT        1661
   Tyr His Ala Asp Pro Ser Leu Val Ser Phe Leu Thr Gly Leu Gly Cys
               490                 495                 500
   CCA AAC TGC ATC GAG TAT TTC ACG TCC CAG GGG TTA CAG AGC ATT TAC        1709
   Pro Asn Cys Ile Glu Tyr Phe Thr Ser Gln Gly Leu Gln Ser Ile Tyr
           505                 510                 515
   CAC CTG CAG AAC CTG ACC ATC GAG GAC CTG GGG GCC CTG AAG ATC CCC        1757
   His Leu Gln Asn Leu Thr Ile Glu Asp Leu Gly Ala Leu Lys Ile Pro
       520                 525                 530
   GAG CAG TAT CGC ATG ACC ATC TGG CGG GGC CTG CAG GAC CTG AAG CAG        1805
   Glu Gln Tyr Arg Met Thr Ile Trp Arg Gly Leu Gln Asp Leu Lys Gln
   535                 540                 545                 550
   GGC CAC GAC TAC GGC GCC GCC GCG CAG CAG CTG CTC CGC TCC AGC AAC        1853
   Gly His Asp Tyr Gly Ala Ala Ala Gln Gln Leu Leu Arg Ser Ser Asn
                   555                 560                 565
   GCG GCC GCC ATT TCC ATC GGC GGC TCC GGG GAG CTG CAG CGC CAG CGG        1901
   Ala Ala Ala Ile Ser Ile Gly Gly Ser Gly Glu Leu Gln Arg Gln Arg
               570                 575                 580
   GTC ATG GAG GCC GTG CAC TTC CGC GTG CGC CAC ACC ATC ACC ATC CCC        1949
   Val Met Glu Ala Val His Phe Arg Val Arg His Thr Ile Thr Ile Pro
           585                 590                 595
   AAC CGC GGC GGC CCC GGC GCC GGC CCC GAC GAG TGG GCG GAC TTC GGC        1997
   Asn Arg Gly Gly Pro Gly Ala Gly Pro Asp Glu Trp Ala Asp Phe Gly
       600                 605                 610
   TTC GAC CTG CCC GAC TGC AAG GCC CGC AAG CAG CCC ATC AAG GAG GAG        2045
   Phe Asp Leu Pro Asp Cys Lys Ala Arg Lys Gln Pro Ile Lys Glu Glu
   615                 620                 625                 630
   TTC ACG GAG GCC GAG ATC CAC TGAGGGGCCG GGCCCAGCCA GAGCCTGTGC           2096
   Phe Thr Glu Ala Glu Ile His
                   635
   CACCGCCCAG AGACCCAGGC CGCCTCGCTC TCCTTCCTGT GTCCAAAACT GCCTCCGGAG      2156
   GCAGGGCCTC CAGGCTGTGC CCGGGGAAAG GCAAGGTCCG GCCCATGCCC CGGCACCTCA      2216
   CCGGCCCCAG GAGAGGCCCA GCCACCAAAG CCGCCTGCGG ACAGCCTGAG TCACCTGCAG    2276
   AACCTTCTGG AGCTGCCCTA ATGCTGGGCT TGCGGGGCAG GGGCCGGCCC ACTCTCAGCC    2336
   CTGCCACTGC CGGGCGTGCT CCATGGCAGG CGTGGGTGGG GACCGCAGTG TCAGCTCCGA    2396
   CCTCCAGGCC TCATCCTAGA GACTCTGTCA TCTGCCGATC AAGCAAGGTC CTTCCAGAGG    2456
   AAAGAATCCT CTTCGCTGGT GGACTGCCAA AAAGTATTTT GCGACATCTT TTGGTTCTGG    2516
   AGAGTGGTGA GCAGCCAAGC GACTGTGTCT GAAACACCGT GCATTTTCAG GGAATGTCCC    2576
   TAACGGGCTG GGGACTCTCT CTGCTGGACT TGGGAGTGGC CTTTGCCCCC AGCACACTGT    2636
   ATTCTGCGGG ACCGCCTCCT TCCTGCCCCT AACAACCACC AAAGTGTTGC TGAAATTGGA    2696
   GAAAACTGGG GAAGGCGCAA CCCCTCCCAG GTGCGGGAAG CATCTGGTAC CGCCTCGGCC    2756
   AGTGCCCCTC AGCCTGGCCA CAGTCACCTC TCCTTGGGGA ACCCTGGGCA GAAAGGGACA    2816
   GCCTGTCCTT AGAGGACCGG AAATTGTCAA TATTTGATAA AATGATACCC TTTTCTAC      2874
(2) INFORMATION POUR LA SEQ ID MO: 2:
   (i) CARACTERISTIQUES DE LA SEQUENCE:
      (A) LONGUEUR: 637 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
(2) INFORMATION POUR LA SEQ ID NO: 3:
   (i) CARACTERISTIQUES DE LA SEQUENCE:
      (A) LONGUEUR: 2034 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (A) ORGANISME: Cebus apella
   (ix) CARACTERISTIQUE ADDITIONELLE:
      (A) NOM/CLE: CDS
      (B) EMPLACEMENT: 156..1652
   (xi) DESCRIPTION DE LA SEQUENCE: SEQ ID NO: 3:
   TGCCTCCCCG CCCGCGCACC CGCCCCGAGG CCTGTGCTCC TGCGAAGGGG ACGCAGCGAA    60
   GCCGGGGCCC GCGCCAGGCC GGCCGGGACG GACGCCGATG CCCGGAGCTG CGACGGCTGC   120
   AGAGCGAGCT GCCCTCGGAG GCCGGTGTGA GGAAG ATG GCC CAG TCC ACC ACC      173
                                          Met Ala Gln Ser Thr Thr
                                           1               5
   ACC TCC CCC GAT GGG GGC ACC ACG TTT GAG CAC CTC TGG AGC TCT CTG     221
   Thr Ser Pro Asp Gly Gly Thr Thr Phe Glu His Leu Trp Ser Ser Leu
                   10                  15                  20
   GAA CCA GAC AGC ACC TAC TTC GAC CTT CCC CAG TCA AGC CGG GGG AAT     269
   Glu Pro Asp Ser Thr Tyr Phe Asp Leu Pro Gln Ser Ser Arg Gly Asn
               25                  30                  35
   AAT GAG GTG GTG GGT GGC ACG GAT TCC AGC ATG GAC GTC TTC CAC CTA     317
   Asn Glu Val Val Gly Gly Thr Asp Ser Ser Met Asp Val Phe His Leu
           40                  45                  50
   GAG GGC ATG ACC ACA TCT GTC ATG GCC CAG TTC AAT TTG CTG AGC AGC     365
   Glu Gly Met Thr Thr Ser Val Met Ala Gln Phe Asn Leu Leu Ser Ser
    55                  60                  65                  70
   ACC ATG GAC CAG ATG AGC AGC CGC GCT GCC TCG GCC AGC CCG TAC ACC     413
   Thr Met Asp Gln Met Ser Ser Arg Ala Ala Ser Ala Ser Pro Tyr Thr
                    75                  80                  85
   CCG GAG CAC GCC GCC AGC GTG CCC ACC CAT TCA CCC TAC GCA CAG CCC     461
   Pro Glu His Ala Ala Ser Val Pro Thr His Ser Pro Tyr Ala Gln Pro
                   90                  95                  100
   AGC TCC ACC TTC GAC ACC ATG TCG CCC GCG CCT GTC ATC CCC TCC AAC     509
   Ser Ser Thr Phe Asp Thr Met Ser Pro Ala Pro Val Ile Pro Ser Asn
               105                 110                 115
   ACC GAC TAT CCC GGA CCC CAC CAC TTC GAG GTC ACT TTC CAG CAG TCC     557
   Thr Asp Tyr Pro Gly Pro His His Phe Glu Val Thr Phe Gln Gln Ser
           120                 125                 130
   AGC ACG GCC AAG TCA GCC ACC TGG ACG TAC TCC CCA CTC TTG AAG AAA     605
   Ser Thr Ala Lys Ser Ala Thr Trp Thr Tyr Ser Pro Leu Leu Lys Lys
   135                 140                 145                 150
   CTC TAC TGC CAG ATC GCC AAG ACA TGC CCC ATC CAG ATC AAG GTG TCC     653
   Leu Tyr Cys Gln Ile Ala Lys Thr Cys Pro Ile Gln Ile Lys Val Ser
                   155                 160                 165
   GCC CCA CCG CCC CCG GGC ACC GCC ATC CGG GCC ATG CCT GTC TAC AAG     701
   Ala Pro Pro Pro Pro Gly Thr Ala Ile Arg Ala Met Pro Val Tyr Lys
               170                 175                 180
   AAG GCG GAG CAC GTG ACC GAC ATC GTG AAG CGC TGC CCC AAC CAC GAG     749
   Lys Ala Glu His Val Thr Asp Ile Val Lys Arg Cys Pro Asn His Glu
           185                 190                 195
   CTC GGG AGG GAC TTC AAC GAA GGA CAG TCT GCC CCA GCC AGC CAC CTC     797
   Leu Gly Arg Asp Phe Asn Glu Gly Gln Ser Ala Pro Ala Ser His Leu
       200                 205                 210
   ATC CGT GTG GAA GGC AAT AAT CTC TCG CAG TAT GTG GAC GAC CCT GTC     845
   Ile Arg Val Glu Gly Asn Asn Leu Ser Gln Tyr Val Asp Asp Pro Val
   215                 220                 225                 230
   ACC GGC AGG CAG AGC GTC GTG GTG CCC TAT GAG CCA CCA CAG GTG GGG     893
   Thr Gly Arg Gln Ser Val Val Val Pro Tyr Glu Pro Pro Gln Val Gly
                   235                 240                 245
   ACA GAA TTC ACC ACC ATC CTG TAC AAC TTC ATG TGT AAC AGC AGC TGT     941
   Thr Glu Phe Thr Thr Ile Leu Tyr Asn Phe Met Cys Asn Ser Ser Cys
               250                 255                 260
   GTG GGG GGC ATG AAC CGA CGG CCC ATC CTC ATC ATC ATC ACC CTG GAG     989
   Val Gly Gly Met Asn Arg Arg Pro Ile Leu Ile Ile Ile Thr Leu Glu
           265                 270                 275
   ACG CGG GAT GGG CAG GTG CTG GGC CGC CGG TCC TTC GAG GGC CGC ATC     1037
   Thr Arg Asp Gly Gln Val Leu Gly Arg Arg Ser Phe Glu Gly Arg Ile
       280                 285                 290
   TGC GCC TGT CCT GGC CGC GAC CGA AAA GCC GAT GAG GAC CAC TAC CGG     1085
   Cys Ala Cys Pro Gly Arg Asp Arg Lys Ala Asp Glu Asp His Tyr Arg
   295                 300                 305                 310
   GAG CAG CAG GCC TTG AAT GAG AGC TCC GCC AAG AAC GGG GCT GCC AGC     1133
   Glu Gln Gln Ala Leu Asn Glu Ser Ser Ala Lys Asn Gly Ala Ala Ser
                   315                 320                 325
   AAG CGC GCC TTC AAG CAG AGT CCC CCT GCC GTC CCC GCC CTG GGC CCG     1181
   Lys Arg Ala Phe Lys Gln Ser Pro Pro Ala Val Pro Ala Leu Gly Pro
               330                 335                 340
   GGT GTG AAG AAG CGG CGG CAC GGA GAC GAG GAC ACG TAC TAC CTG CAG     1229
   Gly Val Lys Lys Arg Arg His Gly Asp Glu Asp Thr Tyr Tyr Leu Gln
           345                 350                 355
   GTG CGA GGC CGC GAG AAC TTC GAG ATC CTG ATG AAG CTG AAG GAG AGC     1277
   Val Arg Gly Arg Glu Asn Phe Glu Ile Leu Met Lys Leu Lys Glu Ser
       360                 365                 370
   CTG GAG CTG ATG GAG TTG GTG CCG CAG CCG CTG GTA GAC TCC TAT CGG     1325
   Leu Glu Leu Met Glu Leu Val Pro Gln Pro Leu Val Asp Ser Tyr Arg
   375                 380                 385                 390
   CAG CAG CAG CAG CTC CTA CAG AGG CCG AGT CAC CTA CAG CCC CCA TCC     1373
   Gln Gln Gln Gln Leu Leu Gln Arg Pro Ser His Leu Gln Pro Pro Ser
                   395                 400                 405
   TAC GGG CCG GTC CTC TCG CCC ATG AAC AAG GTG CAC GGG GGC GTG AAC     1421
   Tyr Gly Pro Val Leu Ser Pro Met Asn Lys Val His Gly Gly Val Asn
               410                 415                 420
   AAG CTG CCC TCC GTC AAC CAG CTG GTG GGC CAG CCT CCC CCG CAC AGC     1469
   Lys Leu Pro Ser Val Asn Gln Leu Val Gly Gln Pro Pro Pro His Ser
           425                 430                 435
   TCG GCA GCT ACA CCC AAC CTG GGA CCT GTG GGC TCT GGG ATG CTC AAC     1517
   Ser Ala Ala Thr Pro Asn Leu Gly Pro Val Gly Ser Gly Met Leu Asn
       440                 445                 450
   AAC CAC GGC CAC GCA GTG CCA GCC AAC AGC GAG ATG ACC AGC AGC CAC     1565
   Asn His Gly His Ala Val Pro Ala Asn Ser Glu Met Thr Ser Ser His
   455                 460                 465                 470
   GGC ACC CAG TCC ATG GTC TCG GGG TCC CAC TGC ACT CCG CCA CCC CCC     1613
   Gly Thr Gln Ser Met Val Ser Gly Ser His Cys Thr Pro Pro Pro Pro
                   475                 480                 485
   TAC CAC GCC GAC CCC AGC CTC GTC AGG ACC TGG GGC CCC TGAAGATCCC      1662
   Tyr His Ala Asp Pro Ser Leu Val Arg Thr Trp Gly Pro
               490                 495
   CGAGCAGTAT CGCATGACCA TCTGGCGGGG CCTGCAGGAC CTGAAGCAGG GCCACGACTA   1722
   CGGCGCCGCC GCGCAGCAGC TGCTCCGCTC CAGCAACGCG GCCGCCATTT CCATCGGCGG   1782
   CTCCGGGGAG CTGCAGCGCC AGCGGGTCAT GGAGGCCGTG CACTTCCGCG TGCGCCACAC   1842
   CATCACCATC CCCAACCGCG GCGGCCCCGG CGCCGGCCCC GACGAGTGGG CGGACTTCGG   1902
   CTTCGACCTG CCCGACTGCA AGGCCCGCAA GCAGCCCATC AAGGAGGAGT TCACGGAGGC   1962
   CGAGATCCAC TGAGGGGCCG GGCCCAGCCA GAGCCTGTGC CACCGCCCAG AGACCCAGGC   2022
   CGCCTCGCTC TC                                                       2034
(2) INFORMATION POUR LA SEQ ID NO: 4:
   (i) CARACTERISTIQUES DE LA SEQUENCE:
      (A) LONGUEUR: 499 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
   Met Ala Gln Ser Thr Thr Thr Ser Pro Asp Gly Gly Thr Thr Phe Glu
    1               5                  10                  15
   His Leu Trp Ser Ser Leu Glu Pro Asp Ser Thr Tyr Phe Asp Leu Pro
               20                  25                  30
   Gln Ser Ser Arg Gly Asn Asn Glu Val Val Gly Gly Thr Asp Ser Ser
           35                  40                  45
   Met Asp Val Phe His Leu Glu Gly Met Thr Thr Ser Val Met Ala Gln
       50                  55                  60
   Phe Asn Leu Leu Ser Ser Thr Met Asp Gln Met Ser Ser Arg Ala Ala
   65                  70                  75                  80
   Ser Ala Ser Pro Tyr Thr Pro Glu His Ala Ala Ser Val Pro Thr His
                   85                  90                  95
   Ser Pro Tyr Ala Gln Pro Ser Ser Thr Phe Asp Thr Met Ser Pro Ala
               100                 105                 110
   Pro Val Ile Pro Ser Asn Thr Asp Tyr Pro Gly Pro His His Phe Glu
           115                 120                 125
   Val Thr Phe Gln Gln Ser Ser Thr Ala Lys Ser Ala Thr Trp Thr Tyr
       130                 135                 140
   Ser Pro Leu Leu Lys Lys Leu Tyr Cys Gln Ile Ala Lys Thr Cys Pro
   145 150 155 160
   Ile Gln Ile Lys Val Ser Ala Pro Pro Pro Pro Gly Thr Ala Ile Arg
   165 170 175
   Ala Met Pro Val Tyr Lys Lys Ala Glu His Val Thr Asp Ile Val Lys
   180 185 190
   Arg Cys Pro Asn His Glu Leu Gly Arg Asp Phe Asn Glu Gly Gln Ser
   195 200 205
   Ala Pro Ala Ser His Leu Ile Arg Val Glu Gly Asn Asn Leu Ser Gln
   210 215 220
   Tyr Val Asp Asp Pro Val Thr Gly Arg Gln Ser Val Val Val Pro Tyr
   225 230 235 240
   Glu Pro Pro Gln Val Gly Thr Glu Phe Thr Thr Ile Leu Tyr Asn Phe
   245 250 255
   Met Cys Asn Ser Ser Cys Val Gly Gly Met Asn Arg Arg Pro Ile Leu
   260 265 270
   Ile Ile Ile Thr Leu Glu Thr Arg Asp Gly Gln Val Leu Gly Arg Arg
   275 280 285
   Ser Phe Glu Gly Arg Ile Cys Ala Cys Pro Gly Arg Asp Arg Lys Ala
   290 295 300
   Asp Glu Asp His Tyr Arg Glu Gln Gln Ala Leu Asn Glu Ser Ser Ala
   305 310 315 320
   Lys Asn Gly Ala Ala Ser Lys Arg Ala Phe Lys Gln Ser Pro Pro Ala
   325 330 335
   Val Pro Ala Leu Gly Pro Gly Val Lys Lys Arg Arg His Gly Asp Glu
   340 345 350
   Asp Thr Tyr Tyr Leu Gln Val Arg Gly Arg Glu Asn Phe Glu Ile Leu
   355 360 365
   Met Lys Leu Lys Glu Ser Leu Glu Leu Met Glu Leu Val Pro Gln Pro
   370 375 380
   Leu Val Asp Ser Tyr Arg Gln Gln Gln Gln Leu Leu Gln Arg Pro Ser
   385 390 395 400
   His Leu Gln Pro Pro Ser Tyr Gly Pro Val Leu Ser Pro Met Asn Lys
   405 410 415
   Val His Gly Gly Val Asn Lys Leu Pro Ser Val Asn Gln Leu Val Gly
   420 425 430
   Gln Pro Pro Pro His Ser Ser Ala Ala Thr Pro Asn Leu Gly Pro Val
   435 440 445
   Gly Ser Gly Met Leu Asn Asn His Gly His Ala Val Pro Ala Asn Ser
   450 455 460
   Glu Met Thr Ser Ser His Gly Thr Gln Ser Met Val Ser Gly Ser His
   465 470 475 480
   Cys Thr Pro Pro Pro Pro Tyr His Ala Asp Pro Ser Leu Val Arg Thr
   485 490 495
   Trp Gly Pro
(2) INFORMATION POUR LA SEQ ID NO: 5:
   (i) CARACTERISTIQUES DE LA SEQUENCE:
      (A) LONGUEUR: 2156 paires de bases.
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (A) ORGANISME: Homo sapiens
   (ix) CARACTERISTIQUE ADDITIONELLE:
      (A) NOM/CLE: CDS
      (B) EMPLACEMENT: 33..1940
   (xi) DESCRIPTION DE LA SEQUENCE: SEQ ID NO: 5:
   GCGAGCTGCC CTCGGAGGCC GGCGTGGGGA AG ATG GCC CAG TCC ACC GCC ACC          53
                                       Met Ala Gln Ser Thr Ala Thr
                                        1               5
   TCC CCT GAT GGG GGC ACC ACG TTT GAG CAC CTC TGG AGC TCT CTG GAA         101
   Ser Pro Asp Gly Gly Thr Thr Phe Glu His Leu Trp Ser Ser Leu Glu
           10                  15                  20
   CCA GAC AGC ACC TAC TTC GAC CTT CCC CAG TCA AGC CGG GGG AAT AAT         149
   Pro Asp Ser Thr Tyr Phe Asp Leu Pro Gln Ser Ser Arg Gly Asn Asn
       25                  30                  35
   GAG GTG GTG GGC GGA ACG GAT TCC AGC ATG GAC GTC TTC CAC CTG GAG         197
   Glu Val Val Gly Gly Thr Asp Ser Ser Met Asp Val Phe His Leu Glu
   40                  45                  50                  55
   GGC ATG ACT ACA TCT GTC ATG GCC CAG TTC AAT CTG CTG AGC AGC ACC         245
   Gly Met Thr Thr Ser Val Met Ala Gln Phe Asn Leu Leu Ser Ser Thr
                   60                  65                  70
   ATG GAC CAG ATG AGC AGC CGC GCG GCC TCG GCC AGC CCC TAC ACC CCA         293
   Met Asp Gln Met Ser Ser Arg Ala Ala Ser Ala Ser Pro Tyr Thr Pro
               75                  80                  85
   GAG CAC GCC GCC AGC GTG CCC ACC CAC TCG CCC TAC GCA CAA CCC AGC         341
   Glu His Ala Ala Ser Val Pro Thr His Ser Pro Tyr Ala Gln Pro Ser
           90                  95                 100
   TCC ACC TTC GAC ACC ATG TCG CCG GCG CCT GTC ATC CCC TCC AAC ACC         389
   Ser Thr Phe Asp Thr Met Ser Pro Ala Pro Val Ile Pro Ser Asn Thr
       105                 110                 115
   GAC TAC CCC GGA CCC CAC CAC TTT GAG GTC ACT TTC CAG CAG TCC AGC         437
   Asp Tyr Pro Gly Pro His His Phe Glu Val Thr Phe Gln Gln Ser Ser
   120                 125                 130                 135
   ACG GCC AAG TCA GCC ACC TGG ACG TAC TCC CCG CTC TTG AAG AAA CTC         485
   Thr Ala Lys Ser Ala Thr Trp Thr Tyr Ser Pro Leu Leu Lys Lys Leu
                   140                 145                 150
   TAC TGC CAG ATC GCC AAG ACA TGC CCC ATC CAG ATC AAG GTG TCC ACC         533
   Tyr Cys Gln Ile Ala Lys Thr Cys Pro Ile Gln Ile Lys Val Ser Thr
               155                 160                 165
   CCG CCA CCC CCA GGC ACT GCC ATC CGG GCC ATG CCT GTT TAC AAG AAA         581
   Pro Pro Pro Pro Gly Thr Ala Ile Arg Ala Met Pro Val Tyr Lys Lys
           170                 175                 180
   GCG GAG CAC GTG ACC GAC GTC GTG AAA CGC TGC CCC AAC CAC GAG CTC         629
   Ala Glu His Val Thr Asp Val Val Lys Arg Cys Pro Asn His Glu Leu
       185                 190                 195
   GGG AGG GAC TTC AAC GAA GGA CAG TCT GCT CCA GCC AGC CAC CTC ATC         677
   Gly Arg Asp Phe Asn Glu Gly Gln Ser Ala Pro Ala Ser His Leu Ile
   200                 205                         210                 215
   CGC GTG GAA GGC AAT AAT CTC TCG CAG TAT GTG GAT GAC CCT GTC ACC      725
   Arg Val Glu Gly Asn Asn Leu Ser Gln Tyr Val Asp Asp Pro Val Thr
               220                     225                 230
   GGC AGG CAG AGC GTC GTG GTG CCC TAT GAG CCA CCA CAG GTG GGG ACG      773
   Gly Arg Gln Ser Val Val Val Pro Tyr Glu Pro Pro Gln Val Gly Thr
               235                 240                 245
   GAA TTC ACC ACC ATC CTG TAC AAC TTC ATG TGT AAC AGC AGC TGT GTA      821
   Glu Phe Thr Thr Ile Leu Tyr Asn Phe Met Cys Asn Ser Ser Cys Val
           250                 255                 260
   GGG GGC ATG AAC CGG CGG CCC ATC CTC ATC ATC ATC ACC CTG GAG ATG      869
   Gly Gly Met Asn Arg Arg Pro Ile Leu Ile Ile Ile Thr Leu Glu Met
       265                 270                 275
   CGG GAT GGG CAG GTG CTG GGC CGC CGG TCC TTT GAG GGC CGC ATC TGC      917
   Arg Asp Gly Gln Val Leu Gly Arg Arg Ser Phe Glu Gly Arg Ile Cys
   280                 285                 290                 295
   GCC TGT CCT GGC CGC GAC CGA AAA GCT GAT GAG GAC CAC TAC CGG GAG      965
   Ala Cys Pro Gly Arg Asp Arg Lys Ala Asp Glu Asp His Tyr Arg Glu
               300                 305                 310
   CAG CAG GCC CTG AAC GAG AGC TCC GCC AAG AAC GGG GCC GCC AGC AAG     1013
   Gln Gln Ala Leu Asn Glu Ser Ser Ala Lys Asn Gly Ala Ala Ser Lys
               315                 320                 325
   CGT GCC TTC AAG CAG AGC CCC CCT GCC GTC CCC GCC CTT GGT GCC GGT     1061
   Arg Ala Phe Lys Gln Ser Pro Pro Ala Val Pro Ala Leu Gly Ala Gly
           330                 335                 340
   GTG AAG AAG CGG CGG CAT GGA GAC GAG GAC ACG TAC TAC CTT CAG GTG     1109
   Val Lys Lys Arg Arg His Gly Asp Glu Asp Thr Tyr Tyr Leu Gln Val
       345                 350                 355
   CGA GGC CGG GAG AAC TTT GAG ATC CTG ATG AAG CTG AAA GAG AGC CTG     1157
   Arg Gly Arg Glu Asn Phe Glu Ile Leu Met Lys Leu Lys Glu Ser Leu
   360                 365                 370                 375
   GAG CTG ATG GAG TTG GTG CCG CAG CCA CTG GTG GAC TCC TAT CGG CAG     1205
   Glu Leu Met Glu Leu Val Pro Gln Pro Leu Val Asp Ser Tyr Arg Gln
               380                 385                 390
   CAG CAG CAG CTC CTA CAG AGG CCG AGT CAC CTA CAG CCC CCG TCC TAC     1253
   Gln Gln Gln Leu Leu Gln Arg Pro Ser His Leu Gln Pro Pro Ser Tyr
               395                 400                 405
   GGG CCG GTC CTC TCG CCC ATG AAC AAG GTG CAC GGG GGC ATG AAC AAG     1301
   Gly Pro Val Leu Ser Pro Met Asn Lys Val His Gly Gly Met Asn Lys
           410                 415                 420
   CTG CCC TCC GTC AAC CAG CTG GTG GGC CAG CCT CCC CCG CAC AGT TCG     1349
   Leu Pro Ser Val Asn Gln Leu Val Gly Gln Pro Pro Pro His Ser Ser
       425                 430                 435
   GCA GCT ACA CCC AAC CTG GGG CCC GTG GGC CCC GGG ATG CTC AAC AAC     1397
   Ala Ala Thr Pro Asn Leu Gly Pro Val Gly Pro Gly Met Leu Asn Asn
   440                 445                 450                 455
   CAT GGC CAC GCA GTG CCA GCC AAC GGC GAG ATG AGC AGC AGC CAC AGC     1445
   His Gly His Ala Val Pro Ala Asn Gly Glu Met Ser Ser Ser His Ser
               460                 465                 470
   GCC CAG TCC ATG GTC TCG GGG TCC CAC TGC ACT CCG CCA CCC CCC TAC     1493
   Ala Gln Ser Met Val Ser Gly Ser His Cys Thr Pro Pro Pro Pro Tyr
           475                 480                 485
   CAC GCC GAC CCC AGC CTC GTC AGT TTT TTA ACA GGA TTG GGG TGT CCA     1541
   His Ala Asp Pro Ser Leu Val Ser Phe Leu Thr Gly Leu Gly Cys Pro
           490                 495                 500
   AAC TGC ATC GAG TAT TTC ACC TCC CAA GGG TTA CAG AGC ATT TAC CAC      1589
   Asn Cys Ile Glu Tyr Phe Thr Ser Gln Gly Leu Gln Ser Ile Tyr His
       505                 510                 515
   CTG CAG AAC CTG ACC ATT GAG GAC CTG GGG GCC CTG AAG ATC CCC GAG      1637
   Leu Gln Asn Leu Thr Ile Glu Asp Leu Gly Ala Leu Lys Ile Pro Glu
   520                 525                 530                 535
   CAG TAC CGC ATG ACC ATC TGG CGG GGC CTG CAG GAC CTG AAG CAG GGC      1685
   Gln Tyr Arg Met Thr Ile Trp Arg Gly Leu Gln Asp Leu Lys Gln Gly
                   540                 545                 550
   CAC GAC TAC AGC ACC GCG CAG CAG CTG CTC CGC TCT AGC AAC GCG GCC      1733
   His Asp Tyr Ser Thr Ala Gln Gln Leu Leu Arg Ser Ser Asn Ala Ala
               555                 560                 565
   ACC ATC TCC ATC GGC GGC TCA GGG GAA CTG CAG CGC CAG CGG GTC ATG      1781
   Thr Ile Ser Ile Gly Gly Ser Gly Glu Leu Gln Arg Gln Arg Val Met
           570                 575                 580
   GAG GCC GTG CAC TTC CGC GTG CGC CAC ACC ATC ACC ATC CCC AAC CGC      1829
   Glu Ala Val His Phe Arg Val Arg His Thr Ile Thr Ile Pro Asn Arg
       585                 590                 595
   GGC GGC CCA GGC GGC GGC CCT GAC GAG TGG GCG GAC TTC GGC TTC GAC      1877
   Gly Gly Pro Gly Gly Gly Pro Asp Glu Trp Ala Asp Phe Gly Phe Asp
   600                 605                 610                 615
   CTG CCC GAC TGC AAG GCC CGC AAG CAG CCC ATC AAG GAG GAG TTC ACG      1925
   Leu Pro Asp Cys Lys Ala Arg Lys Gln Pro Ile Lys Glu Glu Phe Thr
                   620                 625                 630
   GAG GCC GAG ATC CAC TGAGGGCCTC GCCTGGCTGC AGCCTGCGCC ACCGCCCAGA      1980
   Glu Ala Glu Ile His
               635
   GACCCAAGCT GCCTCCCCTC TCCTTCCTGT GTGTCCAAAA CTGCCTCAGG AGGCAGGACC    2040
   TTCGGGCTGT GCCCGGGGAA AGGCAAGGTC CGGCCCATCC CCAGGCACCT CACAGGCCCC    2100
   AGGAAAGGCC CAGCCACCGA AGCCGCCTGT GGACAGCCTG AGTCACCTGC AGAACC        2156
(2) INFORMATION POUR LA SEQ ID NO: 6:
   (i) CARACTERISTIQUES DE LA SEQUENCE:
      (A) LONGUEUR: 636 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 6:
   Met Ala Gln Ser Thr Ala Thr Ser Pro Asp Gly Gly Thr Thr Phe Glu
    1               5                  10                  15
   His Leu Trp Ser Ser Leu Glu Pro Asp Ser Thr Tyr Phe Asp Leu Pro
               20                  25                  30
   Gln Ser Ser Arg Gly Asn Asn Glu Val Val Gly Gly Thr Asp Ser Ser
           35                  40                  45
   Met Asp Val Phe His Leu Glu Gly Met Thr Thr Ser Val Met Ala Gln
       50                  55                  60
   Phe Asn Leu Leu Ser Ser Thr Met Asp Gln Met Ser Ser Arg Ala Ala
   65                  70                  75                  80
   Ser Ala Ser Pro Tyr Thr Pro Glu His Ala Ala Ser Val Pro Thr His
   85 90 95
   Ser Pro Tyr Ala Gln Pro Ser Ser Thr Phe Asp Thr Met Ser Pro Ala
   100 105 110
   Pro Val Ile Pro Ser Asn Thr Asp Tyr Pro Gly Pro His His Phe Glu
   115 120 125
   Val Thr Phe Gln Gln Ser Ser Thr Ala Lys Ser Ala Thr Trp Thr Tyr
   130 135 140
   Ser Pro Leu Leu Lys Lys Leu Tyr Cys Gln Ile Ala Lys Thr Cys Pro
   145 150 155 160
   Ile Gln Ile Lys Val Ser Thr Pro Pro Pro Pro Gly Thr Ala Ile Arg
   165 170 175
   Ala Met Pro Val Tyr Lys Lys Ala Glu His Val Thr Asp Val Val Lys
   180 185 190
   Arg Cys Pro Asn His Glu Leu Gly Arg Asp Phe Asn Glu Gly Gln Ser
   195 200 205
   Ala Pro Ala Ser His Leu Ile Arg Val Glu Gly Asn Asn Leu Ser Gln
   210 215 220
   Tyr Val Asp Asp Pro Val Thr Gly Arg Gln Ser Val Val Val Pro Tyr
   225 230 235 240
   Glu Pro Pro Gln Val Gly Thr Glu Phe Thr Thr Ile Leu Tyr Asn Phe
   245 250 255
   Met Cys Asn Ser Ser Cys Val Gly Gly Met Asn Arg Arg Pro Ile Leu
   260 265 270
   Ile Ile Ile Thr Leu Glu Met Arg Asp Gly Gln Val Leu Gly Arg Arg
   275 280 285
   Ser Phe Glu Gly Arg Ile Cys Ala Cys Pro Gly Arg Asp Arg Lys Ala
   290 295 300
   Asp Glu Asp His Tyr Arg Glu Gln Gln Ala Leu Asn Glu Ser Ser Ala
   305 310 315 320
   Lys Asn Gly Ala Ala Ser Lys Arg Ala Phe Lys Gln Ser Pro Pro Ala
   325 330 335
   Val Pro Ala Leu Gly Ala Gly Val Lys Lys Arg Arg His Gly Asp Glu
   340 345 350
   Asp Thr Tyr Tyr Leu Gln Val Arg Gly Arg Glu Asn Phe Glu Ile Leu
   355 360 365
   Met Lys Leu Lys Glu Ser Leu Glu Leu Met Glu Leu Val Pro Gln Pro
   370 375 380
   Leu Val Asp Ser Tyr Arg Gln Gln Gln Gln Leu Leu Gln Arg Pro Ser
   385 390 395 400
   His Leu Gln Pro Pro Ser Tyr Gly Pro Val Leu Ser Pro Met Asn Lys
   405 410 415
   Val His Gly Gly Met Asn Lys Leu Pro Ser Val Asn Gln Leu Val Gly
   420 425 430
   Gln Pro Pro Pro His Ser Ser Ala Ala Thr Pro Asn Leu Gly Pro Val
   435 440 445
   Gly Pro Gly Met Leu Asn Asn His Gly His Ala Val Pro Ala Asn Gly
   450 455 460
   Glu Met Ser Ser Ser His Ser Ala Gln Ser Met Val Ser Gly Ser His
   465 470 475 480
   Cys Thr Pro Pro Pro Pro Tyr His Ala Asp Pro Ser Leu Val Ser Phe
   485 490 495
   Leu Thr Gly Leu Gly Cys Pro Asn Cys Ile Glu Tyr Phe Thr Ser Gln
   500 505 510
   Gly Leu Gln Ser Ile Tyr His Leu Gln Asn Leu Thr Ile Glu Asp Leu
   515 520 525
   Gly Ala Leu Lys Ile Pro Glu Gln Tyr Arg Met Thr Ile Trp Arg Gly
   530 535 540
   Leu Gln Asp Leu Lys Gln Gly His Asp Tyr Ser Thr Ala Gln Gln Leu
   545 550 555 560
   Leu Arg Ser Ser Asn Ala Ala Thr Ile Ser Ile Gly Gly Ser Gly Glu
   565 570 575
   Leu Gln Arg Gln Arg Val Met Glu Ala Val His Phe Arg Val Arg His
   580 585 590
   Thr Ile Thr Ile Pro Asn Arg Gly Gly Pro Gly Gly Gly Pro Asp Glu
   595 600 605
   Trp Ala Asp Phe Gly Phe Asp Leu Pro Asp Cys Lys Ala Arg Lys Gln
   610 615 620
   Pro Ile Lys Glu Glu Phe Thr Glu Ala Glu Ile His
   625 630 635
(2) INFORMATION POUR LA SEQ ID NO: 7:
   (i) CARACTERISTIQUES DE LA SEQUENCE:
      (A) LONGUEUR: 2040 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (A) ORGANISME: Mus musculus
   (ix) CARACTERISTIQUE ADDITIONELLE:
      (A) NOM/CLE: CDS
      (B) EMPLACEMENT: 124..1890
   (xi) DESCRIPTION DE LA SEQUENCE: SEQ ID NO: 7:
   TGATCTCCCT GTGGCCTGCA GGGGACTGAG CCAGGGAGTA GATGCCCTGA GACCCCAAGG     60
   GACACCCAAG GAAACCTTGC TGGCTTTGAG AAAGGGATCG TCTCTCTCCT GCCCAAGAGA    120
   AGC ATG TGT ATG GGC CCT GTG TAT GAA TCC TTG GGG CAG GCC CAG TTC      168
       Met Cys Met Gly Pro Val Tyr Glu Ser Leu Gly Gln Ala Gln Phe
        1               5                  10                  15
   AAT TTG CTC AGC AGT GCC ATG GAC CAG ATG GGC AGC CGT GCG GCC CCG      216
   Asn Leu Leu Ser Ser Ala Met Asp Gln Met Gly Ser Arg Ala Ala Pro
                   20                  25                  30
   GCG AGC CCC TAC ACC CCG GAG CAC GCC GCC AGC GCG CCC ACC CAC TCG      264
   Ala Ser Pro Tyr Thr Pro Glu His Ala Ala Ser Ala Pro Thr His Ser
               35                  40                  45
   CCC TAC GCG CAG CCC AGC TCC ACC TTC GAC ACC ATG TCT CCG GCG CCT      312
   Pro Tyr Ala Gln Pro Ser Ser Thr Phe Asp Thr Met Ser Pro Ala Pro
           50                  55                  60
   GTC ATC CCT TCC AAT ACC GAC TAC CCC GGC CCC CAC CAC TTC GAG GTC      360
   Val Ile Pro Ser Asn Thr Asp Tyr Pro Gly Pro His His Phe Glu Val
        65                  70                  75
   ACC TTC CAG CAG TCG AGC ACT GCC AAG TCG GCC ACC TGG ACA TAC TCC      408
   Thr Phe Gln Gln Ser Ser Thr Ala Lys Ser Ala Thr Trp Thr Tyr Ser
    80                  85                  90                  95
   CCA CTC TTG AAG AAG TTG TAC TGT CAG ATT GCT AAG ACA TGC CCC ATC      456
   Pro Leu Leu Lys Lys Leu Tyr Cys Gln Ile Ala Lys Thr Cys Pro Ile
                   100                 105                 110
   CAG ATC AAA GTG TCC ACA CCA CCA CCC CCG GGC ACG GCC ATC CGG GCC      504
   Gln Ile Lys Val Ser Thr Pro Pro Pro Pro Gly Thr Ala Ile Arg Ala
               115                 120                 125
   ATG CCT GTC TAC AAG AAG GCA GAG CAT GTG ACC GAC ATT GTT AAG CGC      552
   Met Pro Val Tyr Lys Lys Ala Glu His Val Thr Asp Ile Val Lys Arg
           130                 135                 140
   TGC CCC AAC CAC GAG CTT GGA AGG GAC TTC AAT GAA GGA CAG TCT GCC      600
   Cys Pro Asn His Glu Leu Gly Arg Asp Phe Asn Glu Gly Gln Ser Ala
       145                 150                 155
   CCG GCT AGC CAC CTC ATC CGT GTA GAA GGC AAC AAC CTC GCC CAG TAC      648
   Pro Ala Ser His Leu Ile Arg Val Glu Gly Asn Asn Leu Ala Gln Tyr
   160                 165                 170                 175
   GTG GAT GAC CCT GTC ACC GGA AGG CAG AGT GTG GTT GTG CCG TAT GAA      696
   Val Asp Asp Pro Val Thr Gly Arg Gln Ser Val Val Val Pro Tyr Glu
                   180                 185                 190
   CCC CCA CAG GTG GGA ACA GAA TTT ACC ACC ATC CTG TAC AAC TTC ATG      744
   Pro Pro Gln Val Gly Thr Glu Phe Thr Thr Ile Leu Tyr Asn Phe Met
               195                 200                 205
   TGT AAC AGC AGC TGT GTG GGG GGC ATG AAT CGG AGG CCC ATC CTT GTC      792
   Cys Asn Ser Ser Cys Val Gly Gly Met Asn Arg Arg Pro Ile Leu Val
           210                 215                 220
   ATC ATC ACC CTG GAG ACC CGG GAT GGA CAG GTC CTG GGC CGC CGG TCT      840
   Ile Ile Thr Leu Glu Thr Arg Asp Gly Gln Val Leu Gly Arg Arg Ser
       225                 230                 235
   TTC GAG GGT CGC ATC TGT GCC TGT CCT GGC CGT GAC CGC AAA GCT GAT      888
   Phe Glu Gly Arg Ile Cys Ala Cys Pro Gly Arg Asp Arg Lys Ala Asp
   240                 245                 250                 255
   GAA GAC CAT TAC CGG GAG CAA CAG GCT CTG AAT GAA AGT ACC ACC AAA      936
   Glu Asp His Tyr Arg Glu Gln Gln Ala Leu Asn Glu Ser Thr Thr Lys
                   260                 265                 270
   AAT GGA GCT GCC AGC AAA CGT GCA TTC AAG CAG AGC CCC CCT GCC ATC      984
   Asn Gly Ala Ala Ser Lys Arg Ala Phe Lys Gln Ser Pro Pro Ala Ile
               275                 280                 285
   CCT GCC CTG GGT ACC AAC GTG AAG AAG AGA CGC CAC GGG GAC GAG GAC     1032
   Pro Ala Leu Gly Thr Asn Val Lys Lys Arg Arg His Gly Asp Glu Asp
           290                 295                 300
   ATG TTC TAC ATG CAC GTG CGA GGC CGG GAG AAC TTT GAG ATC TTG ATG     1080
   Met Phe Tyr Met His Val Arg Gly Arg Glu Asn Phe Glu Ile Leu Met
       305                 310                 315
   AAA GTC AAG GAG AGC CTA GAA CTG ATG GAG CTT GTG CCC CAG CCT TTG     1128
   Lys Val Lys Glu Ser Leu Glu Leu Met Glu Leu Val Pro Gln Pro Leu
   320                 325                 330                 335
   GTT GAC TCC TAT CGA CAG CAG CAG CAG CAG CAG CTC CTA CAG AGG CCG     1176
   Val Asp Ser Tyr Arg Gln Gln Gln Gln Gln Gln Leu Leu Gln Arg Pro
                   340                 345                 350
   AGT CAC CTG CAG CCT CCA TCC TAT GGG CCC GTG CTC TCC CCA ATG AAC       1224
   Ser His Leu Gln Pro Pro Ser Tyr Gly Pro Val Leu Ser Pro Met Asn
               355                 360                 365
   AAG GTA CAC GGT GGT GTC AAC AAA CTG CCC TCC GTC AAC CAG CTG GTG       1272
   Lys Val His Gly Gly Val Asn Lys Leu Pro Ser Val Asn Gln Leu Val
           370                 375                 380
   GGC CAG CCT CCC CCG CAC AGC TCA GCA GCT GGG CCC AAC CTG GGG CCC       1320
   Gly Gln Pro Pro Pro His Ser Ser Ala Ala Gly Pro Asn Leu Gly Pro
       385                 390                 395
   ATG GGC TCC GGG ATG CTC AAC AGC CAC GGC CAC AGC ATG CCG GCC AAT       1368
   Met Gly Ser Gly Met Leu Asn Ser His Gly His Ser Met Pro Ala Asn
   400                 405                 410                 415
   GGT GAG ATG AAT GGA GGC CAC AGC TCC CAG ACC ATG GTT TCG GGA TCC       1416
   Gly Glu Met Asn Gly Gly His Ser Ser Gln Thr Met Val Ser Gly Ser
                   420                 425                 430
   CAC TGC ACC CCG CCA CCC CCC TAT CAT GCA GAC CCC AGC CTC GTC AGT       1464
   His Cys Thr Pro Pro Pro Pro Tyr His Ala Asp Pro Ser Leu Val Ser
               435                 440                 445
   TTT TTG ACA GGG TTG GGG TGT CCA AAC TGC ATC GAG TGC TTC ACT TCC       1512
   Phe Leu Thr Gly Leu Gly Cys Pro Asn Cys Ile Glu Cys Phe Thr Ser
           450                 455                 460
   CAA GGG TTG CAG AGC ATC TAC CAC CTG CAG AAC CTT ACC ATC GAG GAC       1560
   Gln Gly Leu Gln Ser Ile Tyr His Leu Gln Asn Leu Thr Ile Glu Asp
       465                 470                 475
   CTT GGG GCT CTG AAG GTC CCT GAC CAG TAC CGT ATG ACC ATC TGG AGG       1608
   Leu Gly Ala Leu Lys Val Pro Asp Gln Tyr Arg Met Thr Ile Trp Arg
   480                 485                 490                 495
   GGC CTA CAG GAC CTG AAG CAG AGC CAT GAC TGC GGC CAG CAA CTG CTA       1656
   Gly Leu Gln Asp Leu Lys Gln Ser His Asp Cys Gly Gln Gln Leu Leu
                   500                 505                 510
   CGC TCC AGC AGC AAC GCG GCC ACC ATC TCC ATC GGC GGC TCT GGC GAG       1704
   Arg Ser Ser Ser Asn Ala Ala Thr Ile Ser Ile Gly Gly Ser Gly Glu
               515                 520                 525
   CTG CAG CGG CAG CGG GTC ATG GAA GCC GTG CAT TTC CGT GTG CGC CAC       1752
   Leu Gln Arg Gln Arg Val Met Glu Ala Val His Phe Arg Val Arg His
           530                 535                 540
   ACC ATC ACA ATC CCC AAC CGT GGA GGC GCA GGT GCG GTG ACA GGT CCC       1800
   Thr Ile Thr Ile Pro Asn Arg Gly Gly Ala Gly Ala Val Thr Gly Pro
       545                 550                 555
   GAC GAG TGG GCG GAC TTT GGC TTT GAC CTG CCT GAC TGC AAG TCC CGT       1848
   Asp Glu Trp Ala Asp Phe Gly Phe Asp Leu Pro Asp Cys Lys Ser Arg
   560                 565                 570                 575
   AAG CAG CCC ATC AAA GAG GAG TTC ACA GAG ACA GAG AGC CAC               1890
   Lys Gln Pro Ile Lys Glu Glu Phe Thr Glu Thr Glu Ser His
                   580                 585
   TGAGGAACGT ACCTTCTTCT CCTGTCCTTC CTCTGTGAGA AACTGCTCTT GGAAGTGGGA     1950
   CCTGTTGGCT GTGCCCACAG AAACCAGCAA GGACCTTCTG CCGGATGCCA TTCCTGAAGG     2010
   GAAGTCGCTC ATGAACTAAC TCCCTCTTGG                                      2040
(2) INFORMATION POUR LA SEQ ID NO: 8:
   (i) CARACTERISTIQUES DE LA SEQUENCE:
      (A) LONGUEUR: 589 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 8:
   Met Cys Met Gly Pro Val Tyr Glu Ser Leu Gly Gln Ala Gln Phe Asn
   1 5 10 15
   Leu Leu Ser Ser Ala Met Asp Gln Met Gly Ser Arg Ala Ala Pro Ala
   20 25 30
   Ser Pro Tyr Thr Pro Glu His Ala Ala Ser Ala Pro Thr His Ser Pro
   35 40 45
   Tyr Ala Gln Pro Ser Ser Thr Phe Asp Thr Met Ser Pro Ala Pro Val
   50 55 60
   Ile Pro Ser Asn Thr Asp Tyr Pro Gly Pro His His Phe Glu Val Thr
   65 70 75 80
   Phe Gln Gln Ser Ser Thr Ala Lys Ser Ala Thr Trp Thr Tyr Ser Pro
   85 90 95
   Leu Leu Lys Lys Leu Tyr Cys Gln Ile Ala Lys Thr Cys Pro Ile Gln
   100 105 110
   Ile Lys Val Ser Thr Pro Pro Pro Pro Gly Thr Ala Ile Arg Ala Met
   115 120 125
   Pro Val Tyr Lys Lys Ala Glu His Val Thr Asp Ile Val Lys Arg Cys
   130 135 140
   Pro Asn His Glu Leu Gly Arg Asp Phe Asn Glu Gly Gln Ser Ala Pro
   145 150 155 160
   Ala Ser His Leu Ile Arg Val Glu Gly Asn Asn Leu Ala Gln Tyr Val
   165 170 175
   Asp Asp Pro Val Thr Gly Arg Gln Ser Val Val Val Pro Tyr Glu Pro
   180 185 190
   Pro Gln Val Gly Thr Glu Phe Thr Thr Ile Leu Tyr Asn Phe Met Cys
   195 200 205
   Asn Ser Ser Cys Val Gly Gly Met Asn Arg Arg Pro Ile Leu Val Ile
   210 215 220
   Ile Thr Leu Glu Thr Arg Asp Gly Gln Val Leu Gly Arg Arg Ser Phe
   225 230 235 240
   Glu Gly Arg Ile Cys Ala Cys Pro Gly Arg Asp Arg Lys Ala Asp Glu
   245 250 255
   Asp His Tyr Arg Glu Gln Gln Ala Leu Asn Glu Ser Thr Thr Lys Asn
   260 265 270
   Gly Ala Ala Ser Lys Arg Ala Phe Lys Gln Ser Pro Pro Ala Ile Pro
   275 280 285
   Ala Leu Gly Thr Asn Val Lys Lys Arg Arg His Gly Asp Glu Asp Met
   290 295 300
   Phe Tyr Met His Val Arg Gly Arg Glu Asn Phe Glu Ile Leu Met Lys
   305 310 315 320
   Val Lys Glu Ser Leu Glu Leu Met Glu Leu Val Pro Gln Pro Leu Val
   325 330 335
   Asp Ser Tyr Arg Gln Gln Gln Gln Gln Gln Leu Leu Gln Arg Pro Ser
   340 345 350
   His Leu Gln Pro Pro Ser Tyr Gly Pro Val Leu Ser Pro Met Asn Lys
           355                 360                 365
   Val His Gly Gly Val Asn Lys Leu Pro Ser Val Asn Gln Leu Val Gly
       370                 375                 380
   Gln Pro Pro Pro His Ser Ser Ala Ala Gly Pro Asn Leu Gly Pro Met
   385                 390                 395                 400
   Gly Ser Gly Met Leu Asn Ser His Gly His Ser Met Pro Ala Asn Gly
                   405                 410                 415
   Glu Met Asn Gly Gly His Ser Ser Gln Thr Met Val Ser Gly Ser His
               420                 425                 430
   Cys Thr Pro Pro Pro Pro Tyr His Ala Asp Pro Ser Leu Val Ser Phe
           435                 440                 445
   Leu Thr Gly Leu Gly Cys Pro Asn Cys Ile Glu Cys Phe Thr Ser Gln
       450                 455                 460
   Gly Leu Gln Ser Ile Tyr His Leu Gln Asn Leu Thr Ile Glu Asp Leu
   465                 470                 475                 480
   Gly Ala Leu Lys Val Pro Asp Gln Tyr Arg Met Thr Ile Trp Arg Gly
                   485                 490                 495
   Leu Gln Asp Leu Lys Gln Ser His Asp Cys Gly Gln Gln Leu Leu Arg
               500                 505                 510
   Ser Ser Ser Asn Ala Ala Thr Ile Ser Ile Gly Gly Ser Gly Glu Leu
           515                 520                 525
   Gln Arg Gln Arg Val Met Glu Ala Val His Phe Arg Val Arg His Thr
       530                 535                 540
   Ile Thr Ile Pro Asn Arg Gly Gly Ala Gly Ala Val Thr Gly Pro Asp
   545                 550                 555                 560
   Glu Trp Ala Asp Phe Gly Phe Asp Leu Pro Asp Cys Lys Ser Arg Lys
                   565                 570                 575
   Gln Pro Ile Lys Glu Glu Phe Thr Glu Thr Glu Ser His
               580                 585
(2) INFORMATION POUR LA SEQ ID NO: 9:
   (i) CARACTERISTIQUES DE LA SEQUENCE:
      (A) LONGUEUR: 758 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (A) ORGANISME: Mus musculus
   (ix) CARACTERISTIQUE ADDITIONELLE:
      (A) NOM/CLE: CDS
      (B) EMPLACEMENT: 389..757
   (xi) DESCRIPTION DE LA SEQUENCE: SEQ ID NO: 9:
   TGGTCCCGCT TCGACCAAGA CTCCGGCTAC CAGCTTGCGG GCCCCGCGGA GGAGGAGACC      60
   CCGCTGGGGC TAGCTGGGCG ACGCGCGCCA AGCGGCGGCG GGAAGGAGGC GGGAGGAGCG     120
   GGGCCCGAGA CCCCGACTCG GGCAGAGCCA GCTGGGGAGG CGGGGCGCGC GTGGGAGCCA     180
   GGGGCCCGGG TGGCCGGCCC TCCTCCGCCA CGGCTGAGTG CCCGCGCTGC CTTCCCGCCG     240
   GTCCGCCAAG AAAGGCGCTA AGCCTGCGGC AGTCCCCTCG CCGCCGCCTC CCTGCTCCGC     300
   ACCCTTATAA CCCGCCGTCC CGCATCCAGG CGAGGAGGCA ACGCTGCAGC CCAGCCCTCG     360
   CCGACGCCGA CGCCCGGCCC GGAGCAGA ATG AGC GGC AGC GTT GGG GAG ATG        412
                                  Met Ser Gly Ser Val Gly Glu Met
                                   1               5
   GCC CAG ACC TCT TCT TCC TCC TCC TCC ACC TTC GAG CAC CTG TGG AGT       460
   Ala Gln Thr Ser Ser Ser Ser Ser Ser Thr Phe Glu His Leu Trp Ser
        10                  15                  20
   TCT CTA GAG CCA GAC AGC ACC TAC TTT GAC CTC CCC CAG CCC AGC CAA       508
   Ser Leu Glu Pro Asp Ser Thr Tyr Phe Asp Leu Pro Gln Pro Ser Gln
   25                  30                  35                  40
   GGG ACT AGC GAG GCA TCA GGC AGC GAG GAG TCC AAC ATG GAT GTC TTC       556
   Gly Thr Ser Glu Ala Ser Gly Ser Glu Glu Ser Asn Met Asp Val Phe
                   45                  50                  55
   CAC CTG CAA GGC ATG GCC CAG TTC AAT TTG CTC AGC AGT GCC ATG GAC       604
   His Leu Gln Gly Met Ala Gln Phe Asn Leu Leu Ser Ser Ala Met Asp
               60                  65                  70
   CAG ATG GGC AGC CGT GCG GCC CCG GCG AGC CCC TAC ACC CCG GAG CAC       652
   Gln Met Gly Ser Arg Ala Ala Pro Ala Ser Pro Tyr Thr Pro Glu His
           75                  80                  85
   GCC GCC AGC GCG CCC ACC CAC TCG CCC TAC GCG CAG CCC AGC TCC ACC       700
   Ala Ala Ser Ala Pro Thr His Ser Pro Tyr Ala Gln Pro Ser Ser Thr
        90                  95                 100
   TTC GAC ACC ATG TCT CCG GCG CCT GTC ATC CCT TCC AAT ACC GAC TAC       748
   Phe Asp Thr Met Ser Pro Ala Pro Val Ile Pro Ser Asn Thr Asp Tyr
   105                 110                 115                 120
   CCC GGC CCC C                                                         758
   Pro Gly Pro
(2) INFORMATION POUR LA SEQ ID NO: 10:
   (i) CARACTERISTIQUES DE LA SEQUENCE:
      (A) LONGUEUR: 123 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 10:
   Met Ser Gly Ser Val Gly Glu Met Ala Gln Thr Ser Ser Ser Ser Ser
   1 5 10 15
   Ser Thr Phe Glu His Leu Trp Ser Ser Leu Glu Pro Asp Ser Thr Tyr
   20 25 30
   Phe Asp Leu Pro Gln Pro Ser Gln Gly Thr Ser Glu Ala Ser Gly Ser
   35 40 45
   Glu Glu Ser Asn Met Asp Val Phe His Leu Gln Gly Met Ala Gln Phe
   50 55 60
   Asn Leu Leu Ser Ser Ala Met Asp Gln Met Gly Ser Arg Ala Ala Pro
   65 70 75 80
   Ala Ser Pro Tyr Thr Pro Glu His Ala Ala Ser Ala Pro Thr His Ser
   85 90 95
   Pro Tyr Ala Gln Pro Ser Ser Thr Phe Asp Thr Met Ser Pro Ala Pro
   100 105 110
   Val Ile Pro Ser Asn Thr Asp Tyr Pro Gly Pro
   115 120
(2) INFORMATION POUR LA SEQ ID NO: 11:
   (i) CARACTERISTIQUES DE LA SEQUENCE:
      (A) LONGUEUR: 559 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (A) ORGANISME: Homo sapiens
   (xi) DESCRIPTION DE LA SEQUENCE: SEQ ID NO: 11:
   CGACCTTCCC CAGTCAAGCC GGGGGAATAA TGAGGTGGTG GGCGGAACGG ATTCCAGCAT 60
   GGACGTCTTC CACCTGGAGG GCATGACTAC ATCTGTCATG CATCCTCGGC TCCTGCCTCA 120
   CTAGCTGCGG AGCCTCTCCC GCTCGGTCCA CGCTGCCGGG CGGCCACGAC CGTGACCCTT 180
   CCCCTCGGGC CGCCCAGATC CATGCCTCGT CCCACGGGAC ACCAGTTCCC TGGCGTGTGC 240
   AGACCCCCCG GCGCCTACCA TGCTGTACGT CGGTGACCCC GCACGGCACC TCGCCACGGC 300
   CCAGTTCAAT CTGCTGAGCA GCACCATGGA CCAGATGAGC AGCCGCGCGG CCTCGGCCAG 360
   CCCCTACACC CCAGAGCACG CCGCCAGCGT GCCCACCCAC TCGCCCTACG CACAACCCAG 420
   CTCCACCTTC GACACCATGT CGCCGGCGCC TGTCATCCCC TCCAACACCG ACTACCCCGG 480
   ACCCCACCAC TTTGAGGTCA CTTTCCAGCA GTCCAGCACG GCCAAGTCAG CCACCTGGAC 540
   GTACTCCCCG CTCTTGAAG 559
(2) INFORMATION POUR LA SEQ ID NO: 12:
   (i) CARACTERISTIQUES DE LA SEQUENCE:
      (A) LONGUEUR: 1764 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (A) ORGANISME: Homo sapiens
   (xi) DESCRIPTION DE LA SEQUENCE: SEQ ID NO: 12:
   ATGCTGTACG TCGGTGACCC CGCACGGCAC CTCGCCACGG CCCAGTTCAA TCTGCTGAGC    60
   AGCACCATGG ACCAGATGAG CAGCCGCGCG GCCTCGGCCA GCCCCTACAC CCCAGAGCAC   120
   GCCGCCAGCG TGCCCACCCA CTCGCCCTAC GCACAACCCA GCTCCACCTT CGACACCATG   180
   TCGCCGGCGC CTGTCATCCC CTCCAACACC GACTACCCCG GACCCCACCA CTTTGAGGTC   240
   ACTTTCCAGC AGTCCAGCAC GGCCAAGTCA GCCACCTGGA CGTACTCCCC GCTCTTGAAG   300
   AAACTCTACT GCCAGATCGC CAAGACATGC CCCATCCAGA TCAAGGTGTC CACCCCGCCA   360
   CCCCCAGGCA CTGCCATCCG GGCCATGCCT GTTTACAAGA AAGCGGAGCA CGTGACCGAC   420
   GTCGTGAAAC GCTGCCCCAA CCACGAGCTC GGGAGGGACT TCAACGAAGG ACAGTCTGCT    480
   CCAGCCAGCC ACCTCATCCG CGTGGAAGGC AATAATCTCT CGCAGTATGT GGATGACCCT    540
   GTCACCGGCA GGCAGAGCGT CGTGGTGCCC TATGAGCCAC CACAGGTGGG GACGGAATTC    600
   ACCACCATCC TGTACAACTT CATGTGTAAC AGCAGCTGTG TAGGGGGCAT GAACCGGCGG    660
   CCCATCCTCA TCATCATCAC CCTGGAGATG CGGGATGGGC AGGTGCTGGG CCGCCGGTCC    720
   TTTGAGGGCC GCATCTGCGC CTGTCCTGGC CGCGACCGAA AAGCTGATGA GGACCACTAC    780
   CGGGAGCAGC AGGCCCTGAA CGAGAGCTCC GCCAAGAACG GGGCCGCCAG CAAGCGTGCC    840
   TTCAAGCAGA GCCCCCCTGC CGTCCCCGCC CTTGGTGCCG GTGTGAAGAA GCGGCGGCAT    900
   GGAGACGAGG ACACGTACTA CCTTCAGGTG CGAGGCCGGG AGAACTTTGA GATCCTGATG    960
   AAGCTGAAAG AGAGCCTGGA GCTGATGGAG TTGGTGCCGC AGCCACTGGT GGACTCCTAT   1020
   CGGCAGCAGC AGCAGCTCCT ACAGAGGCCG AGTCACCTAC AGCCCCCGTC CTACGGGCCG   1080
   GTCCTCTCGC CCATGAACAA GGTGCACGGG GGCATGAACA AGCTGCCCTC CGTCAACCAG   1140
   CTGGTGGGCC AGCCTCCCCC GCACAGTTCG GCAGCTACAC CCAACCTGGG GCCCGTGGGC   1200
   CCCGGGATGC TCAACAACCA TGGCCACGCA GTGCCAGCCA ACGGCGAGAT GAGCAGCAGC   1260
   CACAGCGCCC AGTCCATGGT CTCGGGGTCC CACTGCACTC CGCCACCCCC CTACCACGCC   1320
   GACCCCAGCC TCGTCAGTTT TTTAACAGGA TTGGGGTGTC CAAACTGCAT CGAGTATTTC   1380
   ACCTCCCAAG GGTTACAGAG CATTTACCAC CTGCAGAACC TGACCATTGA GGACCTGGGG   1440
   GCCCTGAAGA TCCCCGAGCA GTACCGCATG ACCATCTGGC GGGGCCTGCA GGACCTGAAG   1500
   CAGGGCCACG ACTACAGCAC CGCGCAGCAG CTGCTCCGCT CTAGCAACGC GGCCACCATC   1560
   TCCATCGGCG GCTCAGGGGA ACTGCAGCGC CAGCGGGTCA TGGAGGCCGT GCACTTCCGC   1620
   GTGCGCCACA CCATCACCAT CCCCAACCGC GGCGGCCCAG GCGGCGGCCC TGACGAGTGG   1680
   GCGGACTTCG GCTTCGACCT GCCCGACTGC AAGGCCCGCA AGCAGCCCAT CAAGGAGGAG   1740
   TTCACGGAGG CCGAGATCCA CTGA                                          1764
(2) INFORMATION POUR LA SEQ ID NO: 13:
   (i) CARACTERISTIQUES DE LA SEQUENCE:
      (A) LONGUEUR: 587 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 13:
   Met Leu Tyr Val Gly Asp Pro Ala Arg His Leu Ala Thr Ala Gln Phe
   1               5                   10                  15
   Asn Leu Leu Ser Ser Thr Met Asp Gln Met Ser Ser Arg Ala Ala Ser
               20                  25                  30
   Ala Ser Pro Tyr Thr Pro Glu His Ala Ala Ser Val Pro Thr His Ser
           35                  40                  45
   Pro Tyr Ala Gln Pro Ser Ser Thr Phe Asp Thr Met Ser Pro Ala Pro
       50                  55                  60
   Val Ile Pro Ser Asn Thr Asp Tyr Pro Gly Pro His His Phe Glu Val
   65 70 75 80
   Thr Phe Gln Gln Ser Ser Thr Ala Lys Ser Ala Thr Trp Thr Tyr Ser
   85 90 95
   Pro Leu Leu Lys Lys Leu Tyr Cys Gln Ile Ala Lys Thr Cys Pro Ile
   100 105 110
   Gln Ile Lys Val Ser Thr Pro Pro Pro Pro Gly Thr Ala Ile Arg Ala
   115 120 125
   Met Pro Val Tyr Lys Lys Ala Glu His Val Thr Asp Val Val Lys Arg
   130 135 140
   Cys Pro Asn His Glu Leu Gly Arg Asp Phe Asn Glu Gly Gln Ser Ala
   145 150 155 160
   Pro Ala Ser His Leu Ile Arg Val Glu Gly Asn Asn Leu Ser Gln Tyr
   165 170 175
   Val Asp Asp Pro Val Thr Gly Arg Gln Ser Val Val Val Pro Tyr Glu
   180 185 190
   Pro Pro Gln Val Gly Thr Glu Phe Thr Thr Ile Leu Tyr Asn Phe Met
   195 200 205
   Cys Asn Ser Ser Cys Val Gly Gly Met Asn Arg Arg Pro Ile Leu Ile
   210 215 220
   Ile Ile Thr Leu Glu Met Arg Asp Gly Gln Val Leu Gly Arg Arg Ser
   225 230 235 240
   Phe Glu Gly Arg Ile Cys Ala Cys Pro Gly Arg Asp Arg Lys Ala Asp
   245 250 255
   Glu Asp His Tyr Arg Glu Gln Gln Ala Leu Asn Glu Ser Ser Ala Lys
   260 265 270
   Asn Gly Ala Ala Ser Lys Arg Ala Phe Lys Gln Ser Pro Pro Ala Val
   275 280 285
   Pro Ala Leu Gly Ala Gly Val Lys Lys Arg Arg His Gly Asp Glu Asp
   290 295 300
   Thr Tyr Tyr Leu Gln Val Arg Gly Arg Glu Asn Phe Glu Ile Leu Met
   305 310 315 320
   Lys Leu Lys Glu Ser Leu Glu Leu Met Glu Leu Val Pro Gln Pro Leu
   325 330 335
   Val Asp Ser Tyr Arg Gln Gln Gln Gln Leu Leu Gln Arg Pro Ser His
   340 345 350
   Leu Gln Pro Pro Ser Tyr Gly Pro Val Leu Ser Pro Met Asn Lys Val
   355 360 365
   His Gly Gly Met Asn Lys Leu Pro Ser Val Asn Gln Leu Val Gly Gln
   370 375 380
   Pro Pro Pro His Ser Ser Ala Ala Thr Pro Asn Leu Gly Pro Val Gly
   385 390 395 400
   Pro Gly Met Leu Asn Asn His Gly His Ala Val Pro Ala Asn Gly Glu
   405 410 415
   Met Ser Ser Ser His Ser Ala Gln Ser Met Val Ser Gly Ser His Cys
   420 425 430
   Thr Pro Pro Pro Pro Tyr His Ala Asp Pro Ser Leu Val Ser Phe Leu
   435 440 445
   Thr Gly Leu Gly Cys Pro Asn Cys Ile Glu Tyr Phe Thr Ser Gln Gly
       450                 455                 460
   Leu Gln Ser Ile Tyr His Leu Gln Asn Leu Thr Ile Glu Asp Leu Gly
   465                 470                 475                 480
   Ala Leu Lys Ile Pro Glu Gln Tyr Arg Met Thr Ile Trp Arg Gly Leu
                   485                 490                 495
   Gln Asp Leu Lys Gln Gly His Asp Tyr Ser Thr Ala Gln Gln Leu Leu
               500                 505                 510
   Arg Ser Ser Asn Ala Ala Thr Ile Ser Ile Gly Gly Ser Gly Glu Leu
           515                 520                 525
   Gln Arg Gln Arg Val Met Glu Ala Val His Phe Arg Val Arg His Thr
       530                 535                 540
   Ile Thr Ile Pro Asn Arg Gly Gly Pro Gly Gly Gly Pro Asp Glu Trp
   545                 550                 555                 560
   Ala Asp Phe Gly Phe Asp Leu Pro Asp Cys Lys Ala Arg Lys Gln Pro
                   565                 570                 575
   Ile Lys Glu Glu Phe Thr Glu Ala Glu Ile His
               580                 585
(2) INFORMATION POUR LA SEQ ID NO: 14:
   (i) CARACTERISTIQUES DE LA SEQUENCE:
      (A) LONGUEUR: 1521 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (A) ORGANISME: Homo sapiens
   (xi) DESCRIPTION DE LA SEQUENCE: SEQ ID NO: 14:
   ATGCTGTACG TCGGTGACCC CGCACGGCAC CTCGCCACGG CCCAGTTCAA TCTGCTGAGC   60
   AGCACCATGG ACCAGATGAG CAGCCGCGCG GCCTCGGCCA GCCCCTACAC CCCAGAGCAC  120
   GCCGCCAGCG TGCCCACCCA CTCGCCCTAC GCACAACCCA GCTCCACCTT CGACACCATG  180
   TCGCCGGCGC CTGTCATCCC CTCCAACACC GACTACCCCG GACCCCACCA CTTTGAGGTC  240
   ACTTTCCAGC AGTCCAGCAC GGCCAAGTCA GCCACCTGGA CGTACTCCCC GCTCTTGAAG  300
   AAACTCTACT GCCAGATCGC CAAGACATGC CCCATCCAGA TCAAGGTGTC CACCCCGCCA  360
   CCCCCAGGCA CTGCCATCCG GGCCATGCCT GTTTACAAGA AAGCGGAGCA CGTGACCGAC  420
   GTCGTGAAAC GCTGCCCCAA CCACGAGCTC GGGAGGGACT TCAACGAAGG ACAGTCTGCT  480
   CCAGCCAGCC ACCTCATCCG CGTGGAAGGC AATAATCTCT CGCAGTATGT GGATGACCCT  540
   GTCACCGGCA GGCAGAGCGT CGTGGTGCCC TATGAGCCAC CACAGGTGGG GACGGAATTC  600
   ACCACCATCC TGTACAACTT CATGTGTAAC AGCAGCTGTG TAGGGGGCAT GAACCGGCGG  660
   CCCATCCTCA TCATCATCAC CCTGGAGATG CGGGATGGGC AGGTGCTGGG CCGCCGGTCC  720
   TTTGAGGGCC GCATCTGCGC CTGTCCTGGC CGCGACCGAA AAGCTGATGA GGACCACTAC  780
   CGGGAGCAGC AGGCCCTGAA CGAGAGCTCC GCCAAGAACG GGGCCGCCAG CAAGCGTGCC  840
   TTCAAGCAGA GCCCCCCTGC CGTCCCCGCC CTTGGTGCCG GTGTGAAGAA GCGGCGGCAT  900
   GGAGACGAGG ACACGTACTA CCTTCAGGTG CGAGGCCGGG AGAACTTTGA GATCCTGATG  960
   AAGCTGAAAG AGAGCCTGGA GCTGATGGAG TTGGTGCCGC AGCCACTGGT GGACTCCTAT 1020
   CGGCAGCAGC AGCAGCTCCT ACAGAGGCCG CCCCGGGATG CTCAACAACC ATGGCCACGC 1080
   AGTGCCAGCC AACGGCGAGA TGAGCAGCAG CCACAGCGCC CAGTCCATGG TCTCGGGGTC 1140
   CCACTGCACT CCGCCACCCC CCTACCACGC CGACCCCAGC CTCGTCAGGA CCTGGGGGCC 1200
   CTGAAGATCC CCGAGCAGTA CCGCATGACC ATCTGGCGGG GCCTGCAGGA CCTGAAGCAG 1260
   GGCCACGACT ACAGCACCGC GCAGCAGCTG CTCCGCTCTA GCAACGCGGC CACCATCTCC 1320
   ATCGGCGGCT CAGGGGAACT GCAGCGCCAG CGGGTCATGG AGGCCGTGCA CTTCCGCGTG 1380
   CGCCACACCA TCACCATCCC CAACCGCGGC GGCCCAGGCG GCGGCCCTGA CGAGTGGGCG 1440
   GACTTCGGCT TCGACCTGCC CGACTGCAAG GCCCGCAAGC AGCCCATCAA GGAGGAGTTC 1500
   ACGGAGGCCG AGATCCACTG A                                          1521
(2) INFORMATION POUR LA SEQ ID NO: 15:
   (i) CARACTERISTIQUES DE LA SEQUENCE:
      (A) LONGUEUR: 506 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 15:
   Met Leu Tyr Val Gly Asp Pro Ala Arg His Leu Ala Thr Ala Gln Phe
   1               5                   10                  15
   Asn Leu Leu Ser Ser Thr Met Asp Gln Met Ser Ser Arg Ala Ala Ser
               20                  25                  30
   Ala Ser Pro Tyr Thr Pro Glu His Ala Ala Ser Val Pro Thr His Ser
           35                  40                  45
   Pro Tyr Ala Gln Pro Ser Ser Thr Phe Asp Thr Met Ser Pro Ala Pro
       50                  55                  60
   Val Ile Pro Ser Asn Thr Asp Tyr Pro Gly Pro His His Phe Glu Val
   65                  70                  75                  80
   Thr Phe Gln Gln Ser Ser Thr Ala Lys Ser Ala Thr Trp Thr Tyr Ser
                   85                  90                  95
   Pro Leu Leu Lys Lys Leu Tyr Cys Gln Ile Ala Lys Thr Cys Pro Ile
               100                 105                 110
   Gln Ile Lys Val Ser Thr Pro Pro Pro Pro Gly Thr Ala Ile Arg Ala
           115                 120                 125
   Met Pro Val Tyr Lys Lys Ala Glu His Val Thr Asp Val Val Lys Arg
       130                 135                 140
   Cys Pro Asn His Glu Leu Gly Arg Asp Phe Asn Glu Gly Gln Ser Ala
   145                 150                 155                 160
   Pro Ala Ser His Leu Ile Arg Val Glu Gly Asn Asn Leu Ser Gln Tyr
                   165                 170                 175
   Val Asp Asp Pro Val Thr Gly Arg Gln Ser Val Val Val Pro Tyr Glu
               180                 185                 190
   Pro Pro Gln Val Gly Thr Glu Phe Thr Thr Ile Leu Tyr Asn Phe Met
           195                 200                 205
   Cys Asn Ser Ser Cys Val Gly Gly Met Asn Arg Arg Pro Ile Leu Ile
       210                 215                 220
   Ile Ile Thr Leu Glu Met Arg Asp Gly Gln Val Leu Gly Arg Arg Ser
   225                 230                 235                 240
   Phe Glu Gly Arg Ile Cys Ala Cys Pro Gly Arg Asp Arg Lys Ala Asp
                   245                 250                 255
   Glu Asp His Tyr Arg Glu Gln Gln Ala Leu Asn Glu Ser Ser Ala Lys
               260                 265                 270
   Asn Gly Ala Ala Ser Lys Arg Ala Phe Lys Gln Ser Pro Pro Ala Val
           275                 280                 285
   Pro Ala Leu Gly Ala Gly Val Lys Lys Arg Arg His Gly Asp Glu Asp
       290                 295                 300
   Thr Tyr Tyr Leu Gln Val Arg Gly Arg Glu Asn Phe Glu Ile Leu Met
   305                 310                 315                 320
   Lys Leu Lys Glu Ser Leu Glu Leu Met Glu Leu Val Pro Gln Pro Leu
                   325                 330                 335
   Val Asp Ser Tyr Arg Gln Gln Gln Gln Leu Leu Gln Arg Pro Pro Arg
               340                 345                 350
   Asp Ala Gln Gln Pro Trp Pro Arg Ser Ala Ser Gln Arg Arg Asp Glu
           355                 360                 365
   Gln Gln Pro Gln Arg Pro Val His Gly Leu Gly Val Pro Leu His Ser
       370                 375                 380
   Ala Thr Pro Leu Pro Arg Arg Pro Gln Pro Arg Gln Asp Leu Gly Ala
   385                 390                 395                 400
   Leu Lys Ile Pro Glu Gln Tyr Arg Met Thr Ile Trp Arg Gly Leu Gln
                   405                 410                 415
   Asp Leu Lys Gln Gly His Asp Tyr Ser Thr Ala Gln Gln Leu Leu Arg
               420                 425                 430
   Ser Ser Asn Ala Ala Thr Ile Ser Ile Gly Gly Ser Gly Glu Leu Gln
           435                 440                 445
   Arg Gln Arg Val Met Glu Ala Val His Phe Arg Val Arg His Thr Ile
       450                 455                 460
   Thr Ile Pro Asn Arg Gly Gly Pro Gly Gly Gly Pro Asp Glu Trp Ala
   465                 470                 475                 480
   Asp Phe Gly Phe Asp Leu Pro Asp Cys Lys Ala Arg Lys Gln Pro Ile
                   485                 490                 495
   Lys Glu Glu Phe Thr Glu Ala Glu Ile His
               500                 505
(2) INFORMATION POUR LA SEQ ID NO: 16:
   (i) CARACTERISTIQUES DE LA SEQUENCE:
      (A) LONGUEUR: 1870 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (A) ORGANISME: Homo sapiens
   (ix) CARACTERISTIQUE ADDITIONELLE:
      (A) NOM/CLE: CDS
      (B) EMPLACEMENT: 104..1867
   (xi) DESCRIPTION DE LA SEQUENCE: SEQ ID NO: 16:
   TGCCCGGGGC TGCGACGGCT GCAGGGAACC AGACAGCACC TACTTCGACC TTCCCCAGTC      60
   AAGCCGGGGG AATAATGAGG TGGTGGGCGG AACGGATTCC AGC ATG GAC GTC TTC       115
                                                   Met Asp Val Phe
                                                    1
   CAC CTG GAG GGC ATG ACT ACA TCT GTC ATG GCC CAG TTC AAT CTG CTG       163
   His Leu Glu Gly Met Thr Thr Ser Val Met Ala Gln Phe Asn Leu Leu
     5                  10                  15                  20
   AGC AGC ACC ATG GAC CAG ATG AGC AGC CGC GCG GCC TCG GCC AGC CCC       211
   Ser Ser Thr Met Asp Gln Met Ser Ser Arg Ala Ala Ser Ala Ser Pro
                    25                  30                  35
   TAC ACC CCA GAG CAC GCC GCC AGC GTG CCC ACC CAC TCG CCC TAC GCA       259
   Tyr Thr Pro Glu His Ala Ala Ser Val Pro Thr His Ser Pro Tyr Ala
                40                  45                  50
   CAA CCC AGC TCC ACC TTC GAC ACC ATG TCG CCG GCG CCT GTC ATC CCC       307
   Gln Pro Ser Ser Thr Phe Asp Thr Met Ser Pro Ala Pro Val Ile Pro
            55                  60                  65
   TCC AAC ACC GAC TAC CCC GGA CCC CAC CAC TTT GAG GTC ACT TTC CAG       355
   Ser Asn Thr Asp Tyr Pro Gly Pro His His Phe Glu Val Thr Phe Gln
        70                  75                  80
   CAG TCC AGC ACG GCC AAG TCA GCC ACC TGG ACG TAC TCC CCG CTC TTG       403
   Gln Ser Ser Thr Ala Lys Ser Ala Thr Trp Thr Tyr Ser Pro Leu Leu
    85                  90                  95                 100
   AAG AAA CTC TAC TGC CAG ATC GCC AAG ACA TGC CCC ATC CAG ATC AAG       451
   Lys Lys Leu Tyr Cys Gln Ile Ala Lys Thr Cys Pro Ile Gln Ile Lys
                   105                 110                 115
   GTG TCC ACC CCG CCA CCC CCA GGC ACT GCC ATC CGG GCC ATG CCT GTT       499
   Val Ser Thr Pro Pro Pro Pro Gly Thr Ala Ile Arg Ala Met Pro Val
               120                 125                 130
   TAC AAG AAA GCG GAG CAC GTG ACC GAC GTC GTG AAA CGC TGC CCC AAC       547
   Tyr Lys Lys Ala Glu His Val Thr Asp Val Val Lys Arg Cys Pro Asn
           135                 140                 145
   CAC GAG CTC GGG AGG GAC TTC AAC GAA GGA CAG TCT GCT CCA GCC AGC       595
   His Glu Leu Gly Arg Asp Phe Asn Glu Gly Gln Ser Ala Pro Ala Ser
       150                 155                 160
   CAC CTC ATC CGC GTG GAA GGC AAT AAT CTC TCG CAG TAT GTG GAT GAC       643
   His Leu Ile Arg Val Glu Gly Asn Asn Leu Ser Gln Tyr Val Asp Asp
   165                 170                 175                 180
   CCT GTC ACC GGC AGG CAG AGC GTC GTG GTG CCC TAT GAG CCA CCA CAG       691
   Pro Val Thr Gly Arg Gln Ser Val Val Val Pro Tyr Glu Pro Pro Gln
                   185                 190                 195
   GTG GGG ACG GAA TTC ACC ACC ATC CTG TAC AAC TTC ATG TGT AAC AGC       739
   Val Gly Thr Glu Phe Thr Thr Ile Leu Tyr Asn Phe Met Cys Asn Ser
               200                 205                 210
   AGC TGT GTA GGG GGC ATG AAC CGG CGG CCC ATC CTC ATC ATC ATC ACC       787
   Ser Cys Val Gly Gly Met Asn Arg Arg Pro Ile Leu Ile Ile Ile Thr
           215                 220                 225
   CTG GAG ATG CGG GAT GGG CAG GTG CTG GGC CGC CGG TCC TTT GAG GGC       835
   Leu Glu Met Arg Asp Gly Gln Val Leu Gly Arg Arg Ser Phe Glu Gly
       230                     235                     240
   CGC ATC TGC GCC TGT CCT GGC CGC GAC CGA AAA GCT GAT GAG GAC CAC      883
   Arg Ile Cys Ala Cys Pro Gly Arg Asp Arg Lys Ala Asp Glu Asp His
   245                 250                 255                 260
   TAC CGG GAG CAG CAG GCC CTG AAC GAG AGC TCC GCC AAG AAC GGG GCC      931
   Tyr Arg Glu Gln Gln Ala Leu Asn Glu Ser Ser Ala Lys Asn Gly Ala
                   265                 270                 275
   GCC AGC AAG CGT GCC TTC AAG CAG AGC CCC CCT GCC GTC CCC GCC CTT      979
   Ala Ser Lys Arg Ala Phe Lys Gln Ser Pro Pro Ala Val Pro Ala Leu
               280                 285                 290
   GGT GCC GGT GTG AAG AAG CGG CGG CAT GGA GAC GAG GAC ACG TAC TAC     1027
   Gly Ala Gly Val Lys Lys Arg Arg His Gly Asp Glu Asp Thr Tyr Tyr
           295                 300                 305
   CTT CAG GTG CGA GGC CGG GAG AAC TTT GAG ATC CTG ATG AAG CTG AAA     1075
   Leu Gln Val Arg Gly Arg Glu Asn Phe Glu Ile Leu Met Lys Leu Lys
       310                 315                 320
   GAG AGC CTG GAG CTG ATG GAG TTG GTG CCG CAG CCA CTG GTG GAC TCC     1123
   Glu Ser Leu Glu Leu Met Glu Leu Val Pro Gln Pro Leu Val Asp Ser
   325                 330                 335                 340
   TAT CGG CAG CAG CAG CAG CTC CTA CAG AGG CCG AGT CAC CTA CAG CCC     1171
   Tyr Arg Gln Gln Gln Gln Leu Leu Gln Arg Pro Ser His Leu Gln Pro
                   345                 350                 355
   CCG TCC TAC GGG CCG GTC CTC TCG CCC ATG AAC AAG GTG CAC GGG GGC     1219
   Pro Ser Tyr Gly Pro Val Leu Ser Pro Met Asn Lys Val His Gly Gly
               360                 365                 370
   ATG AAC AAG CTG CCC TCC GTC AAC CAG CTG GTG GGC CAG CCT CCC CCG     1267
   Met Asn Lys Leu Pro Ser Val Asn Gln Leu Val Gly Gln Pro Pro Pro
           375                 380                 385
   CAC AGT TCG GCA GCT ACA CCC AAC CTG GGG CCC GTG GGC CCC GGG ATG     1315
   His Ser Ser Ala Ala Thr Pro Asn Leu Gly Pro Val Gly Pro Gly Met
       390                 395                 400
   CTC AAC AAC CAT GGC CAC GCA GTG CCA GCC AAC GGC GAG ATG AGC AGC     1363
   Leu Asn Asn His Gly His Ala Val Pro Ala Asn Gly Glu Met Ser Ser
   405                 410                 415                 420
   AGC CAC AGC GCC CAG TCC ATG GTC TCG GGG TCC CAC TGC ACT CCG CCA     1411
   Ser His Ser Ala Gln Ser Met Val Ser Gly Ser His Cys Thr Pro Pro
                   425                 430                 435
   CCC CCC TAC CAC GCC GAC CCC AGC CTC GTC AGT TTT TTA ACA GGA TTG     1459
   Pro Pro Tyr His Ala Asp Pro Ser Leu Val Ser Phe Leu Thr Gly Leu
               440                 445                 450
   GGG TGT CCA AAC TGC ATC GAG TAT TTC ACC TCC CAA GGG TTA CAG AGC     1507
   Gly Cys Pro Asn Cys Ile Glu Tyr Phe Thr Ser Gln Gly Leu Gln Ser
           455                 460                 465
   ATT TAC CAC CTG CAG AAC CTG ACC ATT GAG GAC CTG GGG GCC CTG AAG     1555
   Ile Tyr His Leu Gln Asn Leu Thr Ile Glu Asp Leu Gly Ala Leu Lys
       470                 475                 480
   ATC CCC GAG CAG TAC CGC ATG ACC ATC TGG CGG GGC CTG CAG GAC CTG     1603
   Ile Pro Glu Gln Tyr Arg Met Thr Ile Trp Arg Gly Leu Gln Asp Leu
   485                 490                 495                 500
   AAG CAG GGC CAC GAC TAC AGC ACC GCG CAG CAG CTG CTC CGC TCT AGC     1651
   Lys Gln Gly His Asp Tyr Ser Thr Ala Gln Gln Leu Leu Arg Ser Ser
                   505                 510                 515
   AAC GCG GCC ACC ATC TCC ATC GGC GGC TCA GGG GAA CTG CAG CGC CAG     1699
   Asn Ala Ala Thr Ile Ser Ile Gly Gly Ser Gly Glu Leu Gln Arg Gln
                   520                     525                     530
   CGG GTC ATG GAG GCC GTG CAC TTC CGC GTG CGC CAC ACC ATC ACC ATC        1747
   Arg Val Met Glu Ala Val His Phe Arg Val Arg His Thr Ile Thr Ile
           535                 540                     545
   CCC AAC CGC GGC GGC CCA GGC GGC GGC CCT GAC GAG TGG GCG GAC TTC        1795
   Pro Asn Arg Gly Gly Pro Gly Gly Gly Pro Asp Glu Trp Ala Asp Phe
       550                 555                     560
   GGC TTC GAC CTG CCC GAC TGC AAG GCC CGC AAG CAG CCC ATC AAG GAG        1843
   Gly Phe Asp Leu Pro Asp Cys Lys Ala Arg Lys Gln Pro Ile Lys Glu
   565                     570                 575                 580
   GAG TTC ACG GAG GCC GAG ATC CAC TGA                                    1870
   Glu Phe Thr Glu Ala Glu Ile His
                   585
(2) INFORMATION POUR LA SEQ ID NO: 17:
   (i) CARACTERISTIQUES DE LA SEQUENCE:
      (A) LONGUEUR: 588 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 17:
   Met Asp Val Phe His Leu Glu Gly Met Thr Thr Ser Val Met Ala Gln
   1               5                   10                  15
   Phe Asn Leu Leu Ser Ser Thr Met Asp Gln Met Ser Ser Arg Ala Ala
               20                  25                  30
   Ser Ala Ser Pro Tyr Thr Pro Glu His Ala Ala Ser Val Pro Thr His
           35                  40                  45
   Ser Pro Tyr Ala Gln Pro Ser Ser Thr Phe Asp Thr Met Ser Pro Ala
       50                  55                  60
   Pro Val Ile Pro Ser Asn Thr Asp Tyr Pro Gly Pro His His Phe Glu
   65                  70                  75                  80
   Val Thr Phe Gln Gln Ser Ser Thr Ala Lys Ser Ala Thr Trp Thr Tyr
                   85                  90                  95
   Ser Pro Leu Leu Lys Lys Leu Tyr Cys Gln Ile Ala Lys Thr Cys Pro
               100                 105                 110
   Ile Gln Ile Lys Val Ser Thr Pro Pro Pro Pro Gly Thr Ala Ile Arg
           115                 120                 125
   Ala Met Pro Val Tyr Lys Lys Ala Glu His Val Thr Asp Val Val Lys
       130                 135                 140
   Arg Cys Pro Asn His Glu Leu Gly Arg Asp Phe Asn Glu Gly Gln Ser
   145                 150                 155                 160
   Ala Pro Ala Ser His Leu Ile Arg Val Glu Gly Asn Asn Leu Ser Gln
                   165                 170                 175
   Tyr Val Asp Asp Pro Val Thr Gly Arg Gln Ser Val Val Val Pro Tyr
               180                 185                 190
   Glu Pro Pro Gln Val Gly Thr Glu Phe Thr Thr Ile Leu Tyr Asn Phe
           195                 200                 205
   Met Cys Asn Ser Ser Cys Val Gly Gly Met Asn Arg Arg Pro Ile Leu
       210                 215                 220
   Ile Ile Ile Thr Leu Glu Met Arg Asp Gly Gln Val Leu Gly Arg Arg
   225                 230                 235                 240
   Ser Phe Glu Gly Arg Ile Cys Ala Cys Pro Gly Arg Asp Arg Lys Ala
                   245                 250                 255
   Asp Glu Asp His Tyr Arg Glu Gln Gln Ala Leu Asn Glu Ser Ser Ala
               260                 265                 270
   Lys Asn Gly Ala Ala Ser Lys Arg Ala Phe Lys Gln Ser Pro Pro Ala
           275                 280                 285
   Val Pro Ala Leu Gly Ala Gly Val Lys Lys Arg Arg His Gly Asp Glu
       290                 295                 300
   Asp Thr Tyr Tyr Leu Gln Val Arg Gly Arg Glu Asn Phe Glu Ile Leu
   305                 310                 315                 320
   Met Lys Leu Lys Glu Ser Leu Glu Leu Met Glu Leu Val Pro Gln Pro
                   325                 330                 335
   Leu Val Asp Ser Tyr Arg Gln Gln Gln Gln Leu Leu Gln Arg Pro Ser
               340                 345                 350
   His Leu Gln Pro Pro Ser Tyr Gly Pro Val Leu Ser Pro Met Asn Lys
           355                 360                 365
   Val His Gly Gly Met Asn Lys Leu Pro Ser Val Asn Gln Leu Val Gly
       370                 375                 380
   Gln Pro Pro Pro His Ser Ser Ala Ala Thr Pro Asn Leu Gly Pro Val
   385                 390                 395                 400
   Gly Pro Gly Met Leu Asn Asn His Gly His Ala Val Pro Ala Asn Gly
                   405                 410                 415
   Glu Met Ser Ser Ser His Ser Ala Gln Ser Met Val Ser Gly Ser His
               420                 425                 430
   Cys Thr Pro Pro Pro Pro Tyr His Ala Asp Pro Ser Leu Val Ser Phe
           435                 440                 445
   Leu Thr Gly Leu Gly Cys Pro Asn Cys Ile Glu Tyr Phe Thr Ser Gln
       450                 455                 460
   Gly Leu Gln Ser Ile Tyr His Leu Gln Asn Leu Thr Ile Glu Asp Leu
   465                 470                 475                 480
   Gly Ala Leu Lys Ile Pro Glu Gln Tyr Arg Met Thr Ile Trp Arg Gly
                   485                 490                 495
   Leu Gln Asp Leu Lys Gln Gly His Asp Tyr Ser Thr Ala Gln Gln Leu
               500                 505                 510
   Leu Arg Ser Ser Asn Ala Ala Thr Ile Ser Ile Gly Gly Ser Gly Glu
           515                 520                 525
   Leu Gln Arg Gln Arg Val Met Glu Ala Val His Phe Arg Val Arg His
       530                 535                 540
   Thr Ile Thr Ile Pro Asn Arg Gly Gly Pro Gly Gly Gly Pro Asp Glu
   545                 550                 555                 560
   Trp Ala Asp Phe Gly Phe Asp Leu Pro Asp Cys Lys Ala Arg Lys Gln
                   565                 570                 575
   Pro Ile Lys Glu Glu Phe Thr Glu Ala Glu Ile His
               580                 585
(2) INFORMATION POUR LA SEQ ID NO: 18:
   (i) CARACTERISTIQUES DE LA SEQUENCE:
      (A) LONGUEUR: 1817 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (A) ORGANISME: Homo sapiens
   (xi) DESCRIPTION DE LA SEQUENCE: SEQ ID NO: 18:
   ATGGCCCAGT CCACCGCCAC CTCCCCTGAT GGGGGCACCA CGTTTGAGCA CCTCTGGAGC 60
   TCTCTGGAAC CAGACAGCAC CTACTTCGAC CTTCCCCAGT CAAGCCGGGG GAATAATGAG 120
   GTGGTGGGCG GAACGGATTC CAGCATGGAC GTCTTCCACC TGGAGGGCAT GACTACATCT 180
   GTCATGGCCC AGTTCAATCT GCTGAGCAGC ACCATGGACC AGATGAGCAG CCGCGCGGCC 240
   TCGGCCAGCC CCTACACCCC AGAGCACGCC GCCAGCGTGC CCACCCACTC GCCCTACGCA 300
   CAACCCAGCT CCACCTTCGA CACCATGTCG CCGGCGCCTG TCATCCCCTC CAACACCGAC 360
   TACCCCGGAC CCCACCACTT TGAGGTCACT TTCCAGCAGT CCAGCACGGC CAAGTCAGCC 420
   ACCTGGACGT ACTCCCCGCT CTTGAAGAAA CTCTACTGCC AGATCGCCAA GACATGCCCC 480
   ATCCAGATCA AGGTGTCCAC CCCGCCACCC CCAGGCACTG CCATCCGGGC CATGCCTGTT 540
   TACAAGAAAG CGGAGCACGT GACCGACGTC GTGAAACGCT GCCCCAACCA CGAGCTCGGG 600
   AGGGACTTCA ACGAAGGACA GTCTGCTCCA GCCAGCCACC TCATCCGCGT GGAAGGCAAT 660
   AATCTCTCGC AGTATGTGGA TGACCCTGTC ACCGGCAGGC AGAGCGTCGT GGTGCCCTAT 720
   GAGCCACCAC AGGTGGGGAC GGAATTCACC ACCATCCTGT ACAACTTCAT GTGTAACAGC 780
   AGCTGTGTAG GGGGCATGAA CCGGCGGCCC ATCCTCATCA TCATCACCCT GGAGATGCGG 840
   GATGGGCAGG TGCTGGGCCG CCGGTCCTTT GAGGGCCGCA TCTGCGCCTG TCCTGGCCGC 900
   GACCGAAAAG CTGATGAGGA CCACTACCGG GAGCAGCAGG CCCTGAACGA GAGCTCCGCC 960
   AAGAACGGGG CCGCCAGCAA GCGTGCCTTC AAGCAGAGCC CCCCTGCCGT CCCCGCCCTT 1020
   GGTGCCGGTG TGAAGAAGCG GCGGCATGGA GACGAGGACA CGTACTACCT TCAGGTGCGA 1080
   GGCCGGGAGA ACTTTGAGAT CCTGATGAAG CTGAAAGAGA GCCTGGAGCT GATGGAGTTG 1140
   GTGCCGCAGC CACTGGTGGA CTCCTATCGG CAGCAGCAGC AGCTCCTACA GAGGCCGAGT 1200
   CACCTACAGC CCCCGTCCTA CGGGCCGGTC CTCTCGCCCA TGAACAAGGT GCACGGGGGC 1260
   ATGAACAAGC TGCCCTCCGT CAACCAGCTG GTGGGCCAGC CTCCCCCGCA CAGTTCGGCA 1320
   GCTACACCCA ACCTGGGGCC CGTGGGCCCC GGGATGCTCA ACAACCATGG CCACGCAGTG 1380
   CCAGCCAACG GCGAGATGAG CAGCAGCCAC AGCGCCCAGT CCATGGTCTC GGGGTCCCAC 1440
   TGCACTCCGC CACCCCCCTA CCACGCCGAC CCCAGCCTCG TCAGGACCTG GGGGCCCTGA 1500
   AGATCCCCGA GCAGTACCGC ATGACCATCT GGCGGGGCCT GCAGGACCTG AAGCAGGGCC 1560
   ACGACTACAG CACCGCGCAG CAGCTGCTCC GCTCTAGCAA CGCGGCCACC ATCTCCATCG 1620
   GCGGCTCAGG GGAACTGCAG CGCCAGCGGG TCATGGAGGC CGTGCACTTC CGCGTGCGCC 1680
   ACACCATCAC CATCCCCAAC CGCGGCGGCC CAGGCGGCGG CCCTGACGAG TGGGCGGACT 1740
   TCGGCTTCGA CCTGCCCGAC TGCAAGGCCC GCAAGCAGCC CATCAAGGAG GAGTTCACGG 1800
   AGGCCGAGAT CCACTGA 1817
(2) INFORMATION POUR LA SEQ ID NO: 19:
   (i) CARACTERISTIQUES DE LA SEQUENCE:
      (A) LONGUEUR: 499 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 19:
   Met Ala Gln Ser Thr Ala Thr Ser Pro Asp Gly Gly Thr Thr Phe Glu
   1               5                   10                  15
   His Leu Trp Ser Ser Leu Glu Pro Asp Ser Thr Tyr Phe Asp Leu Pro
               20                  25                  30
   Gln Ser Ser Arg Gly Asn Asn Glu Val Val Gly Gly Thr Asp Ser Ser
           35                  40                  45
   Met Asp Val Phe His Leu Glu Gly Met Thr Thr Ser Val Met Ala Gln
       50                  55                  60
   Phe Asn Leu Leu Ser Ser Thr Met Asp Gln Met Ser Ser Arg Ala Ala
   65                  70                  75                  80
   Ser Ala Ser Pro Tyr Thr Pro Glu His Ala Ala Ser Val Pro Thr His
                   85                  90                  95
   Ser Pro Tyr Ala Gln Pro Ser Ser Thr Phe Asp Thr Met Ser Pro Ala
               100                 105                 110
   Pro Val Ile Pro Ser Asn Thr Asp Tyr Pro Gly Pro His His Phe Glu
           115                 120                 125
   Val Thr Phe Gln Gln Ser Ser Thr Ala Lys Ser Ala Thr Trp Thr Tyr
       130                 135                 140
   Ser Pro Leu Leu Lys Lys Leu Tyr Cys Gln Ile Ala Lys Thr Cys Pro
   145                 150                 155                 160
   Ile Gln Ile Lys Val Ser Thr Pro Pro Pro Pro Gly Thr Ala Ile Arg
                   165                 170                 175
   Ala Met Pro Val Tyr Lys Lys Ala Glu His Val Thr Asp Val Val Lys
               180                 185                 190
   Arg Cys Pro Asn His Glu Leu Gly Arg Asp Phe Asn Glu Gly Gln Ser
           195                 200                 205
   Ala Pro Ala Ser His Leu Ile Arg Val Glu Gly Asn Asn Leu Ser Gln
       210                 215                 220
   Tyr Val Asp Asp Pro Val Thr Gly Arg Gln Ser Val Val Val Pro Tyr
   225                 230                 235                 240
   Glu Pro Pro Gln Val Gly Thr Glu Phe Thr Thr Ile Leu Tyr Asn Phe
                   245                 250                 255
   Met Cys Asn Ser Ser Cys Val Gly Gly Met Asn Arg Arg Pro Ile Leu
               260                 265                 270
   Ile Ile Ile Thr Leu Glu Met Arg Asp Gly Gln Val Leu Gly Arg Arg
           275                 280                 285
   Ser Phe Glu Gly Arg Ile Cys Ala Cys Pro Gly Arg Asp Arg Lys Ala
       290                 295                 300
   Asp Glu Asp His Tyr Arg Glu Gln Gln Ala Leu Asn Glu Ser Ser Ala
   305 310 315 320
   Lys Asn Gly Ala Ala Ser Lys Arg Ala Phe Lys Gln Ser Pro Pro Ala
   325 330 335
   Val Pro Ala Leu Gly Ala Gly Val Lys Lys Arg Arg His Gly Asp Glu
   340 345 350
   Asp Thr Tyr Tyr Leu Gln Val Arg Gly Arg Glu Asn Phe Glu Ile Leu
   355 360 365
   Met Lys Leu Lys Glu Ser Leu Glu Leu Met Glu Leu Val Pro Gln Pro
   370 375 380
   Leu Val Asp Ser Tyr Arg Gln Gln Gln Gln Leu Leu Gln Arg Pro Ser
   385 390 395 400
   His Leu Gln Pro Pro Ser Tyr Gly Pro Val Leu Ser Pro Met Asn Lys
   405 410 415
   Val His Gly Gly Met Asn Lys Leu Pro Ser Val Asn Gln Leu Val Gly
   420 425 430
   Gln Pro Pro Pro His Ser Ser Ala Ala Thr Pro Asn Leu Gly Pro Val
   435 440 445
   Gly Pro Gly Met Leu Asn Asn His Gly His Ala Val Pro Ala Asn Gly
   450 455 460
   Glu Met Ser Ser Ser His Ser Ala Gln Ser Met Val Ser Gly Ser His
   465 470 475 480
   Cys Thr Pro Pro Pro Pro Tyr His Ala Asp Pro Ser Leu Val Arg Thr
   485 490 495
   Trp Gly Pro
(2) INFORMATION POUR LA SEQ ID NO: 20:
   (i) CARACTERISTIQUES DE LA SEQUENCE:
      (A) LONGUEUR: 17 paires de bases
      (B) TYPE: acide nucléique
      (C) NOMBRE DE BRINS: simple
      (D) CONFIGURATION: linéaire
   (ii) TYPE DE MOLECULE: ADN
   (iii) HYPOTHETIQUE: NON
   (iii) ANTI-SENS: NON
   (xi) DESCRIPTION DE LA SEQUENCE: SEQ ID NO: 20:
      GCGAGCTGCC CTCGGAG 17
(2) INFORMATION POUR LA SEQ ID NO: 21:
   (i) CARACTERISTIQUES DE LA SEQUENCE:
      (A) LONGUEUR: 19 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 21:
      GGTTCTGCAG GTGACTCAG 19
(2) INFORMATION POUR LA SEQ ID NO: 22:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: NON
   (xi) DESCRIPTION DE LA SEQUENCE: SEQ ID NO: 22:
      GCCATGCCTG TCTACAAG 18
(2) INFORMATION POUR LA SEQ ID NO: 23:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 23:
      ACCAGCTGGT TGACGGAG 18
(2) INFORMATION POUR LA SEQ ID NO: 24:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: NON
   (xi) DESCRIPTION DE LA SEQUENCE: SEQ ID NO: 24:
      GTCAACCAGC TGGTGGGCCA G 21
(2) INFORMATION POUR LA SEQ ID NO: 25:
   (i) CARACTERISTIQUES DE LA SEQUENCE:
      (A) LONGUEUR: 16 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 25:
      GTGGATCTCG GCCTCC 16
(2) INFORMATION POUR LA SEQ ID NO: 26:
   (i) CARACTERISTIQUES DE LA SEQUENCE:
      (A) LONGUEUR: 17 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: NON
   (xi) DESCRIPTION DE LA SEQUENCE: SEQ ID NO: 26:
      AGGCCGGCGT GGGGAAG 17
(2) INFORMATION POUR LA SEQ ID NO: 27:
   (i) CARACTERISTIQUES DE LA SEQUENCE:
      (A) LONGUEUR: 19 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 27:
      CTTGGCGATC TGGCAGTAG 19
(2) INFORMATION POUR LA SEQ ID NO: 28:
   (i) CARACTERISTIQUES DE LA SEQUENCE:
      (A) LONGUEUR: 17 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: NON
   (xi) DESCRIPTION DE LA SEQUENCE: SEQ ID NO: 28:
      GCGGCCACGA CCGTGAC 17
(2) INFORMATION POUR LA SEQ ID NO: 29:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 29:
      GGCAGCTTGG GTCTCTGG 18
(2) INFORMATION POUR LA SEQ ID NO: 30:
   (1) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: NON
   (xi) DESCRIPTION DE LA SEQUENCE: SEQ ID NO: 30:
      CTGTACGTCG GTGACCCC 18
(2) INFORMATION POUR LA SEQ ID NO: 31:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 31:
      TCAGTGGATC TCGGCCTC 18
(2) INFORMATION POUR LA SEQ ID NO: 32:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: NON
   (xi) DESCRIPTION DE LA SEQUENCE: SEQ ID NO: 32:
      AGGGGACGCA GCGAAACC 18
(2) INFORMATION POUR LA SEQ ID NO: 33:
   (i) CARACTERISTIQUES DE LA SEQUENCE:
      (A) LONGUEUR: 19 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 33:
      CCATCAGCTC CAGGCTCTC 19
(2) INFORMATION POUR LA SEQ ID NO: 34:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 34:
      CCAGGACAGG CGCAGATG 18
(2) INFORMATION POUR LA SEQ ID NO: 35:
   (i) CARACTERISTIQUES DE LA SEQUENCE:
      (A) LONGUEUR: 19 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 35:
      GATGAGGTGG CTGGCTGGA 19
(2) INFORMATION POUR LA SEQ ID NO: 36:
   (i) CARACTERISTIQUES DE LA SEQUENCE:
      (A) LONGUEUR: 19 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 36:
      TGGTCAGGTT CTGCAGGTG 19
(2) INFORMATION POUR LA SEQ ID NO: 37:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: NON
   (xi) DESCRIPTION DE LA SEQUENCE: SEQ ID NO: 37:
      CACCTACTCC AGGGATGC 18
(2) INFORMATION POUR LA SEQ ID NO: 38:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 38:
      AGGAAAATAG AAGCGTCAGT C 21
(2) INFORMATION POUR LA SEQ ID NO: 39:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: NON
   (xi) DESCRIPTION DE LA SEQUENCE: SEQ ID NO: 39:
      CAGGCCCACT TGCCTGCC 18
(2) INFORMATION POUR LA SEQ ID NO: 40:
   (i) CARACTERISTIQUES DE LA SEQUENCE:
      (A) LONGUEUR: 19 paires de bases
      (B) TYPE: acide nucléique
      (C) NOMBRE DE BRINS: simple
      (D) CONFIGURATION: linéaire
   (ii) TYPE DE MOLECULE: ADN
   (iii) ANTI-SENS: OUI
   (xi) DESCRIPTION DE LA SEQUENCE: SEQ ID NO: 40:
      CTGTCCCCAA GCTGATGAG 19

## Description

L'invention concerne de nouvelles séquences d'acides nucléiques de la famille des gènes suppresseurs de tumeurs apparentée avec le géne de la protéine p53, et les séquences protéiques correspondantes.

L'invention concerne également les applications prophylactiques, thérapeutiques et diagnostiques de celles-ci, notamment dans le domaine des pathologies liées aux phénomènes d'apoptose ou de transformation cellulaire.

Les gènes suppresseurs de tumeurs jouent un rôle clef dans la protection contre les phénomènes de cancérisation, et toute modification susceptible d'entrainer la perte de l'un de ces génes, son inactivation ou son dysfonctionnement, peut avoir un caractère oncogène, créant ainsi des conditions favorables au développement d'un cancer.

Les auteurs de la présente invention ont identifié les produits de transcription d'un nouveau gène ainsi que les protéines correspondantes. Ce gène SR-p70 est apparenté au gène suppresseur de tumeur p53, dont activité anti-tumorale est liée à son activité de facteur de transcription et plus spécifiquement aux contrôles exercés sur l'activité des gènes Bax et Bcl-2, instrumentaux dans les mécanismes de mort cellulaire.

La présente invention est donc relative à des protéines purifiées SR-p70.

L'invention concerne également des séquences d'acides nucléiques isolées codant pour lesdites protéines et des oligonucléotides spécifiques obtenues à partir de ces séquences.

Elle vise en outre les vecteurs de clonage et/ou d'expression contenant au moins l'une des séquences nucléotidiques définies ci-dessus, et les cellules hôtes transfectées par ces vecteurs de clonage et/ou d'expression dans des conditions permettant la réplication et/ou l'expression de l'une desdites séquences nucléotidiques.

Les méthodes de production de protéines recombinantes SR-p70 par les cellules hôtes transfectées font également partie de l'invention.

L'invention comprend également des anticorps ou des dérivés d'anticorps spécifiques des protéines définies ci-dessus.

Elle vise en outre des méthodes de détection des cancers, soit par la mesure de l'accumulation des protéines SR-p70 dans les tumeurs selon des techniques d'immuno-histochimie, soit par la mise en évidence dans le sérum de patients d'auto-anticorps dirigés contre ces protéines.

La demande décuit également tout inhibiteur ou activateur de l'activité du SR-p70 par exemple d'interaction protéine-protéine faisant intervenir le SR-p70.

Elle décrit aussi des séquences oligonucléotidiques antisens, spécifiques des séquences d'acides nucléiques ci-dessus, pouvant moduler *in vivo* l'expression du gène SP-p70.

La demande décrit enfin une méthode de thérapie génique dans laquelle des vecteurs tels que par exemple des vecteurs viraux inactivés capables de transférer des séquences codantes pour une protéine selon l'invention sont injectés à des cellules déficientes pour cette protéine, à des fins de régulation des phénomènes d'apoptose ou de réversion de la transformation.

La présente invention a pour objet un polypeptide purifié comprenant une séquence d'acides aminés choisie parmi :
a) la séquence SEQ ID n° 2 ;
b) la séquence SEQ ID n° 4 ;
c) la séquence SEQ ID n° 6 ;
d) la séquence SEQ ID n° 8 ;
e) la séquence SEQ ID n° 10 :
f) la séquence SEQ ID n° 13 ;
g) la séquence SEQ ID n° 15 ;
h) la séquence SEQ ID n° 17 ;
i) la séquence SEQ ID n° 19.

La demamde décrit en outre une séquence biologiquement active dérivée de SEQ ID n° 2. SEQ ID n° 4, SEQ ID n° 6, SEQ ID n° 8, SEQ ID n° 10, SEQ ID n° 13, SEQ ID n° 15, SEQ ID n° 17 ou SEQ ID n° 19.

Dans la description, on utilise les définitions suivantes :
- protéine SR-p70 : un polypeptide comprenant une séquence d'acides aminés choisie parmi les séquences SEQ ID n° 2, SEQ ID n° 4, SEQ ID n° 6, SEQ ID n° 8, SEQ ID n° 10, SEQ ID n°13, SEQ ID n° 15, SEQ ID n° 17 ou SEQ ID n° 19.
- dérivé : tout polypeptide variant du polypeptide de séquence SEQ ID n°2, SEQ ID n° 4, SEQ ID n° 6, SEQ ID n° 8, SEQ ID n° 10, SEQ ID n° 13, SEQ ID n° 15, SEQ ID n° 17 ou SEQ ID n° 19 ou toute molécule résultant d'une modification de nature génétique et/ou chimique de la séquence SEQ ID n° 2, SEQ ID n° 4, SEQ ID n° 6, SEQ ID n° 8, SEQ ID n° 10, SEQ ID n°13, SEQ ID n° 15, SEQ ID n° 17 ou SEQ ID n° 19 c'est-à-dire obtenue par mutation, délétion, addition, substitution et/ou modification chimique d'un seul ou d'un nombre limité d'acides aminés, ainsi que toute séquence isoforme, c'est-à-dire une séquence identique à la séquence SEQ ID n° 2, SEQ ID n° 4, SEQ ID n° 6, SEQ ID n° 8, SEQ ID n° 10, SEQ ID n° 13, SEQ ID n° 15, SEQ ID n° 17 ou SEQ ID n° 19 à l'un de ses fragments ou séquences modifiées, contenant un ou plusieurs acides aminés sous la forme d'énantiomère D, lesdites séquences variantes, modifiées ou isoformes ayant conservé au moins l'une des propnétés les rendant biologiquement actives.
- biologiquement actif : capable de se lier à l'ADN et/ou d'exercer une activité de facteur de transcription et/ou de participer au contrôle du cycle cellulaire, de la différenciation et de l'apoptose et/ou capable d'être reconnu par les anticorps spécifiques du polypeptide de séquence SEQ ID n°2, SEQ ID n°4, SEQ ID n° 6, SEQ ID n° 8, SEQ ID n° 10, SEQ ID n°13, SEQ ID n° 15, SEQ ID n° 17 ou SEQ ID n° 19 et/ou capable d'induire des anticorps qui reconnaissent ce polypeptide.

La fabrication de dérivés peut avoir différents objectifs, dont en particulier celui d'augmenter l'affinité du polypeptide pour l'ADN ou son activité de facteur de transcription, celui d'améliorer ses taux de production, d'augmenter sa résistance à des protéases, de modifier ses activités biologiques ou de lui conférer de nouvelles propriétés pharmaceutiques et/ou biologiques.

Parmi les polypeptides de l'invention, on préfère le polypeptide d'origine humaine, comprenant la séquence SEQ ID n° 6, SEQ ID n° 13, SEQ ID n°15, SEQ ID n°17 ou SEQ ID n°19. Le polypeptide de 636 acides aminés correspondant à la séquence SEQ ID n° 6 est identique à plus de 97 % au polypeptide de séquence SEQ ID n° 2. Le polypeptide de séquence SEQ ID n° 2 et celui de séquence SEQ ID n° 4 sont deux produits d'expression d'un même gène, de même pour les séquences SEQ ID n° 8 et

SEQ ID n° 10 et pour les séquences SEQ ID n° 6, SEQ ID n°13, SEQ ID n° 15, SEQ ID n° 17 et SEQ ID n° 19.

Comme il sera expliqué dans les exemples, le polypeptide de séquence SEQ ID n° 4 correspond à une terminaison prématurée du peptide de séquence SEQ ID n° 2, liée à un épissage alternatif du transcript codant pour le polypeptide de SEQ ID n° 2 le plus long (ARN messager) du gène correspondant. De même chez l'humain, les polypeptides correspondant aux séquences SEQ ID n° 6, SEQ ID n° 13, SEQ ID n°15, SEQ ID n° 17 et SEQ ID n° 19 divergent dans leur composition au niveau des parties. N- et/ou -C terminales et ce consécutif à des épissages alternatifs d'un même transcript primaire. La séquence peptidique N-terminale de la séquence SEQ ID n° 10 est délétée, ce lié à un épissage alternatif de son transcript codant Avantageusement, l'invention vise un polypeptide correspondant au domaine de fixation sur l'ADN de l'un des polypeptides précédents.

Ce domaine correspond à la séquence comprise entre le résidu 110 et le résidu 310 pour les séquences SEQ ID n° 2 ou 6, et entre le résidu 60 et le résidu 260 pour la séquence SEQ ID n° 8.

La présente invention a également pour objet des séquences d'acides nucléiques codant pour une protéine SR-p70.

Plus préférentiellement, l'invention a pour objet une séquence d'acides nucléiques isolée choisie parmi :
a) la séquence SEO ID n° 1 ;
b) la séquence SEQ ID n° 3 ;
c) la séquence SEQ ID n° 5 ;
d) la séquence SEQ ID n°7 ;
e) la séquence SEQ ID n°9 ;
f) la séquence SEQ ID n° 11 ;
g) la séquence SEQ ID n° 12 ;
h) la séquence SEQ ID n° 14 ;
i) la séquence SEQ ID n° 16 ; et
j) la séquence SEQ ID n° 18.

La demande décrit également des séquences d'acides nucléiques capables de s'hybrider spécifiquement à la séquence SEQ ID n° 1, SEQ ID n° 3, SEQ ID n° 5, SEQ ID n°7, SEQ ID n°9, SEQ ID n° 11, SEQ ID n° 12, SEO ID n°14 ou SEO ID n° 16 ou SEQ ID n° 18 ou à leurs séquences complémentaires, ou de s'hybrider spécifiquement à leurs séquences proximales ; et

des séquences dérivées des séquences a), b), c), d), e), f), g), h), i), j) ou k) du fait de la dégénérescence du code génétique.

Selon un mode de réalisation préféré, l'invention a pour objet les séquences nucléotidiques SEQ ID n° 5, SEQ ID n° 12. SEO ID n° 14, SEQ ID n° 16 et SEQ ID n° 18 correspondant respectivement aux ADNc des protéines humaines des séquences SEQ ID n° 6, SEQ ID n° 13, SEQ ID n° 15, SEO ID n° 17 et SEQ ID n° 19.

Les différentes séquences nucléotidiques de l'invention peuvent être d'origine artificielle ou non. Il peut s'agir de séquences d'ADN ou d'ARN, obtenues par criblage de banques de séquences au moyen de sondes élaborées sur la base des séquences SEO ID n° 1, 3, 5, 7, 9, 11, 12, 14, 16 ou 18. De telles banques peuvent être préparées par des techniques classiques de biologie moléculaire, connues de l'homme de l'art.

Les séquences nucléotidiques selon l'invention peuvent également être préparées par synthèse chimique, ou encore par des méthodes mixtes incluant la modification chimique ou enzymatique de séquences obtenues par criblage de banques.

Ces séquences nucléotidiques permettent la réalisation de sondes nucléotldiques, capables de s'hybrider fortement et spécifiquement avec une séquence d'acides nucléiques, d'un ADN génomique ou d'un ARN messager, codant pour un polypeptide selon l'invention. De telles sondes peuvent être utilisées comme outil de diagnositic *in vitro* pour la détection, par des expériences d'hybridation, de transcripts spécifiques des polypeptides de l'invention dans des échantillons biologiques ou pour la mise en évidence de synthèses aberrantes ou d'anomalies génétiques telles que la perte d'hétérozygotie ou le réarrangement génétique, résultant d'un polymorphisme, de mutations ou d'un épissage différent.

Des sondes de l'invention comportent la totalité de la séquence du gène SR-p70 ou de son ADNc contenu par exemple dans un cosmide.

Parmi les sondes les plus courtes, c'est-à-dire d'environ 10 à 20 nucléotides, les conditions d'hybridation appropriées correspondent aux conditions stringentes usuellement utilisées par l'homme de métier.

La température utilisée est de préférence comprise entre Tₘ -5° C à Tₘ -30° C, de préférence encore entre Tₘ -5° C et Tₘ -10° C, Tₘ étant la température de fusion, température à laquelle 50 % des brins d'ADN appariés se séparent.

L'hybridation est de préférence menée dans des solutions à force ionique élevée, telles que notamment des solutions 6 x SSC.

De manière avantageuse, les conditions d'hybridation utilisées sont les suivantes :
- température : 42° C,
- tampon d'hybridation : 6 x SSC, 5 x Oenhart's, 0,1 % SDS,
telles que décrites dans l'exemple III.

Ces sondes selon l'invention sont représentées par les oligonucléotides suivants ou leurs complémentaires :
SEQ ID n° 20 : GCG AGC TGC CCT CGG AG
SEQ ID n° 21 : GGT TCT GCA GGT GAC TCA G
SEQ ID n° 22 : GCC ATG CCT GTC TAC AAG
SEQ ID n° 23 : ACC AGC TGG TTG ACG GAG
SEO ID n° 24 : GTC AAC CAG CTG GTG GGC CAG
SEQ ID n° 25 : GTG GAT CTC GGC CTC C
SEQ ID n° 26 : AGG CCG GCG TGG GGA AG
SEQ ID n° 27 : CTT GGC GAT CTG GCA GTA G
SEQ ID n° 28 : GCG GCC ACG ACC GTG AC
SEQ ID n° 29 : GGC AGC TTG GGT CTC TGG
SEQ ID n° 30 : CTG TAC GTC GGT GAC CCC
SEQ ID n° 31 : TCA GTG GAT CTC GGC CTC
SEQ ID n° 32 : AGG GGA CGC AGC GAA ACC
SEQ ID n° 33: CCA TCA GCT CCA GGC TCT C
SEQ ID n° 34 : CCA GGA CAG GCG CAG ATG
SEQ ID n° 35 : GAT GAG GTG GCT GGC TGG A
SEQ ID n° 36 : TGG TCA GGT TCT GCA GGT G
SEQ ID n° 37 : CAC CTA CTC CAG GGA TGC
SEQ ID n° 38 : AGG AAA ATA GAA GCG TCA GTC
SEQ ID n° 39 : CAG GCC CAC TTG CCT GCC
SEQ ID n° 40 : CTG TCC CCA AGC TGA TGA G

Préférentiellement, les sondes de l'invention sont marquées, préalablement à leur utilisation. Pour cela, plusieurs techniques sont à la portée de l'homme du métier (marquage fluorescent, radioactif, chimioluminescent, enzymatique, etc).

Les méthodes de diagnostic *in vitro* dans lesquelles ces sondes nucléotidiques sont mises en oeuvre, sont incluses dans l'objet de la présente invention.

Ces méthodes concernent par exemple la détection de synthèses anormales (ex. accumulation de produits de transcription) ou d'anomalies génétiques, telles que la perte d'hétérozygotie et le réarrangement génétique, et les mutations ponctuelles au niveau des séquences nucléotidiques d'acides nucléiques codant pour une protéine SR-p70, selon la définition donnée précédemment.

Les séquences nucléotidiques de l'invention sont également utiles pour la fabrication et l'utilisation d'amorces oligonucléotidiques pour des réactions de séquençage ou d'amplification spécifique selon la technique dite de PCR ou toute variante de celle-ci (Ligase Chain Réaction (LCR), ...).

Des paires d'amorces préférées sont constituées par des amorces choisies sur les séquences nucléotidiques : SEQ ID n° 1 : séquence de singe de 2 874 nucléotides et

SEQ ID n°5 : ADNc SR-p70a humain, notamment en amont du codon ATG d'initiation et en aval du codon TGA d'arrêt de traduction.

Avantageusement, ces amorces sont représentées par les couples suivants:
- couple n°1 :
   amorce sens : GCG AGC TGC CCT CGG AG (SEQ ID n° 20)
   amorce antisens : GGT TCT GCA GGT GAC TCA G (SEQ ID n° 21)
- couple n°2 :
   amorce sens : GCC ATG CCT GTC TAC AAG (SEQ ID n°22)
   amorce antisens : ACC AGC TGG TTG ACG GAG (SEQ ID n° 23)
- couple n° 3 :
   amorce sens : GTC AAC CAG CTG GTG GGC CAG (SEQ ID n° 24)
   amorce antisens : GTG GAT CTC GGC CTC C (SEQ ID n° 25)
- couple n° 4 :
   amorce sens: AGG CCG GCG TGG GGA AG (SEQ ID n° 26)
   amorce antisens : CTT GGC GAT CTG GCA GTA G (SEQ ID n° 27)
- couple n° 5 :
   amorce sens : GCG GCC ACG ACC GTG A (SEQ ID n° 28)
   amorce antisens : GGC AGC TTG GGT CTC TGG (SEQ ID n°29)
- couple n° 6 :
   amorce sens: CTG TAC GTC GGT GAC CCC (SEQ ID n°30)
   amorce antisens : TCA GTG GAT CTC GGC CTC (SEQ ID n° 31)
- couple n° 7 :
   amorce sens : AGG GGA CGC AGC GAA ACC (SEQ ID n° 32)
   amorce antisens : GGC AGC TTG GGT CTC TGG (SEQ ID n° 29)
- couple n° 8 :
   amorce sens : CCCCCCCCCCCCCCN (où N est égal à G, A ou T)
   amorce antisens : CCA TCA GCT CCA GGC TCT C (SEQ ID n° 33)
- couple n° 9 :
   amorce sens : CCCCCCCCCCCCCCN (où N est égal à G, A ou T)
   amorce antisens : CCA GGA CAG GCG CAG ATG (SEQ ID n° 34)
- couple n° 10 :
   amorce sens : CCCCCCCCCCCCCCCN (où N est égal à G, A ou T)
   amorce antisens : CTT GGC GAT CTG GCA GTA G (SEQ ID n° 27)
- couple n° 11 :
   amorce sens : CAC CTA CTC CAG GGA TGC (SEQ ID n° 37)
   amorce antisens : AGG AAA ATA GAA GCG TCA GTC (SEQ ID n° 38)
- couple n° 12 :
   amorce sens : CAG GCC CAC TTG CCT GCC (SEQ ID n° 39)
   amorce antisens : CTG TCC CCA AGC TGA TGA G (SEQ ID n° 40)

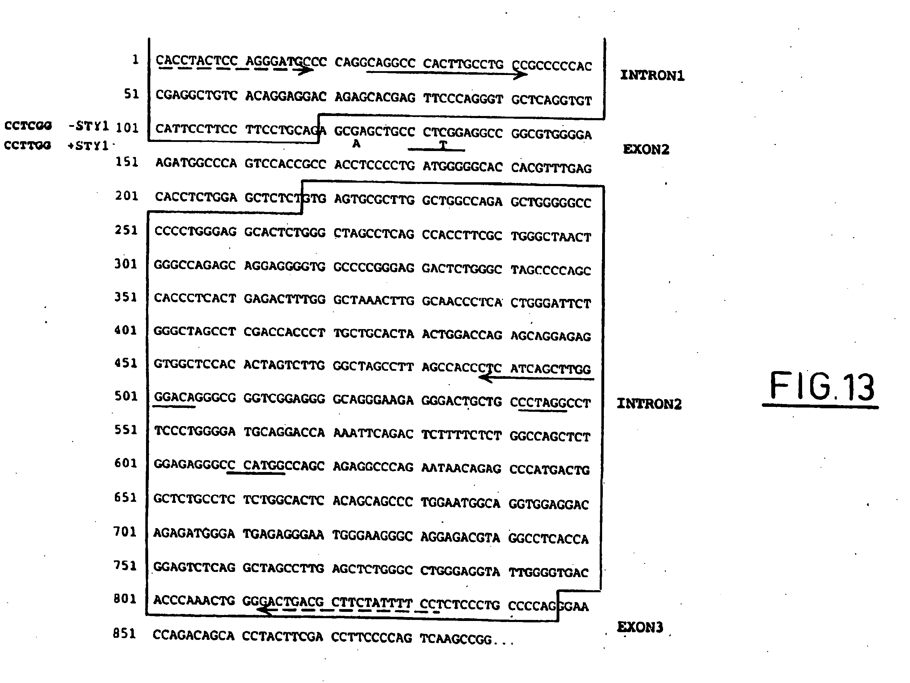

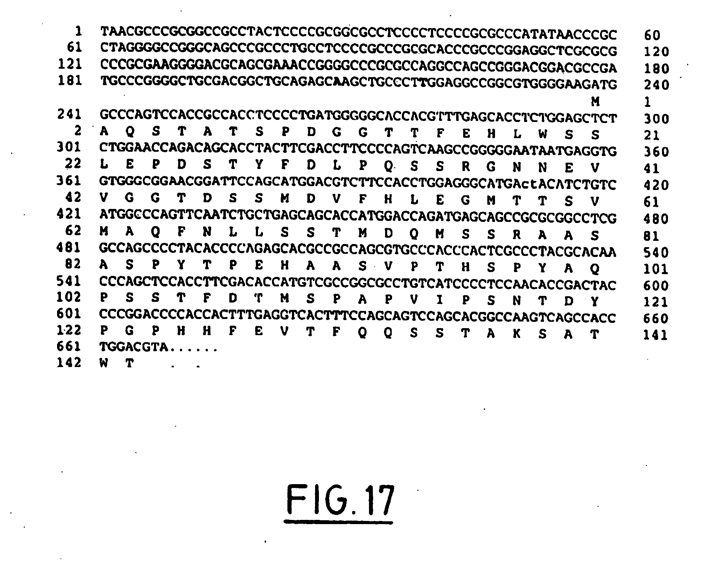

Ces amorces correspondent aux séquences allant respectivement :
- du nucléotide n° 124 au nucléotide n° 140 sur SEQ ID n° 1 et du nucléotide n° 1 au nucléotide n° 17 sur SEQ ID n°5 pour SEQ ID N° 20
- du nucléotide n° 2280 au nucléotide n° 2262 sur SEQ ID n° 1 et du nucléotide n° 2156 au nucléotide 2138 sur SEQ ID n°5 pour SEQ ID N° 21
- du nucléotide n° 684 au nucléotide n° 701 sur SEQ ID n° 1 pour SEQ ID N° 22
- du nucléotide n° 1447 au nucléotide n° 1430 sur SEQ ID n° 1 et du nucléotide 1324 au nucléotide 1307 sur SEQ ID n°5 pour SEQ ID N° 23
- du nucléotide 1434 au nucléotide 1454 sur SEQ ID n°1 et du nucléotide 1311 au nucléotide1331 sur SEQ ID n°5 pour SEQ ID n° 24
- du nucléotide 2066 au nucléotide 2051 sur SEQ ID n°1 et du nucléotide 1940 au nucléotide 1925 sur SEQ ID n°5 pour SEQ ID n°25.
- du nucléotide 16 au nucléotide 32 sur SEQ ID n° 5 pour SEQ ID n° 26
- du nucléotide 503 au nucléotide 485 sur SEQ ID n° 5 pour SEQ ID n° 27
- du nucléotide 160 au nucléotide 176 sur SEQ ID n° 11 pour SEQ ID n° 28
- du nucléotide 1993 au nucléotide 1976 sur SEQ ID n° 5 pour SEQ ID n° 29
- du nucléotide 263 au nucléotide 280 sur SEQ ID n° 11 pour SEQ ID n° 30
- du nucléotide 1943 au nucléotide 1926 sur SEQ ID n° 5 pour SEQ ID n° 31
- du nucléotide 128 au nucléotide 145 sur la séquence nucléotidique représentée à la figure 17 pour SEQ ID n° 32
- du nucléotide 1167 au nucléotide 1149 sur SEQ ID n° 5 pour SEQ ID n° 33
- du nucléotide 928 au nucléotide 911 sur SEQ ID n° 5 pour SEQ ID n° 34
- du nucléotide 677 au nucléotide 659 sur SEQ ID n° 5 pour SEQ ID n° 35
- du nucléotide 1605 au nucléotide 1587 sur SEQ ID n° 5 pour SEQ ID n° 36
- du nucléotide 1 au nucléotide 18 sur la séquence nucléotidique représentée à la figure 13 pour SEQ ID n° 37
- du nucléctide 833 au nucléctide 813 sur la séquence nucléotidique représentée à la figure 13 pour SEQ ID n° 38
- du nucléotide 25 au nucléotide 42 sur la séquences nucléotidique représentée à la figure 13 pour SEQ ID n° 39
- du nucléotide 508 au nucléotide 488 sur la séquence nucléotidique représentée à la figure 13 pour SEQ ID n° 40

Les séquences nucléotidiques selon l'invention peuvent par ailleurs être utilisées pour la production de protéines recombinantes SR-p70, selon la définition qui a été donnée à ce terme.

Ces protéines peuvent être produites à partir des séquences nucléotidiques définies ci-dessus, selon des techniques de production de produits recombinants connues de l'homme du métier. Dans ce cas, la séquences nucléotidique utilisée est placée sous le contrôle de signaux permettant son expression dans un hôte cellulaire.

Un système efficace de production d'une protéine recombinant nécessite de disposer d'un vecteur, par exemple d'origine plasmidique ou virale, et d'une cellule hôte compatible.

L'hôte cellulaire peut être choisi parmi des systèmes procaryotes, comme les bactéries, ou eucaryotes, comme par exemple les levures, cellules d'insectes, CHO (cellules d'ovaires de hamster chinois) ou tout autre système avantageusement disponible. Un hôte cellulaire préféré pour l'expression des protéines de l'invention est constitué par la bactérie *E. coli*, notamment la souche MC 1061 (Clontec).

Le vecteur doit comporter un promoteur, des signaux d'initiation et de terminaison de la traduction, ainsi que les régions appropriées de régulation de la transcription. If doit pouvoir être maintenu de façon stable dans la cellule et peut éventuellement posséder des signaux particuliers spécifiant la sécrétion de la protéine traduite.

Ces différents signaux de contrôle sont choisis en fonction de l'hôte cellulaire utilisés. A cet effet, les séquences nucléotidiques selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation.

Les vecteurs de clonage et/ou d'expression contenant au moins l'une des séquences nucléotidiques définies ci-dessus font également partie de la présente invention..

Un vecteur de clonage et d'expression préféré est le plasmide pSE1 qui comporte à la fois les éléments nécessaires pour son utilisation comme vecteur de clonage dans *E.coli* (origine de réplication dans *E. coli* et gène de résistance à l'ampicilline, provenant du plasmide pTZ 18R), et comme vecteur d'expression dans les cellules animales (promoteur, intron, site de polyadenylation, origine de réplication du virus SV40), ainsi que les éléments permettant sa copie en simple brin dans un but de séquençage (origine de réplication du phage (1).

Les caractéristiques de ce plasmide sont décrites dans la demande EP 0 506 574.

Sa construction, ainsi que l'intégration des ADNc provenant des séquences d'acides nucléiques de l'invention sont par ailleurs décrites dans les exemples ci-après.

Selon un mode de réalisation préféré, les protéines de l'invention sont sous forme de protéines de fusion, notamment sous forme de protéine fusionnée avec la glutathione S-transférase (GGT). Un vecteur d'expression désigné dans ce cas est représenté par le vecteur plasmidique pGEX-4T-3 (Pharmacia ref-27.4583).

L'invention vise en outre les cellules hôtes transfectées par ces vecteurs précédents.

Ces cellules peuvent être obtenues par l'introduction dans des cellules hôtes d'une séquence nucléotidique insérée dans un vecteur tel que défini ci-dessus, puis la mise en culture desdites cellules dans des conditions permettant la réplication et/ou l'expression de la séquence nuciéotidique transfectée.

Ces cellules sont utilisables dans une méthode de production d'un polypeptide recombinant de séquence SEQ ID n° 2, SEQ ID n° 4, SEQ ID n° 6. SEQ ID n°8, SEQ ID n°10, SEQ ID n° 12, SEQ ID n° 14, SEQ ID n° 16 ou SEQ ID n° 18.

La méthode de production d'un polypeptide de l'invention sous forme recombinante est elle-méme comprise dans la présente invention, et se **caractérise en ce que** l'on cultive les cellules transfectées dans des conditions permettant l'expression d'un polypeptide recombinant de séquence SEQ ID n° 2, SEQ ID n° 4, SEQ ID n° 6. SEQ ID n°8. SEQ ID n°10, SEQ ID n° 12, SEQ ID n° 14, SEQ ID n° 16 ou SEQ ID n° 18, et que l'on récupère ledit polypeptide recombinant

Les procédés de purification utilises sont connus de l'homme du métier. Le polypeptide recombinant peut être purifié à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps mono ou polyclonaux spécifiques, etc. Une variante préférée consiste à produire un polypeptide recombinant fusionné à une protéine "porteuse" (protéine chimère). L'avantage de ce système est qu'il permet une stabilisation et une diminution de la protéolyse du produit recombinant, une augmentation de la solubilité au cours de la renaturation *in vitro* et/ou une simplification de la purification lorsque le partenaire de fusion possède une affinité pour un ligand spécifique.

Avantageusement, les polypeptides de l'invention sont fusionnés avec la glutathion S-transférase en position N-terminale (système "GST" Pharmacia). Le produit de fusion est dans ce cas détecté et quantifié grâce à l'activité enzymatique de la GST. Le réactif colorimétrique utilisé est un accepteur de gluthation, substrat de la GST. Le produit recombinant est purifié sur un support de chromatographie auquel ont été préalablement couplées des molécules de glutathion.

Les anticorps mono ou polyclonaux capables de reconnaître spécifiquement une protéine SR-p70 selon la définition donnée précédemment font également partie de l'invention. Des anticorps polyclonaux peuvent être obtenus à partir du sérum d'un animal immunisé contre la protéine, produite par exemple par recombinaison génétique suivant la méthode décrite ci-dessus, selon les modes opératoires usuels. Les anticorps monoclonaux peuvent être obtenus selon la méthode classique de culture d'hybridomes décrite par Köhler et Milstein, Nature, 1975, 256, 495-497.

Des anticorps avantageux sont des anticorps dirigés contre la région centrale comprise entre le résidu 110 et le résidu 310 pour les séquences SEQ ID n° 2 ou 6 ou entre le résidu 60 et le résidu 260 pour la séquence SEQ ID n° 8.

Les anticorps selon l'invention sont par exemple des anticorps chimériques, des anticorps humanisés, des fragments Fab et F(ab')2. Ils peuvent également se présenter sous forme d'immunoconjugués ou d'anticorps marqués.

Par ailleurs, outre leur utilisation pour la purification des polypeptides recombinants, les anticorps de l'invention, en particulier les anticorps monoclonaux, peuvent également être utilisés pour la détection de ces polypeptides dans un échantillon biologique.

Ils constituent ainsi un moyen d'analyse immunocytochimique ou immunohistochimique de l'expression de protéines SR-p70 sur des coupes de tissus spécifiques, par exemple par immunofluorescence, marquage à l'or, immunoconjugués enzymatiques.

Ils permettent notamment de mettre en évidence une accumulation anormale de protéines SR-p70 dans certains tissus ou prélèvements biologiques, ce qui les rend utiles pour la détection des cancers ou le suivi de l'évolution ou de la rémission de cancers préexistants.

Plus généralement, les anticorps de l'invention peuvent être avantageusement mis en oeuvre dans toute situation où l'expression d'une protéine SR-p70 doit être observée. L'invention concerne donc également un procédé de diagnostic *in vitro* de pathologies corrélées à une expression ou une accumulation anormale de protéines SR-p70. notamment les phénomènes de cancérisation, à partir d'un prélèvement biologique, **caractérisé en ce que** l'on met en contact au moins un anticorps de l'invention avec ledit prélèvement biologique, dans des conditions permettant la formation éventuelle de complexes immunologiques spécifiques entre une protéine SR-p70 et le ou lesdits anticorps et en ce que l'on détecte les complexes immunologiques spécifiques éventuellement formés.

L'invention concerne également un kit pour le diagnostic *in vitro* d'une expression ou une accumulation anormale de protéines SR-p70 dans un prélèvement biologique et/ou pour la mesure du taux d'expression de celle-ci dans ledit prélèvement comprenant :
- au moins un anticorps spécifique d'une protéine SR-p70, éventuellement fixé sur un support,
- des moyens de révélation de la formation de complexes antigènes/anticorps spécifiques entre une protéine SR-p70 et ledit anticorps et/ou des moyens de quantification de ces complexes.

L'invention vise également une méthode de diagnostic précoce de la formation des tumeurs, par la mise en évidence dans le sérum d'un individu, d'auto-anticorps dirigés contre une protéine SR-p70.

Une telle méthode de diagnostic précoce est **caractérisée en ce que** l'on met en contact un échantillon de sérum prélevé chez un individu avec un polypeptide de l'invention, éventuellement fixé sur un support, dans des conditions permettant la formation de complexes immunologiques spécifiques entre ledit polypeptide et les auto-anticorps éventuellement présents dans l'échantillon de sérum, et en ce que l'on détecte les complexes immunologiques spécifiques éventuellement formés. L'invention a également pour objet une méthode de détermination d'une variabilité allélique, d'une mutation, d'une délétion, d'une insertion, d'une perte d'hétérozygotie ou d'une anomalie génétique du gène SR-p70, pouvant être impliquées dans des pathologies **caractérisée en ce qu**'elle utilise au moins une séquence nucléotidique décnte ci-dessus. Parmi les méthodes de détermination d'une variabilité allélique, d'une mutation, d'une délétion, d'une insertion, d'une perte d'hétérozygotie ou d'une anomalie génétique du gène SR-p70, on préfère la méthode **caractérisée en ce qu**'elle comprend au moins une étape d'amplification par PCR de la séquence nucléique cible du SR-p70 susceptible de présenter un polymorphisme, une mutation, une délétion ou une insertion à l'aide de couple d'amorces de séquences nucléotidiques définies ci-dessus, une étape au cours de laquelle on procède au traitement des produits amplifiés à l'aide d'enzyme de restriction approprié et une étape au cours de laquelle on procède à la détection ou au dosage d'au moins l'un des produits de la réaction enzymatique.

L'invention comprend également des compositions pharmaceutiques comprenant comme principe actif un polypeptide répondant aux définitions précédentes, préférentiellement sous forme soluble, associé à un véhicule pharmaceutiquement acceptable.

De telles compositions offrent une nouvelle approche pour traiter les phénomènes de cancérisation au niveau du contrôle de la multiplication et la différenciation cellulaire.

Préférentiellement, ces compositions peuvent être administrées par voie systémique, de préférence par voie intraveineuse, par voie intramusculaire, intradermique ou par voie orale.

Leurs modes d'administration, posologies et formes galéniques optimaux peuvent être déterminés selon les critères généralement pris en compte dans l'établissement d'un traitement thérapeutique adapté à un patient comme par exemple l'âge ou le poids corporel du patient, la gravité de son état général, la tolérance au traitement et les effets secondaires constatés, etc.

D'autres caractéristiques et avantages de l'Invention apparaissent dans la suite de la description avec les exemples et les figures dont les légendes sont représentées ci-après.

### LEGENDE DES FIGURES

- Figure 1 :: Comparaison nucléique de l'ADNc du SR-p70a de singe (correspondant à SEQ ID n°1) avec la séquence nucléique de l'ADNc de p53 de singe.
- Figure 2 :: Comparaison protéique de SR-p70a de singe avec la protéine p53 de singe (sw : p53-cerae).
- Figure 3 :: Comparaison de la séquence nucléique de l'ADNc de SR-p70a et b de singe (correspondant respectivement à SEQ ID n° 1 et SEQ ID n° 3).
- Figure 4 :: Séquence nucléique et séquence protéique déduite de SR-p70a de singe.
- Figure 5 :: Séquence nucléique partielle et séquence protéique déduite complète de SR-p70b de singe.
- Figure 6 :: Séquence nucléique partielle et séquence protéique complète déduite de SR-p70a humain (correspondant à SEQ ID n° 5).
- Figure 7 :: Séquence nucléique partielle et séquence protéique déduite complète de SR-p70c de souris (correspondant à SEQ ID n° 7).
- Figure 8 :: Séquence nucléique partielle et séquence protéique déduite partielle de SR-p70a de souris (correspondant à SEQ ID n° 9).
- Figure 9 :: Multialignement des protéines déduites des ADNc SR-p70 de singe (a et b), humain (a) et de souris (a et c).
- Figure 10a :: Immunoempreinte de la protéine SR-p70.
- Figure 10b :: Détection de la protéine endogène SR-p70.
- Figure 11 :: Localisation chromosomique du gène SR-p70 humain. Le signal apparaît sur le chromosome 1, dans la région p36.
- Figure 12 :: Structure génomique du gène SR-p70 et comparaison avec celle du gène p53. Les séquences protéiques humaines du SR-p70a (ligne du haut de l'alignement) et de la p53 (ligne du bas) sont morcelées en peptides en fonction des exons respectifs à partir desquels ils sont codés. Les chiffres au niveau des flèches correspondent à la numérotation des exons correspondants.
- Figure 13 :: Séquence génomique humaine du SR-p70 depuis le 3' de l'intron 1 jusqu'au 5' de l'exon 3. Les introns sont encadrés. Aux positions 123 et 133, sont localisées deux positions nucléiques variables (G → A en 123 et C → T en 133). Les sites de restriction de l'enzyme Styl sont soulignés (position 130 dans le cas où il y a présence d'un T au lieu d'un C à la position 133, position 542 et position 610). Les flèches positionnent les amorces nucléiques utilisées dans l'exemple XI.
- Figure 14 :: Comparaison nucléique du 5' des ADNc humains du SR-p70d et du SR-p70a.
- Figure 15:: Multialignement des séquences nucléiques correspondant au SR-p70 humain a, b, d, e, et f.
- Figure 16 :: Multi-alignement des protéines déduites des ADNc SR-p70 humains (a, b, d, e et f).
- Figure 17 :: Séquence nucléique partielle et séquence protéique déduite partielle du SR-p70a humain. Les deux bases en caractères gras correspondent à deux positions variables (voir figure 6). Cette séquence présente une région 5' non codante plus complète que celle présentée dans la figure 6.
- Figure 18 :: Analyse des transcrits SR-p70a après amplification par PCR.
piste M : marqueurs de poids moléculaires "1 kb ladder" (GIBCO-BRL)
piste 1 : lignée HT29
piste 3 : lignée SK-N-AS
piste 5 : lignée UMR-32
piste 7 : lignée U-373 MG
piste 9 : lignée SW 480
piste 11 : lignée CHP 212
piste 13 : lignée SK-N-MC
pistes 2, 4, 6, 8, 10, 12, 14 : témoins négatifs correspondant aux pistes 1, 3, 5, 7, 9, 11 et 13 respectivement (absence de transcriptase inverse dans la réaction RT-PCR).

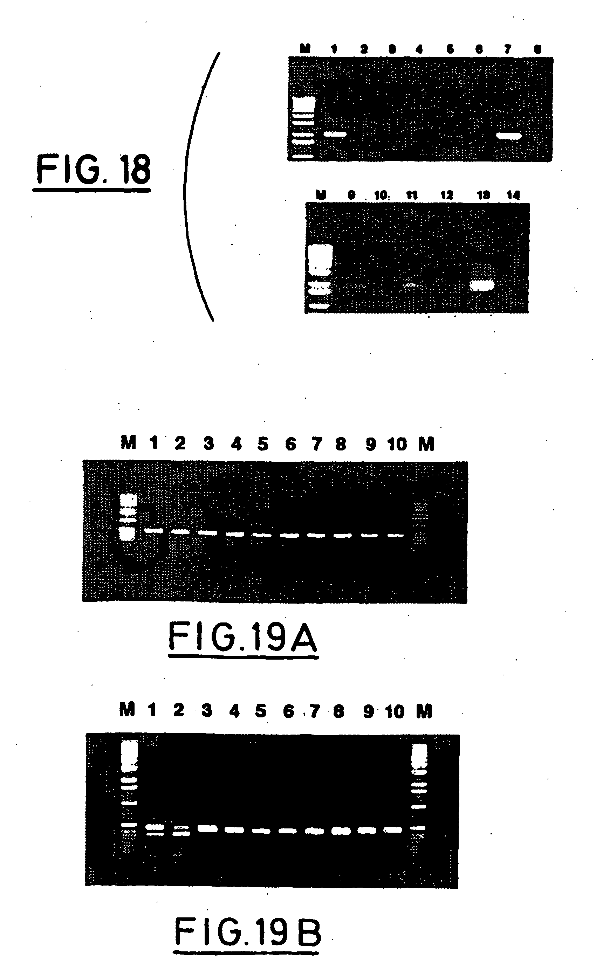

- Figure 19 :: A : Analyse par électrophorèse sur gel d'agarose des fragments génomiques amplifiés par PCR (depuis le 3' de l'intron 1 jusqu'au 5' de l'exon 3). La numérotation des pistes correspond à la numérotation de l'échantillonnage témoin. Piste M : marqueurs de poids moléculaires ("1 kb ladder").
B : Analyse identique à celle de la partie A après une digestion par l'enzyme de restriction Styl des mêmes échantillons.

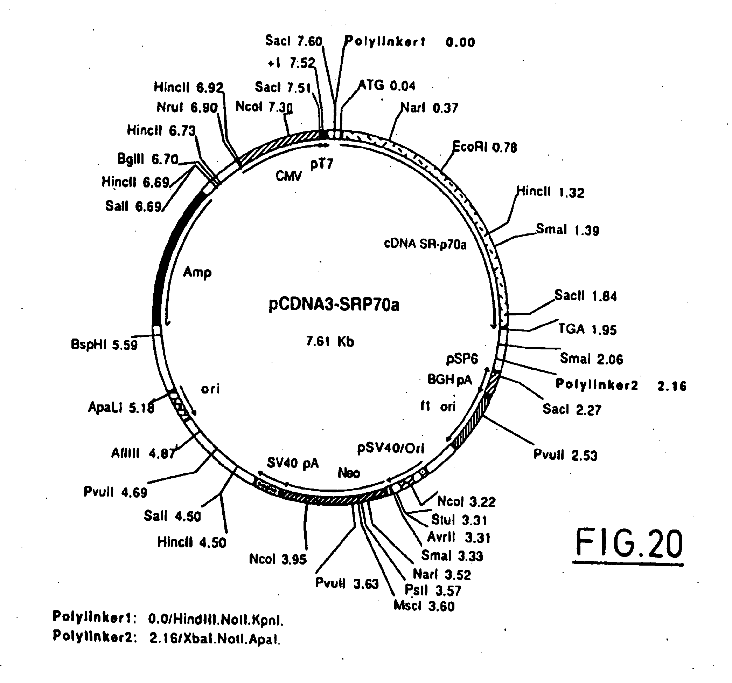

- Figure 20 :: Représentation schématique avec une carte de restriction partielle du plasmide pCDNA3 contenant le SR-p70a humain.

### EXEMPLE I

Clonage de l'ADNc du SR-p70 de cellules COS-3.

### 1. Culture des cellules COS-3

Les cellules COS-3 (cellules de rein de singe vert d'Afrique transformées par l'antigène T du virus SV 40) sont cultivées dans le milieu DMEM (GIBCO-BRL référence 41 965-047) contenant 2 mM de L-glutamine et supplémenté avec 50 mg/l de gentamycine et de 5 % de sérum de bovin foetal (GIBCO-BRL référence 10231-074) jusqu'à semi-confluence.

### 2. Préparation de l'ARN messager

### a) extraction de l'ARN messager

Les cellules sont récupérées de la façon suivante :
- les cellules adhérentes sont lavées deux fois avec du tampon PBS (phosphate buffered saline, référence 04104040-GIBCO-BRL) puis grattées avec un grattoir en caoutchouc et centrifugées.
   Le culot cellulaire est mis en suspension dans le tampon de lyse de composition suivante : guanidine-thiocyanate 4M ; citrate de sodium 25mM pH 7 ; sarcosyl 0,5 % ; β-mercaptoéthanol 0,1 M. La suspension est soniquée à l'aide d'un sonicateur
   Ultra- Turrax n° 231256(Janke et Kundel) à puissance maximale pendant une minute. On ajoute de l'acétate de sodium pH 4 jusqu'à 0,2 M. La solution est extraite avec un volume d'un mélange phénol/chloroforme (v/v ; 5/1). On précipite à -20°C l'ARN contenu dans la phase aqueuse à l'aide d'un volume d'isopropanol. Le culot est resuspendu dans le tampon de lyse. La solution est à nouveau extraite avec un mélange phénol/chloroforme et l'ARN est précipité avec de l'isopropanol. Après lavage du culot avec de l'éthanol 70 % puis 100 %, l'ARN est resuspendu dans de l'eau.

### b) Purification de la fraction poly A⁺ de l'ARN

La purification de la fraction poly A⁺ de l'ARN est réalisée à l'aide du kit Dynabeads oligo (dT)₂₅ de DYNAL (référence 610.05) suivant le protocole préconisé par le fabricant. Le principe est basé sur l'utilisation de billes polystyrène super-paramagnétique sur lesquelles est attaché un oligonucléotide poly(dT)₂₅. La fraction poly A⁺ de l'ARN est hybridée sur l'oligo(dT)₂₅ couplé aux billes que l'on piège sur un support magnétique.

### 3. Constitution de la banque d'ADN complémentaire

### a) préparation de l'ADN complémentaire

A partir de 0,5 µg des ARN-poly A⁺ de cellules COS-3 obtenus à l'issue de l'étape 2, on prépare l'ADN complémentaire simple-brin marqué au ³²P-dCTP (l'ADN complémentaire obtenu présente une activité spécifique de 3000 dpm/ng) avec l'amorce synthétique de séquence suivante (comprenant un site BamHI) :
5'<GATCCGGGCC CTTTTTTTTT TTT<3'
dans un volume de 30 µl de tampon de composition : Tris HCl 50 mM pH 8,3, MgCl₂ 6 mM, DTT 10 mM, KCl 40 mM, contenant 0,5 mM de chacun des désoxynucléotides triphosphates, 30µCi de dCTP α³²P et 30 U de RNasin (promega). Après une heure d'incubation à 37°C, puis 10 minutes à 50°C, puis de nouveau 10 minutes à 37°C, avec 200 unités de l'enzyme transcriptase inverse RNase H⁻ (GISCO-BRL référence 8064A), on ajoute 4 µl d'EDTA.

### b) Hydrolyse alcaline de la matrice ARN

On ajoute 6 µl d'une solution de NaOH 2N, puis on incube pendant 5 minutes à 65° C.

### c) Purification sur colonne sephacryl S400

Afin d'éliminer l'amorce synthétique, on purifie l'ADN complémentaire sur une colonne de 1 ml de sephacryl S400 (Pharmacia), équilibrée dans du tampon TE.

Les deux premières fractions radioactives sont regroupées et précipitées avec 1/10 de volume d'une solution d'acétate d'ammonium 10 M et 2,5 volumes d'éthanol, ceci après une extraction, avec un volume de chloroforme.

### d) Addition homopolyménque de dG

On allonge l'ADN complémentaire en 3' avec une "queue" de dG avec 20 unités de l'enzyme terminale transférase (Pharmacia 27073001). On incube dans 20 µl de tampon de composition : Tris HCl 30 mM pH 7,6 ; chlorure de cobalt 1mM, acide cacodylique 140 mM, DTT 0,1mM, dGTP 1 mM, pendant 15 minutes à 37°C, puis on ajoute 2 µl d'EDTA 0,5 M.

### e) On répète à nouveau les étapes b) et c)

f) Appariement du vecteur de clonage pSE1 (EP 508 574) et de l'ADN complémentaire en présence de l'adaptateur.

On centrifuge, le culot est dissous dans 33 µl de tampon TE, on ajoute 5 µl (125 ng) de vecteur de clonage pSE1, 1 µl(120 ng) de l'adaptateur de séquence suivante (comprenant un site Apal) :
5'AAAAAAAAAAAAAGGGCCCG3'.
10 µl d'une solution de NaCl 200 mM, on incube pendant 5 minutes à 65°C puis on laisse refroidir le mélange réactionnel jusqu'à température ambiante.

### g) Ligation

On ligue le vecteur de clonage et l'ADNc simple brin dans un volume de 100 µl avec 32,5 unités de l'enzyme ADN ligase du phage T4 (Pharmacia référence 270 87002) pendant une nuit à 15°C dans un tampon de composition : Tris HCl 50 mM pH 7,5, MgCl₂ 10 mM, ATP 1 mM.

### h) Synthèse du deuxième brin de l'ADNc

On élimine les protéines par extraction au phénol suivie d'une extraction au chloroforme, puis on ajoute 1/10ème de volume d'une solution d'acétate d'ammonium 10 mM, puis 2,5 volumes d'éthanol. On centrifuge, le culot est dissous dans le tampon de composition Tris acétate 33 mM pH 7,9 acétate de potassium 62,5 mM, acétate de magnésium 1 mM et dithiothréitol (DTT) 1 mM, le deuxième brin d'ADN complémentaire est synthétisé dans un volume de 30 µl avec 30 unités de l'enzyme ADN polymérase du phage T4 (Pharmacia, référence 270718) et un mélange de 1 mM des quatre désoxynucléotides triphosphates de dATP, dCTP, dGTP et dTTP, ainsi que deux unités de la protéine du gène 32 du phage T4 (Pharmacia, référence 27-0213)) pendant une heure à 37°C. On extrait au phénol et on retire les traces de phénol par une colonne de polyacrylamide P10 (Biogel P10-200-400 mesh - référence 15011050 - Biorad).

### i) Transformation par électroporation

On transforme des cellules *E. Coli* MC 1061 avec l'ADN recombinant obtenu précédemment par électroporation à l'aide de l'appareil Biorad Gene Pulser (Biorad) utilisé à 2,5 kV dans les conditions prescrites par le fabricant, puis on fait pousser les bactéries pendant une heure dans du milieu dit milieu LB (Sambrook *op cite*) de composition ; bactotryptone 10 g/l ; extrait de levure 5 g/l ; NaCl 10 g/l.

On détermine le nombre de clones indépendants en étalant une dilution au 1/1000ème de la transformation après la première heure d'incubation sur une boite de milieu LB additionné de 1,5 % d'agar (p/v) et de 100 µg/ml d'ampicilline, appelé par la suite milieu LB gélosé. Le nombre de clones indépendants est de 1 million.

### j) Analyse des ADNc de la banque

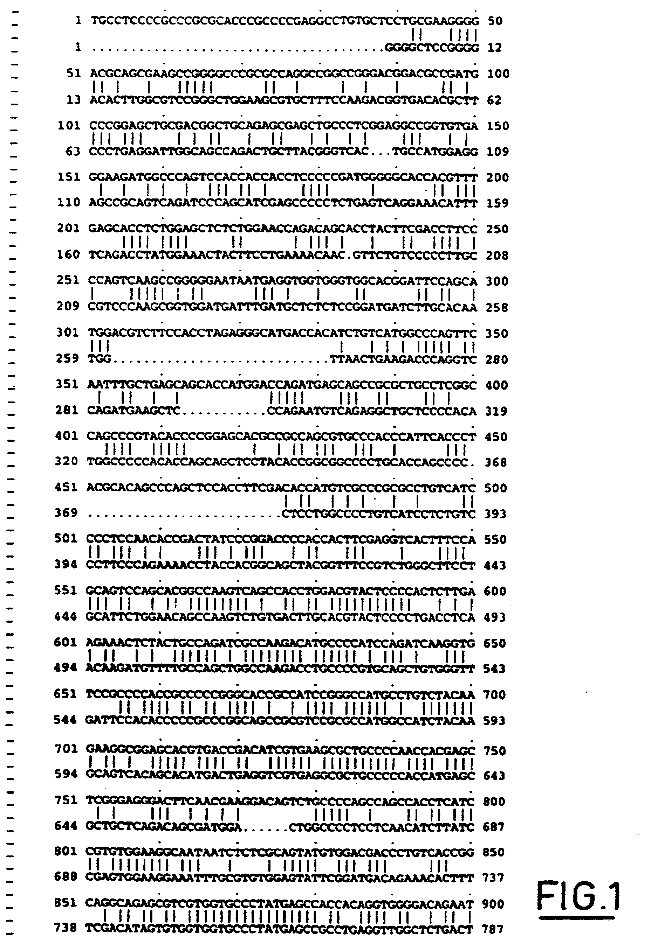

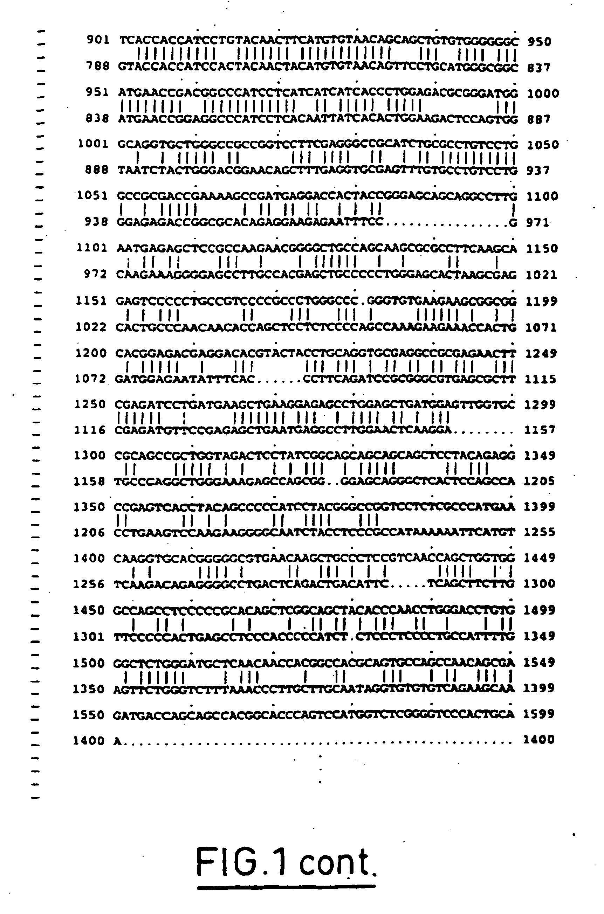

Dans le cadre de l'analyse de clones individualisés de la banque par un séquençage nucléique du 5' des ADNc, un clone, dénommé SR-p70a s'est révélé présenter une homologie partielle avec l'ADNc de la protéine déjà connue, la protéine p53 (Genbank X 02469 et X 16384) (Figure 1). Les séquences ont été réalisées avec le kit United States Biochemical (référence 70770) et/ou le kit Applied Biosystems (références 401434 et/ou 401628) qui utilisent la méthode de Sanger et al., Proc. Natl. Acad. Sci. USA ; 1977, 14, 5463-5467. L'ADN plasmidique est préparé à partir du kit WIZARD mini préparation (Promega référence A7510). Les amorces utilisées sont des oligonucléotides de 16 à 22 mer, complémentaires soit au vecteur pSE1 dans la région immédiatement en 5' de l'AONc, soit à la séquence de l'ADNc.

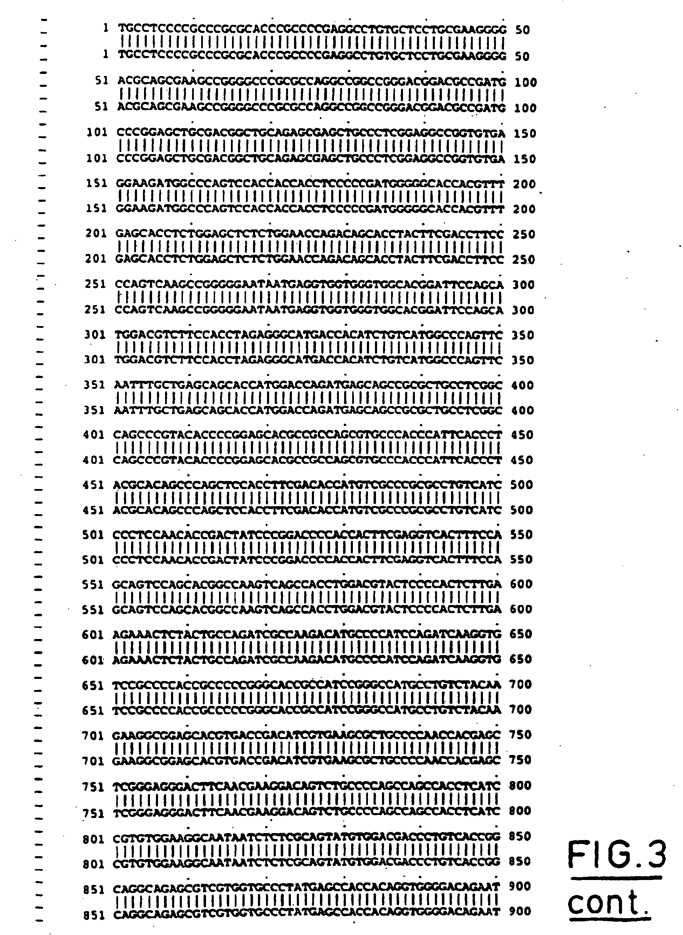

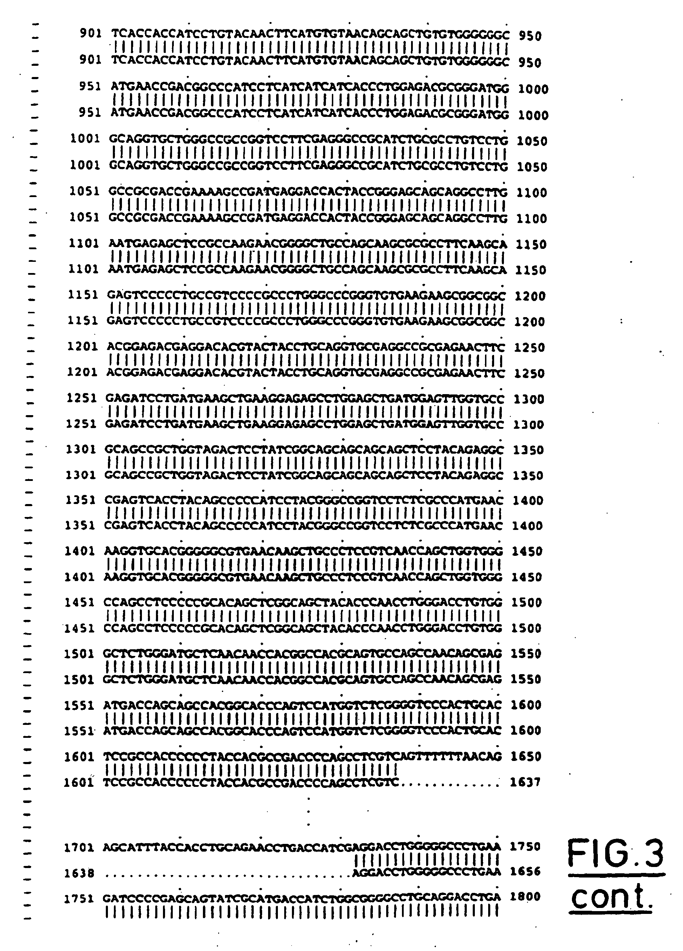

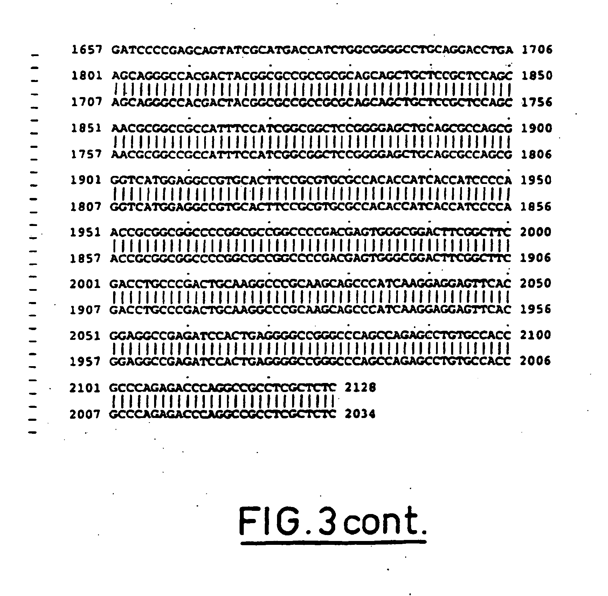

Un second ADNc a été isolé à partir de la même banque en criblant de manière similaire à la technique décrite dans l'EXEMPLE III 3) ci-après avec un fragment de l'ADN SR-p70a marqué au ³²P avec le kit BRL "Random Primers DNA labelling systems" (référence 18187-013). Les tampons d'hybridation et de lavage sont additionnés de 50 % de formamide. Le dernier lavage est réalisé en 0,1 x SSC/SDS 0,1 % à 60°C. Cette seconde séquence (ADNc SR-p70b) est identique à la première mais présente un fragment interne délété (Figure 3).

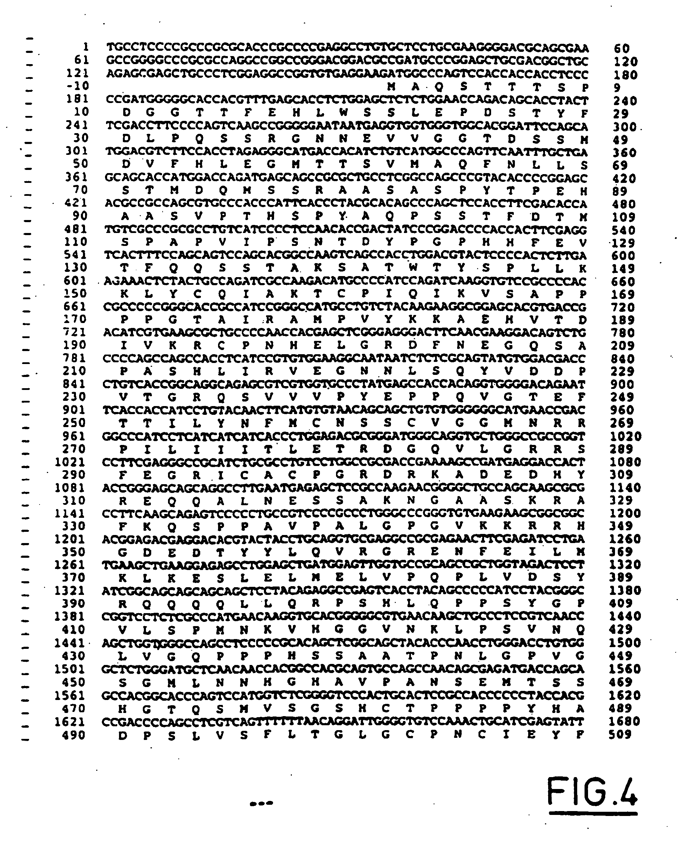

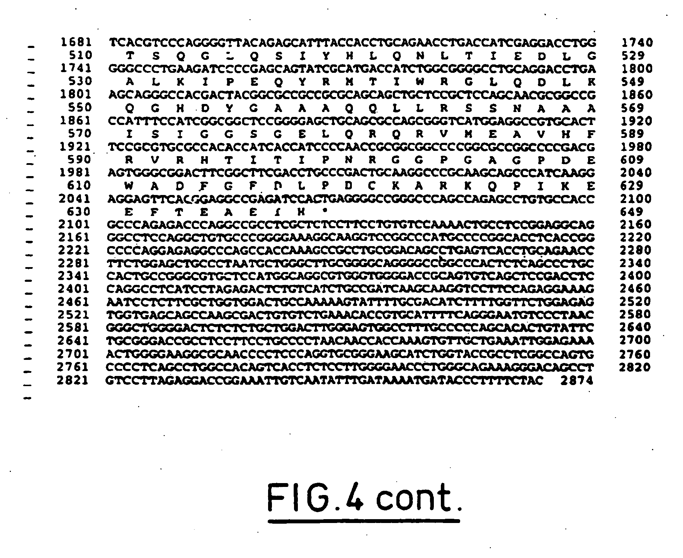

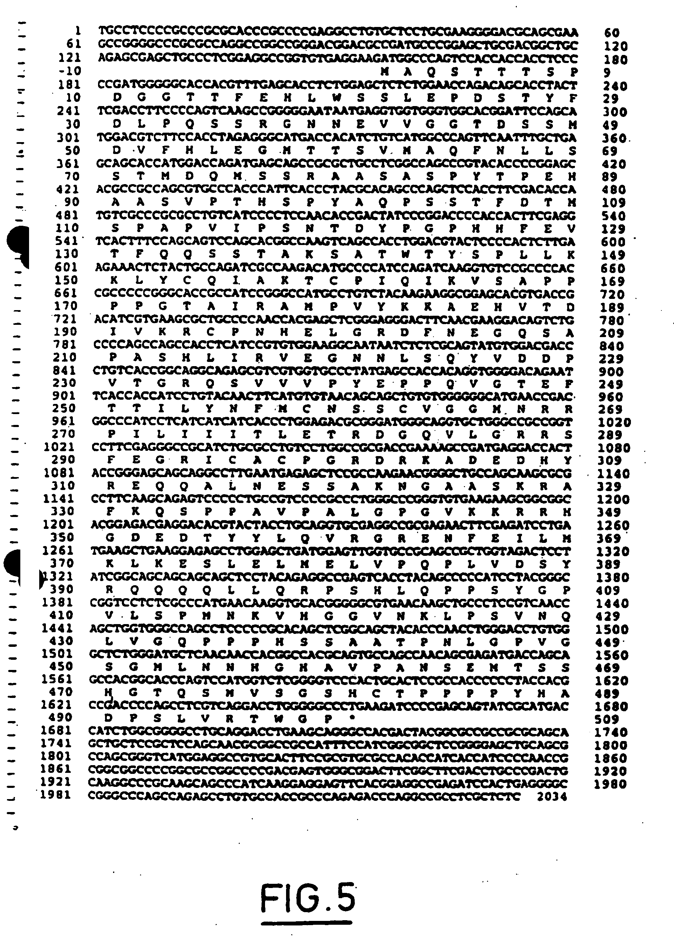

Les deux ADNc SR-p70, d'une longueur de 2874 nucléotides (SR-p70a) et de 2780 nucléotides (SR-p70b) correspondent aux produits d'un seul gène, un épissage alternatif entrainant une déletion de 94 bases entre les nucléotides 1637 et 1732 et une terminaison prématurée de la protéine codée correspondante. Les protéines déduites des deux ADNc présentent respectivement 637 acides aminés et 499 acides aminés (Figures 4 et 5).

### EXEMPLE II

Obtention de la séquence et clonage de l'ADNc de la protéine SR-p70a à partir de cellules HT-29 (Adénocarcinome de colon humain).

### 1) Culture des cellules HT-29

Les cellules sont cultivées en milieu McCoy 5 (GIBCO 26600-023) additionné de 10 % de sérum foetal de veau (GIBCO 10081-23) et 50 mg/l de gentamycine jusqu'à semi-confluence.

### 2) Préparation de l'ADN complémentaire

L'ARN messager est préparé comme décrit dans l'EXEMPLE I.2. L'ADNc est préparé de manière similaire à celle décrite dans L'EXEMPLE I.3 avec 5 µg d'ARN messager total en utilisant une amorce poly(T)₁₂. La réaction n'est pas interrompue avec de l'EDTA.

### 3) Amplification spécifique de l'ADNc humain parla technique dite de PCR

La polymérisation est réalisée avec 4 µl d'ADNc dans 50 µl final avec le tampon de composition suivante : Tris HCl 10 mM pH 8,3, MgCl₂ 2,5 mM, KCl 50 mM en présence de 10 % DMSO, dNTP 0,5 mM, 4 µg/ml de chacune des deux amorces nucléiques et de 2,5 unités de Taq ADN polymérase (Boehringer). Les couples d'amorces ont été choisis sur la séquence nucléique du clone SR-p70 de COS-3, notamment en amont de l'ATG d'initiation et en aval du TGA d'arrêt de traduction et sont de compositions suivantes :
amorce sens : ACT GGT ACC GCG AGC TGC CCT CGG AG
   site de restriction Kpn I
amorce antisens : GAC TCT AGA GGT TCT GCA GGT GAC TCA G.
   site de restriction Xba I

La réaction est réalisée durant 30 cycles 94°C/1 minute, 54-60°C/1 minute 30 secondes et 72°C/1 minute 30 secondes, suivi d'un dernier cycle de 72°C/6 minutes.

### 4) Obtention de la séquence de l'ADNc humain

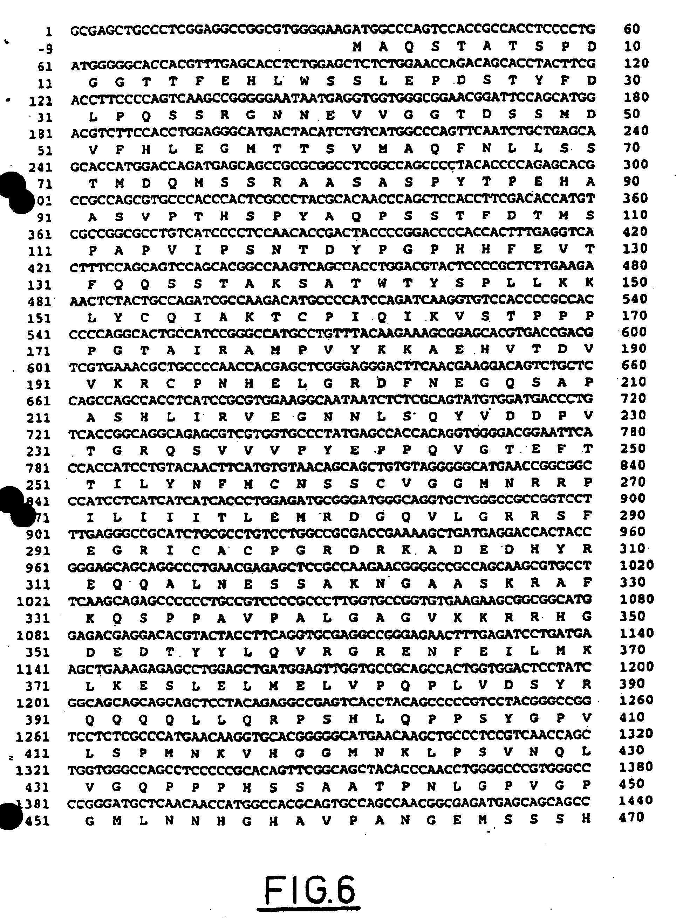

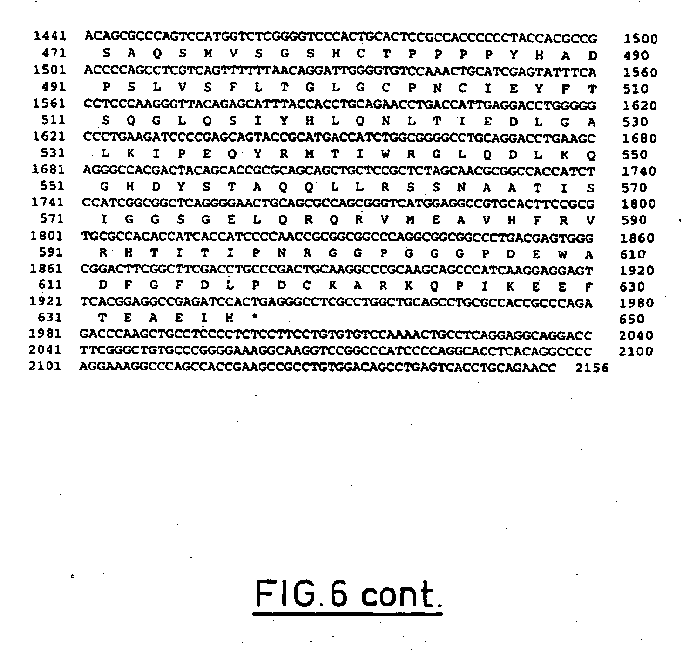

Dans un premier temps, le produit de PCR est éliminé des oligonucléotides sur une colonne de sephacryl S400 puis déssalé par chromatographie d'exclusion sur une colonne de polyacrylamide P10 (Biorad référence 1504144). Les réactions de séquençage sont réalisées à l'aide du kit Applied Biosystems (référence 401628) avec des oligonucléctides spécifiques de l'ADNc. La séquence obtenue est très similaire à celle du SR-p70a de singe et la protéine déduite contient 636 acides aminés (Figure 6).

De manière similaire, d'autres séquences issues de lignées ou de tissus humains ont été obtenues pour la partie codante du SR-p70 humain, notamment à partir du poumon ou du pancréas. Les protéines déduites de ces séquences sont identiques à celles obtenues pour la lignée HT-29.

### 5) Clonage de l'ADNc humain dans le plasmide pCDNA3 (Invitrogen V 790-20)

Le produit PCR obtenu en 3) ainsi que le plasmide sont digérés par les deux enzymes de restriction Kpn I et Xba I puis purifiés après migration sur un gel d'agarose 1 % à l'aide du kit Geneclean (Bio 101 référence 3105). Après ligation avec 100 ng d'insert et 10 ng de vecteur et transformation (technique décrite dans l'EXEMPLE I.3.g et i, les clones recombinants sont vérifiés par séquençage à l'aide du kit Applied Biosystems cité ci-dessus.

### EXEMPLE III

Clonage de l'ADNc du SR-p70 de souris à partir de cellules AtT-20 (tumeur hypophysaire)

### 1) Culture cellulaire de la lignée AtT-20

Les cellules sont cultivées dans du milieu Ham F10 (GIBCO 31550-023) additionné de 15 % de sérum de cheval (GIBCO 26050-047) et de 2,5 % de sérum foetal de veau (GIBCO 10081-073) et de 50 mg/l de gentamycine jusqu'à semi-confluence.

### 2) Préparation de la banque d'ADN complémentaire

La banque est réalisée comme décrit dans l'EXEMPLE 1, 2) et 3) à partir des cellules cultivées ci-dessus.

### 3) Criblage de la banque

### a) Préparation des membranes

Les clones de la banque sont étalés sur du milieu LB gélosé (boites de petri diamètre 150) revêtu de membranes Biodyne A (PALL référence BNNG 132). Après une nuit à 37°C, les clones sont transférés par contact sur de nouvelles membranes. Ces dernières sont traitées en les déposant sur du papier Whatman 3 mm imbibé des solutions suivantes : NaOH 0.5 N, NaCl 1.5 M pendant 5 minutes puis Tris HCl 0.5 M pH 8 , NaCl 1.5 M pendant 5 minutes. Après un traitement à la protéinase K dans le tampon suivant : Tris HCl 10 mM pH 8 , EDTA 10 mM, NaCl 50 mM, SDS 0.1 %, protéinase K 100 µg/ml pendant une heure à température ambiante, les membranes sont lavées abondamment dans du 2 x SSC (sodium citrate NaCl), séchées, puis incubées au four sous vide à 80°C pendant 20 minutes.

### b) Préparation de la sonde

Sur la base de séquences des ADNc SR-p70 de singe et d'humain, une première séquence a été réalisée sur un fragment amplifié à partir de l'ARNm de la lignée AtT-20 comme décrit dans l'EXEMPLE II.3 et 4 avec les oligomères de compositions suivantes :
- amorce sens :: GCC ATG CCT GTC TAC AAG
- amorce antisens :: ACC AGC TGG TTG ACG GAG.

Sur la base de cette séquence, une sonde oligomérique spécifique de souris a été choisie et présente la composition suivante :
GAG CAT GTG ACC GAC ATT G.
100 ng de la sonde sont marqués en 3' avec 10 unités de Terminal Transférase (Pharmacia) et 100 µCi de dCTP α³²P 3000 Ci/mmole (Amersham référence PB 10205) dans 10 µl du tampon suivant : Tris HCl 30 mM pH 7.6 , acide cacodylique 140 mM, COCl₂ 1 mM, DTT 0.1 mM pendant 15 minutes à 37°C, Les nucléotides radiomarqués non incorporés sont éliminés sur une colonne de polyacrylamide P10 (Biorad, référence 1504144). La sonde obtenue a une activité spécifique environ de 5.10⁸ dpm/µg.

### c) Préhybridation et hybridation

Les membranes préparées en a) sont préhybridées 30 minutes à 42°C dans 6 x SSC, 5 x Denhart's, 0,1 % SDS puis hybridées quelques heures dans le même tampon additionné de la sonde préparée en b) à raison de 10⁶ dpm/ml.

### d) Lavage et exposition des membranes

Les membranes sont lavées deux fois à température ambiante dans le tampon 2 x SSC/SDS 0.1 % puis une heure à 56°C en 6 x SSC/SDS 0.1 %. Les clones hybridés sont révélés avec des films KODAK XOMAT. Un clone positif contenant le SR-p70 de souris est sélectionné et dénommé ci-après SR-p70c.

### 4) Séquençage du SR-p70 de souris et analyse de la séquence

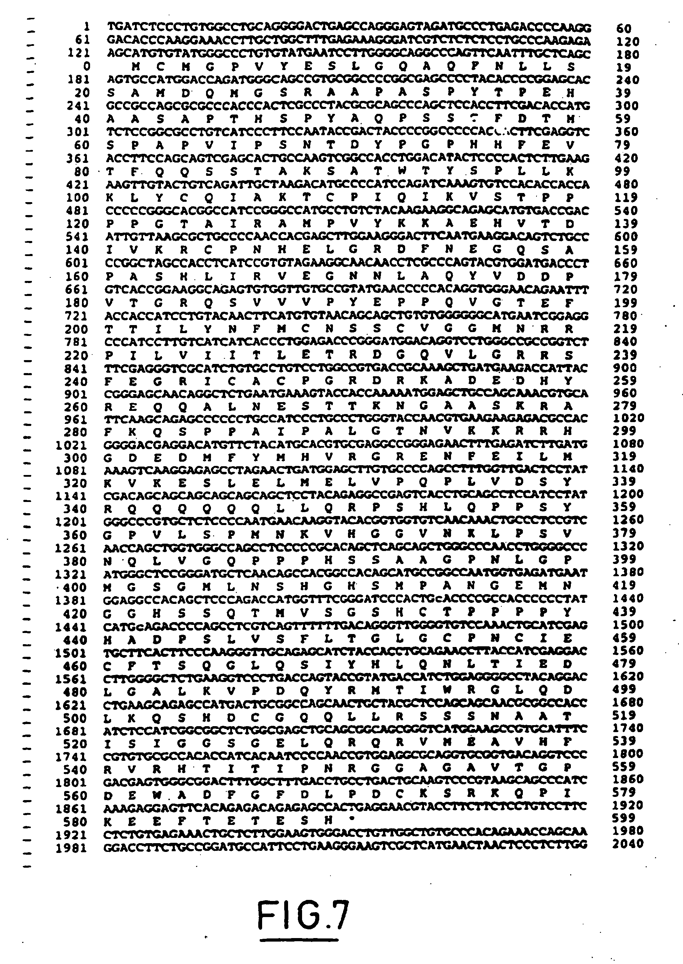

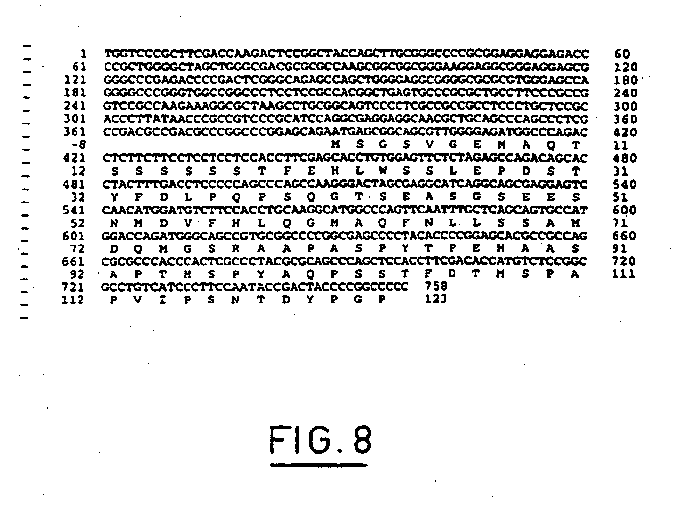

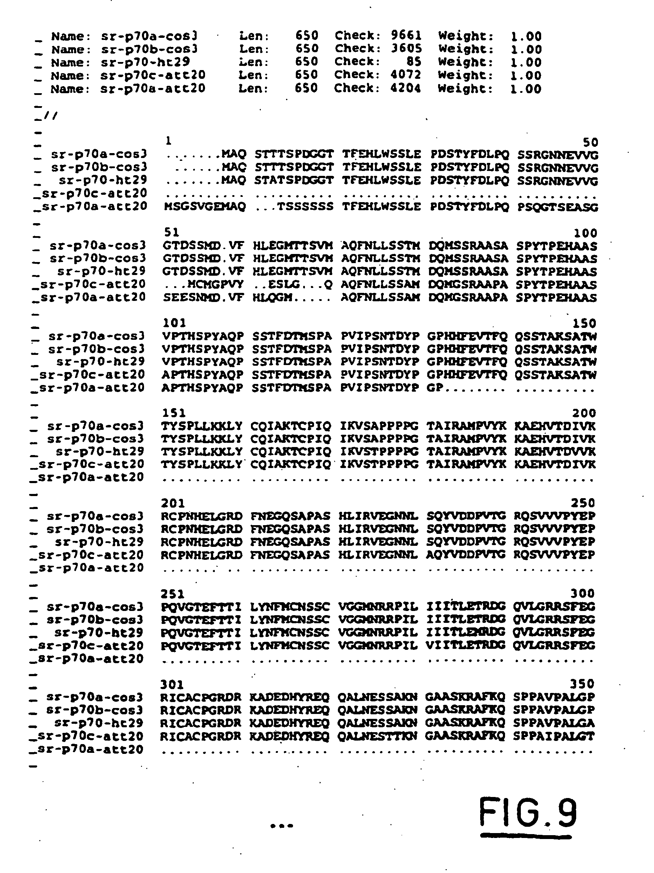

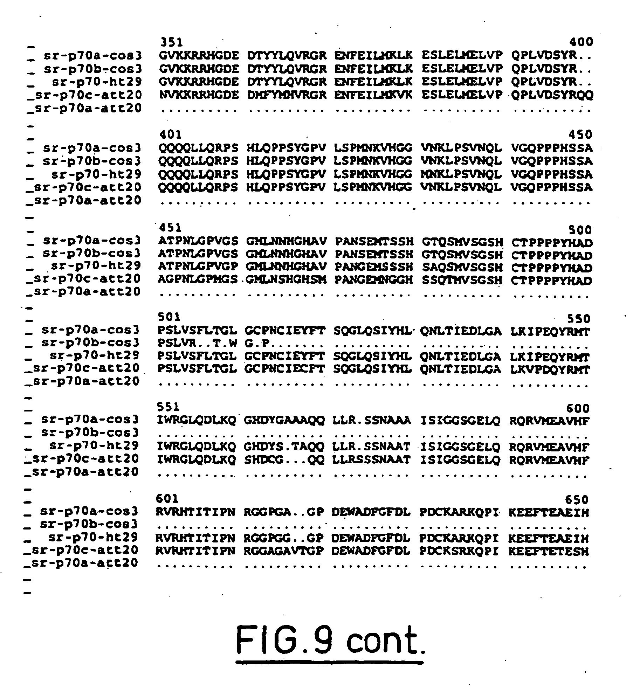

La séquence est obtenue à l'aide du kit Applied Biosystem (référence 401628). La séquence protéique déduite de l'ADNc SR-p70c de souris (Figure 7) présente une très forte homologie avec celles de l'humain et de singe excepté dans la partie N-terminale qui diverge fortement (voir Figure 9). A l'aide de la technique dite de PCR, de manière similaire à celle décrite dans l'EXEMPLE II.3 et 4, une seconde séquence 5' (issue de la même banque AtT-20) a été obtenue (Figure 8). La séquence protéique N-terminale déduite (séquence dénommé SR-p70a) est très similaire à celle déduite des ADNc SR-p70 humain et de singe (SR-p70a) (Figure 9). La lignée AtT-20 présente donc au moins deux transcripts SR-p70. Ces 2 derniers divergent dans la partie N-terminale par des épissages différents.

### EXEMPLE IV

### 1) Production de protéine recombinante SR-p70 dans E. coli

### a) Construction du plasmide d'expression

Elle consiste à mettre la partie -COOH terminale de la protéine SR-p70a de singe, depuis la valine en position 427 à l'histidine -COOH terminale en position 637, en fusion avec la glutathione S-transferase (GST) du vecteur plasmidique pGEX-4T-3 (Pharmacia référence 27-4583). Pour cela, l'insert correspondant de la SR-p70a (position 1434 à 2066) a été amplifié par PCR avec 10 ng de plasmide contenant l'ADNc SR-p70a de singe. Les amorces nucléiques sont de composition suivante :
amorce sens : TTT GGA TCC GTC AAC CAG CTG GTG GGC CAG
   site de restriction BamHI
amorce antisens : AAA GTC GAC GTG GAT CTC GGC CTC C.
site Sal I

Le fragment obtenu ainsi que le vecteur sont digérés par les enzymes de restriction BamHI et Sal I et le clonage est réalisé comme décrit dans l'EXEMPLE II.5. Le clone sélectionné est appelé pG SR-p70.

### b) Expression et purification de la protéine fusion GST-pSR-p70

Cette étape a été réalisée en utilisant le kit "bulk GST purification module" (Pharmacia Référence 27-4570-01).

De manière schématique, le clone recombinant a été mis en culture à 37°C dans un litre de milieu 2x YTA + ampicilline 100 µg/ml. A DO 0,8, l'expression est induite avec 0,5 mM d'IPTG pendant 2 heures à 37°C. Après centrifugation, le culot cellulaire est repris dans du PBS froid puis soniqué par ultrason. Après adjonction de 1 % triton X-100, on incube 30 minutes sous agitation à température ambiante. Après centrifugation à 12 000 g, 10 minutes à 4°C, on récupère le surnageant. La purification est ensuite réalisée sur une colonne de chromatographie d'affinité glutathion sepharose 4B. La fixation et le lavage sont réalisées en tampon PBS et l'élution est réalisée par compétition avec du glutathion réduit. La concentration finale est amenée à 300 µg/ml de protéine fusion.

### 2) Production de protéine SR-p70a dans les cellules COS-3.

Les cellules COS-3 sont transfectées avec de l'ADN plasmidique pSE1 dans lequel a été cloné l'ADNc de SR-p70a de singe (EXEMPLE I.1) ou avec de l'ADN plasmidique du vecteur pSE1 en tant que témoin par la technique du DEAE Dextran : les cellules COS-3 sont ensemencées à 5 x 10⁵ cellules par boite de 6 cm en milieu de culture contenant 5 % de sérum de bovin foetal (EXEMPLE I.1). Après culture, les cellules sont rincées avec du PBS. On ajoute 1 ml du mélange suivant : milieu contenant 6,5 µg d'ADN, 250 µg/ml de DEAE Dextran et 100 µM de chloroquine. Les cellules sont incubées à 37°C en 5 % CO₂ durant 4 à 5 heures. Le milieu est aspiré, on ajoute 2 ml de PBS additionné de 10 % DMSO et les cellules sont incubées pendant une minute en remuant légèrement les boites. Le milieu est à nouveau aspiré et les cellules sont rincées deux fois avec du PBS. Les cellules sont alors incubées à 37°C avec du milieu contenant 2 % de sérum de bovin foetal pendant la durée de l'expression qui est généralement de 3 jours.

La protéine SR-p70a est alors analysée comme décrit dans l'EXEMPLE VI par immunoempreinte.

### EXEMPLE V

### Préparation d'anticorps spécifiques

150 µg de protéines de l'échantillon préparé selon l'EXEMPLE IV ont été utilisés pour immuniser un lapin (mâle de 1,5 à 2 kg environ, New-Zealand). Les immunisations ont été effectuées tous les 15 jours selon le protocole décrit par Vaitukaitis, Methods in Enzymology, 1981, 73, 46. Pour la première injection, un volume de solution antigénique est émulsifié par un volume d'adjuvant complet de Freund (Sigma référence 4258). Cinq rappels ont été administrés en adjuvant incomplet de Freund (Sigma référence 5506).

### EXEMPLE VI

### Détection de la protéine SR-p70 "Western immunoblotting" (immunoempreinte)

### 1) Matériels utilisés pour l'immunoempreinte

### a) Lignées cellulaires utilisées pour l'immunoempreinte.

Les lignées cellulaires suivantes ont été cultivées, comme décrit dans le catalogue «catalogue of cell lines and hybridomas, 7^{th} edition, 1992» de l'ATCC (American Type Culture Collection) : COS-3, CV-1 (lignée de cellules de rein de singe), HT-29, U-373MG (glioblastome humain), MCF7 (adenocarcinome mammaire humain), SKNAS (neuroblastome.humain cultivé dans les mêmes conditions que COS-3), SK-N-MC (neuroblastome humain), IMR-32 (neuroblastome humain), CHP212 (neuroblastome humain cultivé dans les mêmes conditions que CV-1), Saos-2 (ostéosarcome), SK-OV-3 (adénocarcinome d'ovaire) et SW 480 (adénocarcinome de colon humain).

### b) Cellules COS-3 transfectées par l'ADNc SR-p70a.

Les cellules COS-3 ont été transfectées comme décrit dans l'EXEMPLE IV.2. En tant que témoin, les cellules ont été transfectées avec de l'ADN plasmidique pSE1 ne contenant pas l'ADNc recombinant SR-p70a.

### 2) Préparation des échantillons protéiques à partir de culture cellulaire eucaryote ou de cellules transfectées.

Après culture, les cellules sont lavées avec du PBS puis reprises dans un tampon RIPA (PBS avec 1 % NP40, 0,5 % sodium déoxycholate, 0,5 % SDS) complémenté avec 10 µg/ml RNAse A, 20µg/ml ONAse 1, 2 µg/ml aprotinine, 0,5 µg/ml leupeptine, 0,7 µg/ml pepstatine et 170 µg/ml PMSF. Les cellules sont soniquées par ultrason à 4 °C et laissées 30 minutes à 4°C. Après microcentrifugation à 12 000 rpm, on récupère le surnageant. La concentration de protéine est mesurée par la méthode de Bradford.

### 3) "Westerm blotting"

5 ou 50 µg de protéines (50 µg pour les lignées cellulaires et 5 µg pour des cellules transfectées) sont mis dans 0,2 volume du tampon d'électrophorèse 6X suivant : Tris HCl 0,35 mM pH 6,8 10,3 % SDS, 36 % glycérol, 0,6 mM DTT, 0,012 % bleu de bromophénol. Les échantillons sont déposés et mis à migrer dans un gel SDS-Page 10 % (30/0,8 Bis) puis électrotransférés sur une membrane de nitrocellulose.

### 4) Révélation par l'anticorps

La membrane est incubée 30 minutes dans le tampon de blocage TBST (Tris HCl 10 mM pH 8, NaCl 150 mM, 0,2 % Tween 20) additionné de 5 % de lait (GIBCO- SKIM MILK) à température ambiante. La membrane est successivement mise en présence de l'anticorps anti-SR-p70 (αSR-p70) dans le même tampon 16 heures à 4°C, lavée 3 fois pendant 10 minutes avec du TBST, puis incubée une heure à 37°C avec un second anticorps anti-immunoglobuline de lapin couplé avec de la peroxydase (SIGMA A055). Après trois lavages de 15 minutes, la révélation est effectuée en utilisant le kit ECL (Amersham RPN2106) par chimioluminescence.

Parallèlement, les mêmes échantillons ont été révélés par un anticorps anti-p53 (α p53) (sigma BP5312) suivi d'un second anticorps anti-immunoglobine de souris.

### 5) Figures et résultats.

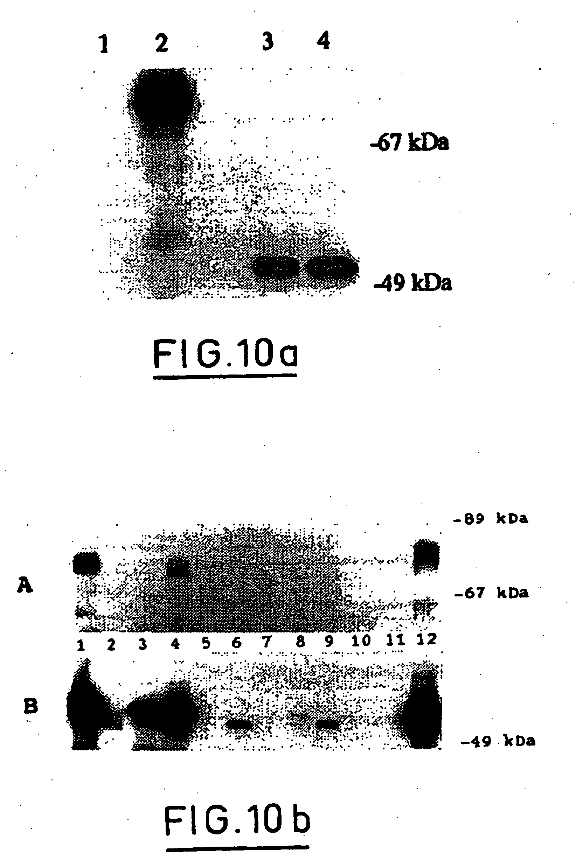

### Figure 10 : Immunoempreinte de la protéine SR-p70

Figure 10a : Détection de la protéine recombinante SR-p70
- colonnes 1 et 3 : COS-3 transfectée par le vecteur pSE1.
- colonnes 2 et 4 : COS-3 transfectée par le plasmide pSE1 contenant l'ADNc du SR-p70a.
- colonnes 1 et 2 : Révélation par l'anticorps anti-SR-p70 (αSR-pT0).
- colonnes 3 et 4 : Révélation par l'anticorps anti-P53 (ap53).
   Figure 10b : Détection de la protéine endogène SR-p70
- colonnes 1 : COS-3 ; 2 : CV-1 ; 3 : HT-29 ; 4 : U-373 MG ; 5 ; MCF7 ; 6 : SKNAS ; 7 : SK-N-MC ; 8 : IMR-32 ; 9 : CHP212 ; 10: Saos-2 ; 11 : SK-OV-3 et 12 : SW480.

A : Révélation par l'anticorps αSR-p70.

B : Révélation par l'anticorps αp53.

L'anticorps αSR-p70 reconnait spécifiquement les protéines recombinantes (Figure 10a) et endogènes (Figure 10b) et ne croise pas avec la p53. L'analyse de lignées cellulaires humaines ou de singe montre que la protéine SR-p70 comme la p53 est généralement faiblement détectable. Par contre, lorsqu'une accumulation de p53 existe, la SR-p70 devient elle aussi plus facilement détectable (Figure 10b). Une étude par RT-PCR de la distribution des transcrits SR-p70 montre que le gène est exprimé dans tous les types cellulaires testés.

### EXEMPLE VII

Clonage du gène du SR-p70 et localisation chromosomique.

### 1) Clonage du gène SR-p70

La banque utilisée est une banque de cosmides, préparée avec de l'ADN génomique humain purifié de placenta, et commercialisée par Stratagène (référence 95 1202).

Le criblage du gène est réalisé comme décrit dans l'exemple III.3 avec un fragment d'ADN SR-p70 marqué au ³²p avec le kit BRL "Random Primers DNA Labelling Systems" (référence 18187-013). Les tampons d'hybridation et de lavage sont additionnés de 50 % formamide. Le dernier lavage est réalisé en 0,1 x SSC/SDS 0,1 % à 60°C. De manière similaire, le gène SR-p70 a été isolé à partir d'une banque préparée avec de l'ADN génomique de la souris black C57.

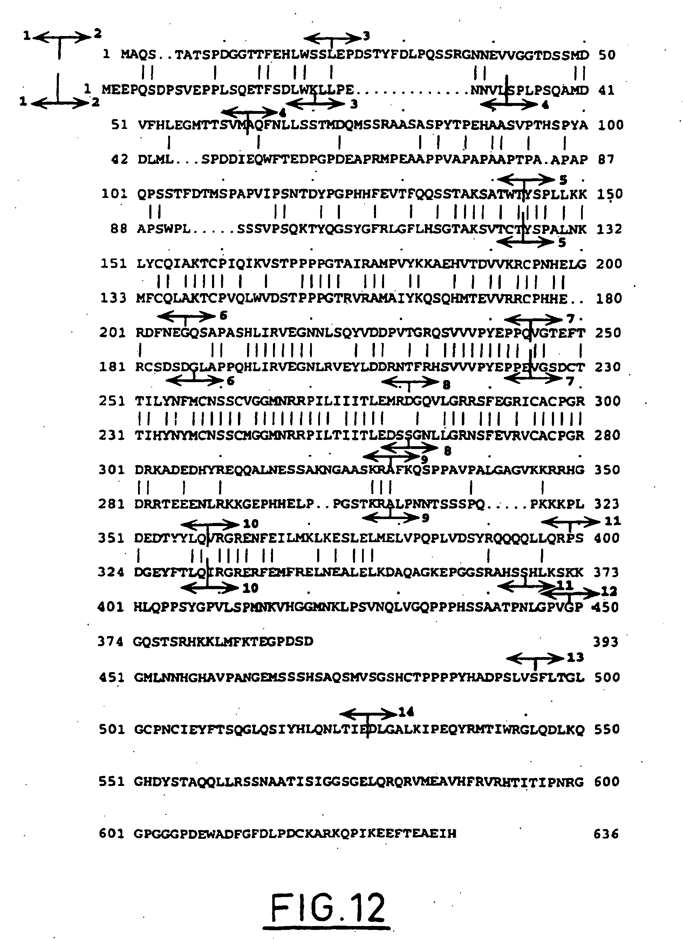

Une analyse et un séquençage partiel des clones mettent en évidence la présence de 14 exons avec une structure proche de celle du gène p53, notamment dans la partie centrale où la taille et le positionnement des exons sont très conservés (Figure 12). Cette structure a été définie partiellement chez la souris et chez l'homme.

A titre d'exemple, les séquences génomiques humaines du 3' de l'intron 1, de l'exon 2, de l'intron 2, et du 5' de l'exon 3 sont présentées dans la figure 13.

### 2) Localisation chromosomique du gène SR-p70 chez l'homme

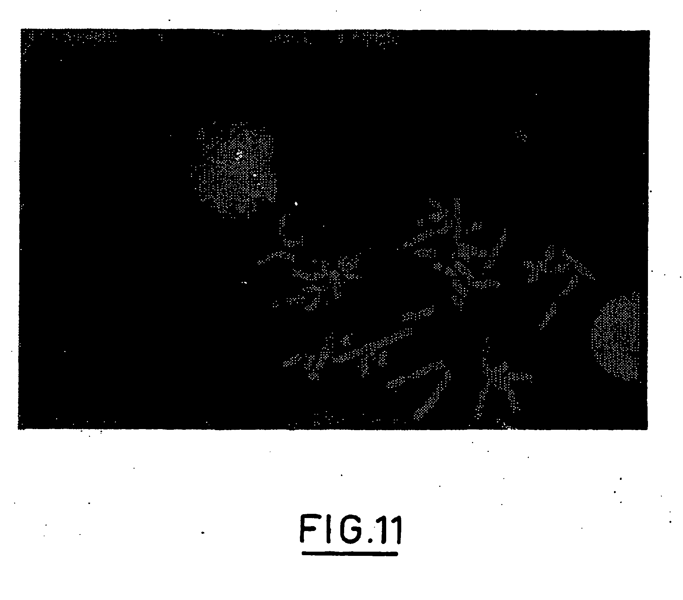

Elle a été réalisée avec de l'ADN du gène SR-70 humain en utilisant la technique décrite par R. Slim et al., Hum. Genet., 1991, 88, 21-26. Cinquante mitoses ont été analysées dont plus de 80% avaient des doubles spots localisés en 1p36 sur les deux chromosomes et plus particulièrement en 1p36.2 -1p36.3 (Figure 11). L'identification du chromosome 1 et son orientation sont basées sur l'hétérochromatine de la constriction secondaire. Les images ont été faites sur un microscope Zeiss Axiophot, saisies par une caméra CCD refroidie LHESA et traitées par Optilab.

### EXEMPLE VIII

### A) Mise en évidence d'un ARNm codant pour une protéine SR-p70 humaine déduite présentant à la fois une extrémité N-terminale plus courte et une divergence.

### 1) Cultures des cellules IMR-32 (neuroblastome humain)

Les cellules ont été cultivées comme décrit dans le catalogue "catalogue of cell lines and hybridomas, 7th edition, 1992" de l'ATCC (American Type Culture Collection).

### 2) Préparation de l'ADNc

L'ARN est préparé comme décrit dans l'exemple I.2.a. L'ADNc est préparé de manière similaire à celle décrite dans l'exemple I.3 avec 5 µg d'ARN total dans un volume final de 20 µl en utilisant une amorce poly (T)₁₂ et avec des nucléotides froids. La réaction n'est pas interrompue avec de l'EDTA.

### 3) Amplification spécifique de l'ADNc SR-P70 par la technique dite de PCR

La polymérisation est réalisée avec 2 µl d'ADNc dans 50 µl final avec le tampon de composition suivant : Tris HCl 50 mM pH 9,2, 16 mM (NH₄)₂ SO₄, 1,75 mM MgCl₂, en présence de DMSO 10%, de NTP 0.4 mM, de 100 ng de chacune des deux amorces nucléiques et de 3,5 unités du mélange des Taq et PWO polymérases (Boehringer Mannheim, réf. 1681 842).

Le couple d'amorce est de composition suivante :
amorce sens : AGGCCGGCGTGGGGAAG (position 16 à 32, Figure 6)
amorce antisens : CTTGGCGATCTGGCAGTAG (position 503 à 485, Figure 6).

La réaction est réalisée durant 30 cycles à 95°C/30 secondes, 58°C/1 minute et 68°C/2 minutes 30 secondes suivi d'un dernier cycle de 68°C/10 minutes.

Le produit PCR est soumis à une électrophorèse sur un gel d'agarose 1% (tampon TAE). Après coloration au bromure d'ethidium, deux bandes majeures sont révélées : une bande d'une taille d'environ 490 pb (taille attendue (voir Figure 6)) et une bande supplémentaire d'une taille d'environ 700 pb. Cette dernière est extraite du gel à l'aide du kit "geneclean" (Bio 101, réf 1001 400). Après un dessalage sur une colonne de polyacrylamide P10 (Biorad, réf 15011050), le fragment est soumis à une nouvelle amplification par PCR durant 10 cycles comme décrit ci-dessus.

### 4) Détermination de la séquence du produit amplifié

Dans un premier temps, le produit PCR est éliminé des oligonucléotides sur une colonne de sephacryl S400 (Pharmacia 17-0609-01) puis dessalé sur une colonne de P10. La réaction de séquençage est réalisée à l'aide du kit Applied Biosystems (réf. 401 628) (373 DNA sequencer) avec l'amorce antisens.

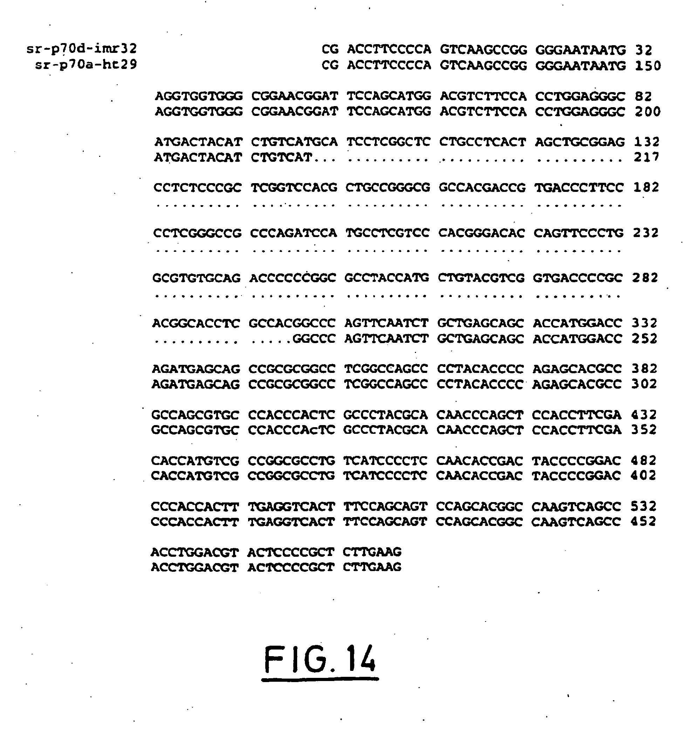

La séquence obtenue est identique à la séquence de l'ADNc SR-p70 (exemple II.4) avec une insertion de 198 pb entre les positions 217 et 218 (Figure 14). La séquence protéique N-terminale déduite (séquence dénommée SR-p70d) est plus courte de 49 acides aminés avec une divergence des 13 premiers acides aminés (séquence ID N°13). Il y a donc co-existence d'au moins deux transcrits différents SR-p70 comme déjà décrit pour la lignée AtT-20 de souris.

### B) Clonage du SR-p70 humain et mise en évidence d'un ARNm codant pour une protéine SR-p70 humaine déduite présentant la même extrémité N-terminale que le SR-p70d et une divergence dans la partie C-terminale

### 1) Amplification spécifique de l'ADNc du SR-p70 par la technique dite de PCR

L'amplification a été réalisée comme décrit dans l'EXEMPLE VIII.A à partir d'ARN purifié des cellules IMR-32 avec le couple d'amorces de composition suivante:
amorce sens : GCG GCC ACG ACC GTG AC (position 160 à 176, séquence ID N° 11)
amorce antisens : GGC AGC TTG GGT CTC TGG (position 1993 à 1976. Figure 6).

Après élimination de l'excès d'amorces sur colonne S400 et dessalage sur colonne P10, 1µl de l'échantillon est de nouveau soumis à une PCR avec le couple d'amorces de composition suivante :
amorce sens : TAT CTC GAG CTG TAC GTC GGT GAC CCC
   Xho I (position 263 à 280, séquence ID N° 11)
amorce antisens : ATA TCT AGA TCA GTG GAT CTC GGC CTC
   Xba I (position 1943 à 1926, Figure 6).

### 2) Clonage du produit amplifié dans le plasmide pCDNA3

Le produit PCR obtenu en 1) est dessalé sur colonne P10, digéré par les enzymes de restriction Xho I et Xba I, puis cloné dans le plasmide pCDNA3 comme décrit dans l'EXEMPLE II.5. Deux clones recombinants sont séquencés à l'aide du kit Applied Biosystems avec les oligonucléotides spécifiques de l'ADNc du SR-p70.

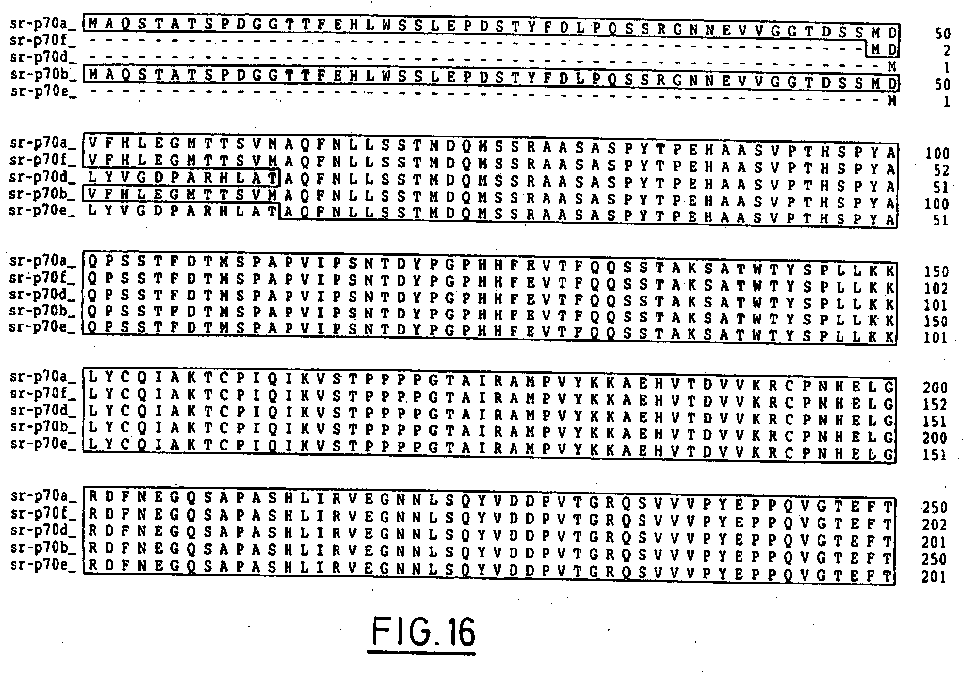

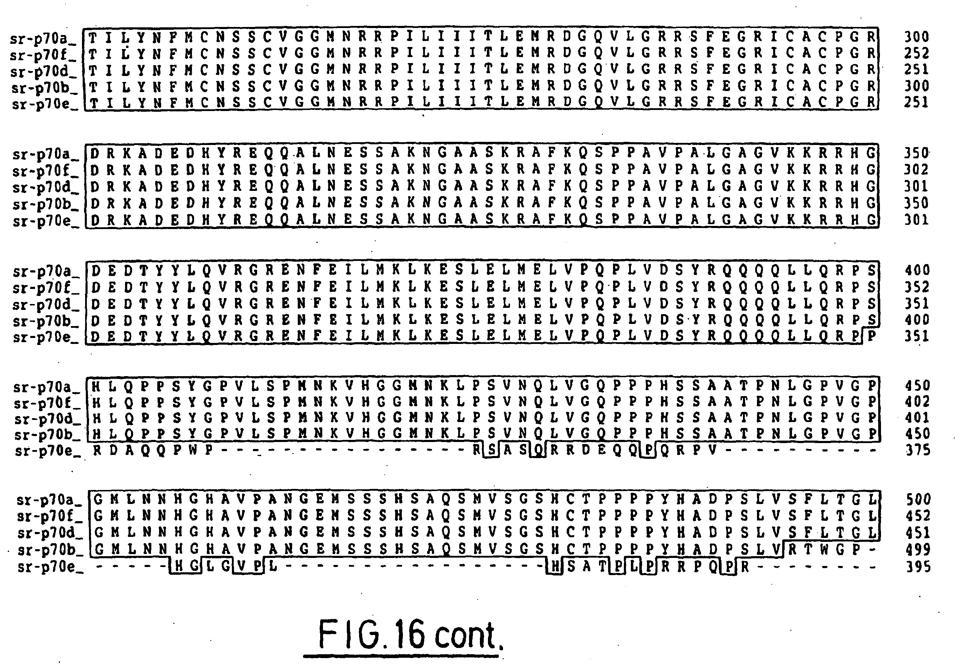

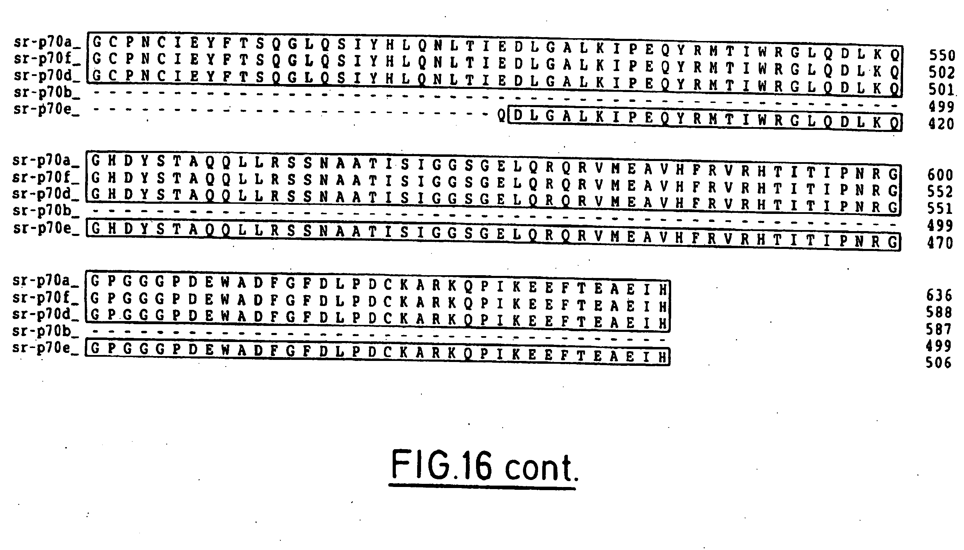

La première séquence obtenue correspond à la séquence complète de l'ARNm codant pour le SR-p70 décrit dans l'EXEMPLE VIII.a .La protéine déduite comporte 587 acides aminés (séquence ID N° 13 et Figure 16).

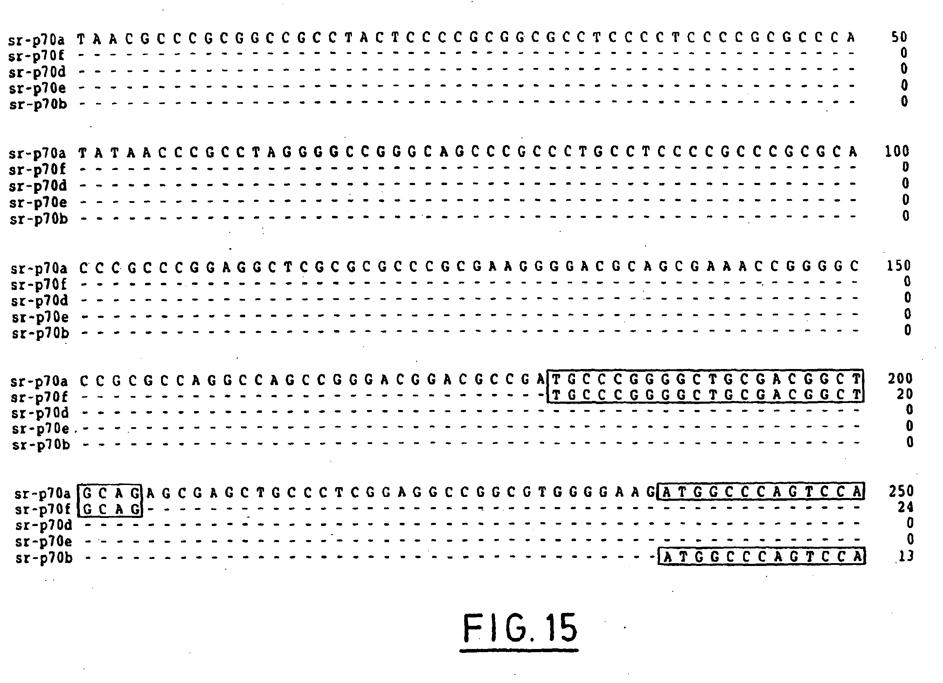

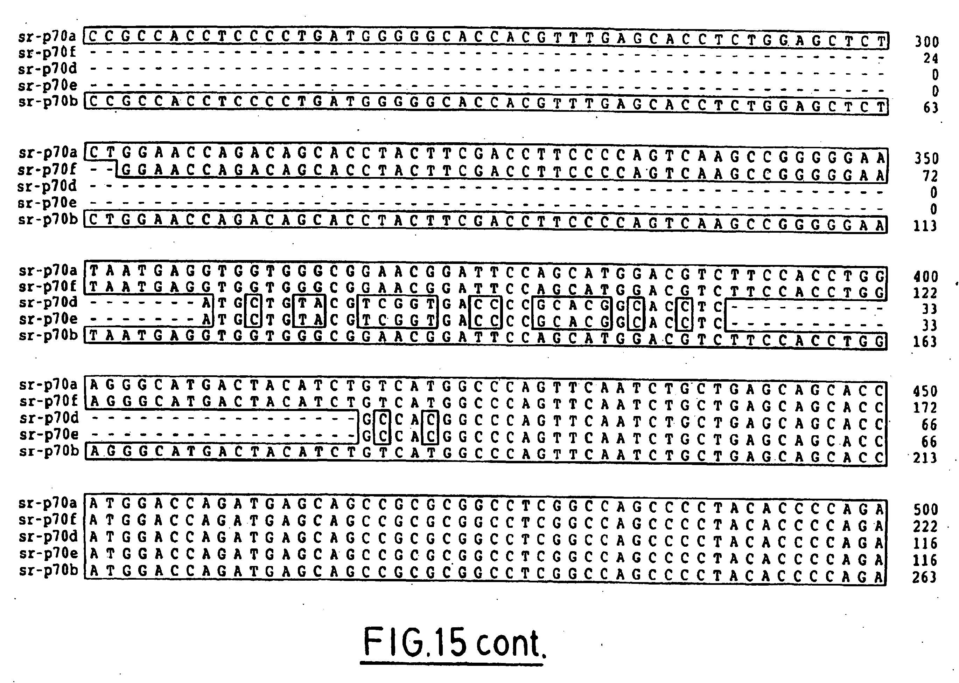

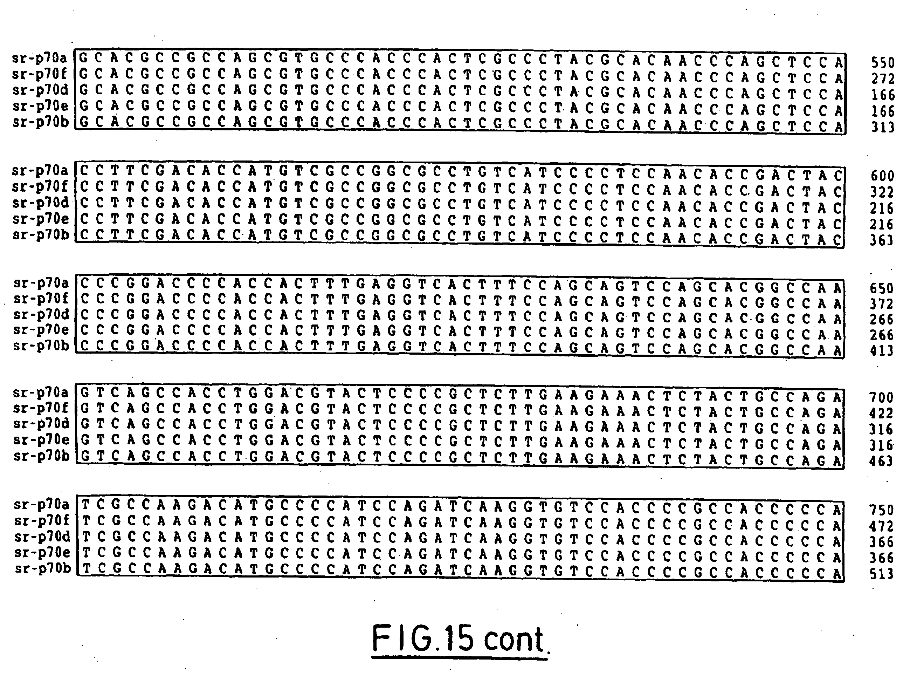

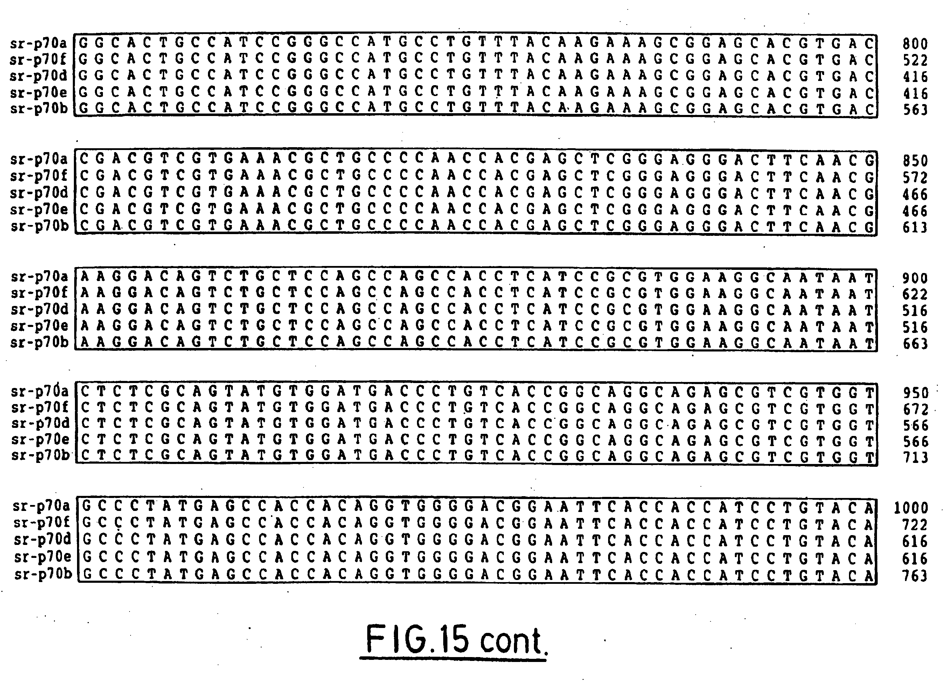

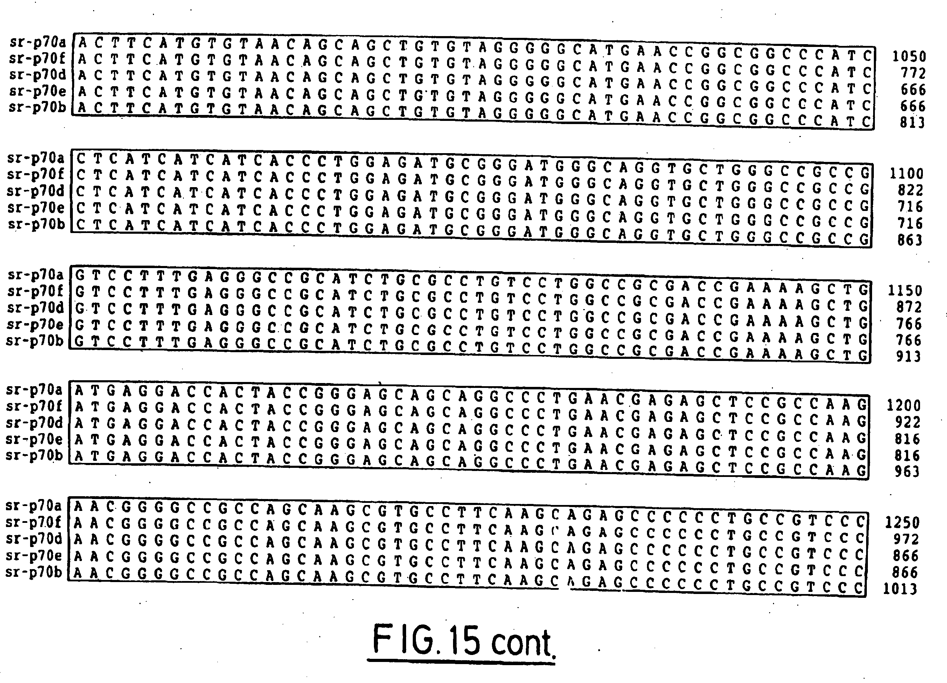

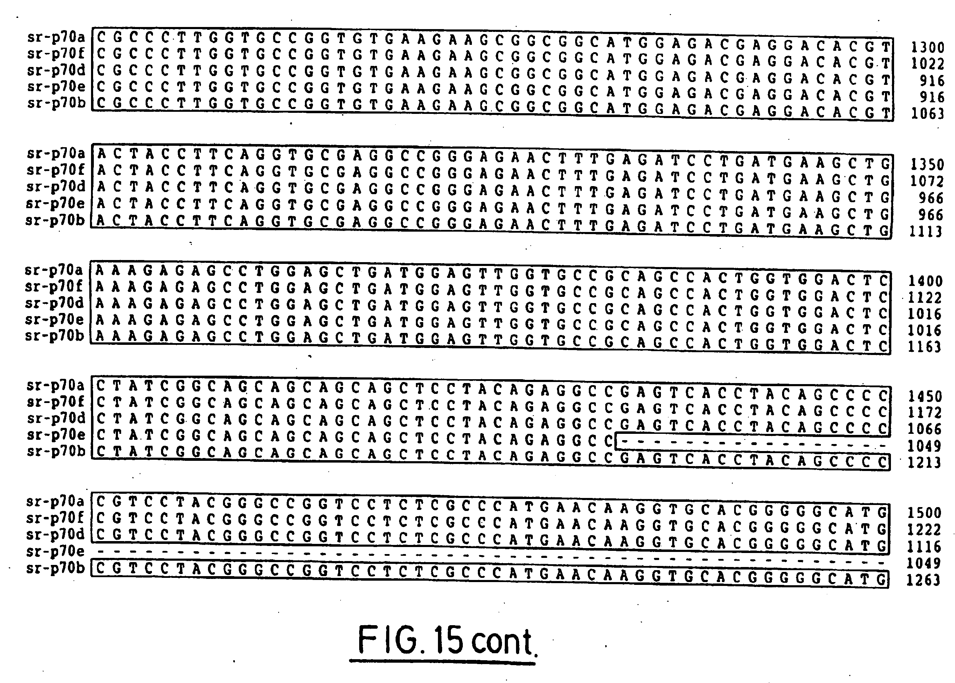

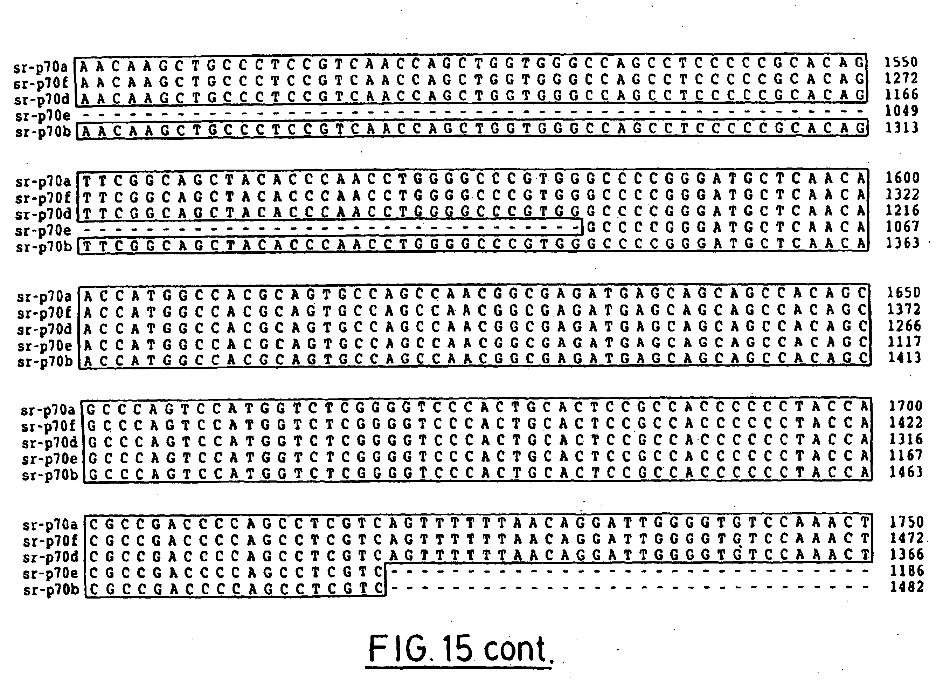

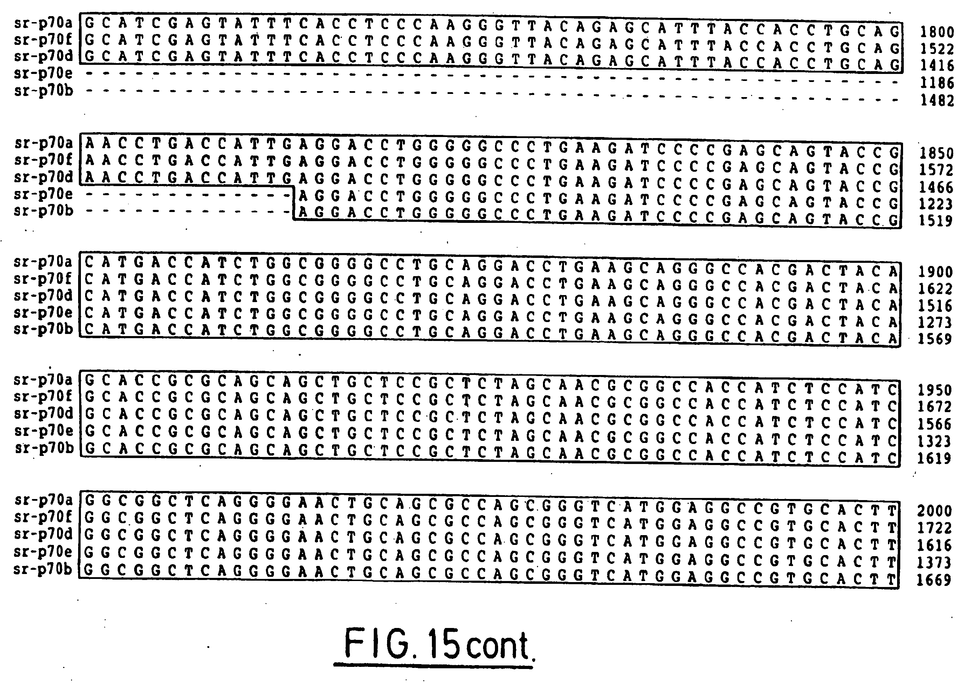

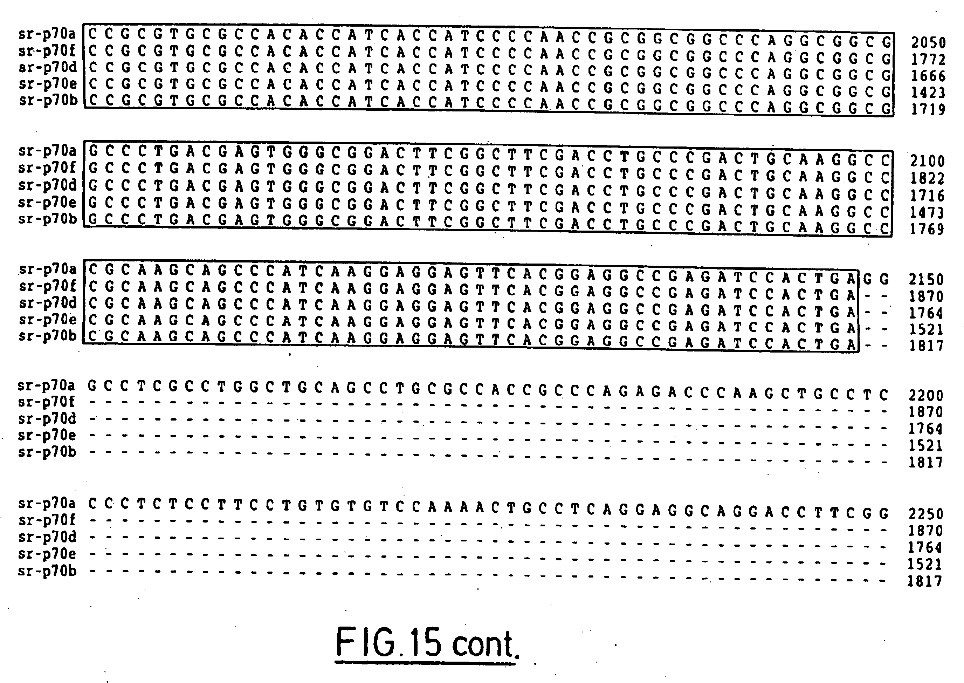

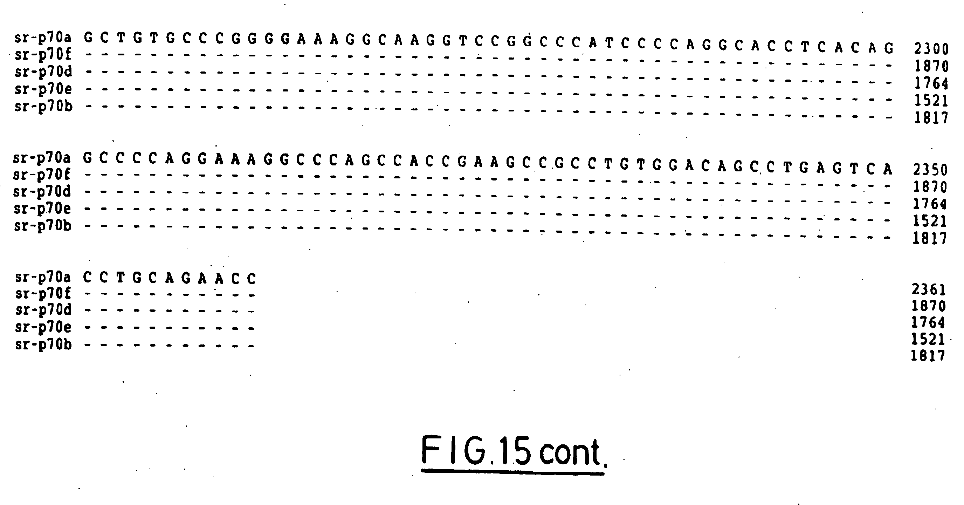

La seconde séquence obtenue est identique à la séquence de l' ADNc de SR-p70d décrite ci-dessus mais avec deux délétions de 149 pb et de 94 pb entre les positions 1049 et 1050 d'une part et entre les positions 1188 et 1189 d'autre part (séquence ID N° 14 et Figure 15). La séquence protéique déduite de cette seconde séquence révèle une protéine ayant une partie N-terminale plus courte de 49 acides aminés avec une divergence dans les 13 premiers acides aminés ainsi qu'une divergence de séquence protéique entre les acides aminés 350 et 397 (séquence ID N° 15 et Figure 16) (séquence dénommée SR-p70e). La protéine déduite comporte 506 acides aminés.

### C) Mise en évidence d'un ARNm codant pour une protéine SR-p70 humaine déduite présentant une extrémité N-terminale plus courte

### 1) Culture des cellules SK-N-SH (neuroblastome humain)

Les cellules sont cultivées comme décrit dans le « catalogue of cell lines and hybridomas, 7th édition, 1992 » de l'ATCC (American Type Culture Collection).

### 2) Préparation de l'ADNc et amplification de l'ADNc du SR-p70 par la technique dite de PCR

Ces étapes sont réalisées comme décrit dans l'EXEMPLE VIII.A avec le couple d'amorces de composition suivante:
amorce sens: AGG GGA CGC AGC GAA ACC (position 128 à 145. Figure 17)
amorce anti sens :GGC AGC TTG GGT CTC TGG (position 1993 à 1976, Figure 6).

Le séquençage est réalisé avec le kit Applied Biosystem avec des amorces spécifiques de l'ADNc du SR-p70 et révèle deux ADNc:
- un premier ADNc correspondant à l'ARNm codant pour le SR-p70a
- un second ADNc présentant une délétion de 98 pb entre les positions 24 et 25 (séquence 10 N° 16 et Figure 15).
   Cette délétion comprend l'ATG d'initiation de traduction du SR-p70a. La protéine déduite (dénommée SR-p70f) de ce second ADNc présente un ATG initiateur de traduction en aval correspondant à un ATG interne du SR-p70a. La protéine déduite comporte donc 588 acides aminés (séquence ID N° 17 et Figure 16) et est tronquée des 48 acides aminés N-terminaux du SR-p70a.

### D) Mise en évidence d'un ARNm codant pour le SR-p70b humain.

### 1) Culture des cellules K562

Les cellules sont cultivées comme décrit dans le "catalogue of cell lines and hybridomas, 7 th édition, 1992" de l'ATCC (American Type Cylture Collection).

### 2) Préparation de l'AONc, amplification de l'ADNc du SR-p70 par la technique dite de PCR et séquençage.

Ces étapes sont réalisées comme décrit dans l'EXEMPLE VIII.C.

Le séquençage révèle deux ADNc:
Un premier ADNc correspondant à l'ARNm codant pour le SR-p70a et un second ADNc présentant une déletion de 94 pb entre les positions 1516 et 1517 (séquence ID N° 18 et Figure 15). La protéine déduite (dénommée SR-p70b) comporte 199 acides aminés et présente une séquence C-terminale tronquée de 137 acides aminés par rapport au SR-p70a avec les 4 derniers acides aminés divergents (séquences ID N° 19 et Figure 21).
Cet ADNc est similaire à celui décrit dans l'EXEMPLE I relatif au SR-p70b de singe.

Les molécules décrites dans cet exemple (EXEMPLE VIII. A. B. C, et D) mettent en évidence des variants du SR-p70 consécutifs à des épissages différentiels de l'ARNm primaire transcrits par le gène SR-p70.

Le SR-p70a est codé par un ARNm composé de 14 exons (voir EXEMPLE VII). C'est la protéine de référence. Le SR-p70b est consécutif à une insertion entre les exons 3 et 4 et à l'absence des exons 11 et 13. Le SR-p70f est consécutif à l'absence de l'exon 2. Cet exemple décrit l'existence de variants du SR-p70 de manière non exhaustive, avec une probabilité forte d'existence d'autres variants. De même, l'existence de ces variants décrits dans cet exemple ainsi que le SR-p70a ne se limite pas aux lignées dans lesquelles ils ont été mis en évidence. En effet des études effectuées par RT-PCR ont montré que ces variants sont retrouvés dans les diverses lignées étudiées.

De plus, la méthionine d'initiation du SR-p70f correspond à une méthionine interne SR-p70a, suggérant la possibilité d'initiation en aval sur l'ARNm codant pour le SR-p70a.

### EXEMPLE IX

### Obtention d'une séquence 5' de l'ARNm SR-P70a humaine.

### 1) Amplification de l'extrémité de 5'de l'ADNc SR-P70 par PCR

La culture cellulaire et les préparations d'ARN total et d'AONc sont réalisées comme décrit dans l'EXEMPLE VIII.1 et 2. La matrice ARN est hydrolysée par incubation 5 minutes à 65°C après adjonction de 4 µl EDTA 500 mM et 4 µl NaOH 2N. L'échantillon est ensuite dessalé sur colonne P10. L'ADNc est allongé en 3' avec une "queue" de dG comme décrit dans l'EXEMPLE I.3.d, dans un volume final de 40 µl. Après adjonction de 4 µl EDTA 500 mM et 4 µl NaOH 2N, l'ADNc est incubé à 65°C pendant 3 minutes puis dessalé sur une colonne P10. L'amplification par PCR est réalisée comme décrit dans l'EXEMPLE VIII.3 avec 8 µl d'AONc et durant 30 cycles avec le couple d'amorces de composition suivante :
amorce sens : **CCCCCCCCCCCCCCN** (où N est égal à G,A ou T)
amorce antisens: CCATCAGCTCCAGGCTCTC (position 1167 à 1149, Figure 6).

Après élimination de l'excès d'amorces sur colonne S400 et dessalage sur colonne P10, 1 µl de l'échantillon est de nouveau soumis à un PCR avec le couple de composition suivante :
amorce sens: **CCCCCCCCCCCCCCN**
amorce antisens: CCAGGACAGGCGCAGATG (position 928 à 911, Figure 6).

L'échantillon, de nouveau passé sur une colonne S400 et une colonne P10, est soumis à une troisième amplification durant 20 cycles avec le couple suivant :
amorce sens : **CCCCCCCCCCCCCCCN**
amorce antisens: CTTGGCGATCTGGCAGTAG (position 503 à 485, Figure 6).

### 2) Détermination de la séquence 5' ADNc SR-P70

La séquence est réalisée comme décrit dans l'EXEMPLE VIII.4). Cette séquence fait apparaître un 5' non codant d'au moins 237 bases en amont de l'ATG d'initiation du SR-p70a (Figure 17). Par comparaison de cette séquence (obtenue à partir de la lignée IMR-32) avec celle obtenue à partir de la lignée HT-29 notamment (Figure 6), deux différences ponctuelles (Figure 17 : voir caractères gras) sont mises en évidence (G → A et C → T) respectivement positionné à -20 et -30 de l'ATG d'initiation du SR-p70a (Figures 6 et 17). Cette variabilité est située au niveau de l'exon 2 (Figure 13). II n'est pas exclu que cette variabilité se retrouve également à l'intérieur d'une phase codante consécutivement à un épissage alternatif comme décrit dans les EXEMPLES III chez la souris et VIII chez l'homme ou bien consécutivement à une initiation de la traduction sur un CTG (comme cela a été démontré pour le FGFb (Proc. Natl. Acad. Sci USA, 1989, 88, 1836 - 1840).

De même, il n'est pas exclu que cette variabilité ait une implication sur la traduction du SR-p70 ou sur l'épissage de l'ARN primaire.

En tout état de cause, cette variabilité, probablement d'origine allélique, peut servir de marqueur soit au niveau génomique (voir EXEMPLE XI), soit au niveau de l'ARNm (voir EXEMPLE X).

### EXEMPLE X

### 1) Analyse par PCR, de l'expression transcriptionnelle du SR-P70a dans les échantillons cellulaires (RT - PCR)

Les cultures cellulaires (SK-N-AS, SK-N-MC, HT-29, U-373MG, SW480. IMR-32. CHP212) sont réalisées comme décrit dans l'exemple VI.1.a (référées au catalogue "catalogue of cell Unes and hybridomas, 7th edition" 1992 de l'ATCC).

La préparation de l'ADNc et l'amplification par PCR sont réalisées comme décrit dans l'exemple VIII.2 et 3. Le couple d'amorce utilisé est de composition suivante :
amorce sens : AGGGGACGCAGCGAAACC (position 128 à 145. Figure 17)
amorce antisens : GGCAGCTTGGGTCTCTGG (position 1993 à 1976, Figure 6).

Les échantillons sont analysés par électrophorèse sur un gel d'agarose 1% et révélation au Bromure d'éthidium (Figure 18).

La taille de la bande obtenue dans les échantillons correspond à la taille attendue (environ 2 kb, Figures 6 et 17). L'intensité des bandes obtenues est reproductible. Une réamplification de 1 µl de l'échantillon dans les mêmes conditions durant 20 cycles fait apparaître une bande dans chacun des échantillons.

### 2) Détermination de la séquence des produits amplifiés

Après passage des échantillons sur colonnes S400 et P10, le séquençage est réalisé sur le séquenceur 373 de Applied Biosystems avec le kit de référence 401 628. Les amorces utilisées sont entre autres les suivantes :

| | position | figure |
|---|---|---|
| AGGGGACGCAGCGAAACC | 128 à 145 | 22 |
| CTTGGCGATCTGGCAGTAG | 503 à 485 | 6 |
| GATGAGGTGGCTGGCTGGA | 677 à 659 | 6 |
| CCATCAGCTCCAGGCTCTC | 1167 à 1149 | 6 |
| TGGTCAGGTTCTGCAGGTG | 1605 à 1587 | 6 |
| GGCAGCTTGGGTCTCTGG | 1993 à 1976 | 6 |

Aucune différence protéique du SR-p70a n'a été décelée. Cependant, les séquences obtenues font apparaître une double variabilité aux positions -20 et -30 en amont de l'ATG d'initiation du SR-p70a (Figures 6 et 17). Cette variabilité, probablement d'origine allélique, permet de définir deux classes de transcrits : une première classe présentant un G à la position -30 et un C à la position -20 (classe G⁻³⁰/C⁻²⁰) et une seconde classe présentant une différence aux deux positions : un A en -30 et un T en -20 (classe A⁻³⁰/T⁻²⁰).
Première classe : SK-N-AS, SK-N-MC, HT-29, U-373MG, SW480.
Deuxième classe : IMR-32, CHP212.

### EXEMPLE XI

Méthode d'analyse pour la détermination de la répartition allélique du gène SR-p70 dans un échantillonnage de 10 personnes.

Cette répartition allélique est basée sur la variabilité allélique mise en évidence dans les exemples IX et X :
- Allèle G⁻³⁰/C⁻²⁰ présentant respectivement un G et un C aux positions -30 et -20
- en amont de l'ATG d'initiation du SR-p70a.
- Allèle A⁻³⁰/T⁻²⁰ présentant respectivement un A et un T aux mêmes positions. Cette variabilité peut être mise en évidence par l'utilisation d'enzymes de restriction différenciant les deux allèles (Figure 13). A titre d'exemple:

- Enzyme Bpl I présentant un site de coupure uniquement sur l'allèle G⁻³⁰/C⁻²⁰ dans la zone d'intérêt (ce site englobe les deux positions valables).
- Enzyme Styl présentant un site de coupure uniquement sur l'allèle A⁻³⁰/T⁻²⁰ dans la zone d'intérêt.

### 1) Amplification génomique de l'exon 2 par PCR

La réaction de polymérisation est réalisée, avec 500 ng d'ADN génomique purifié, dans 50 µl final avec les conditions décrites dans l'exemple VIII.3. Le couple d'amorces est de composition suivante :
amorce sens : CACCTACTCCAGGGATGC (position 1 à 18, Figure 13)
amorce antisens : AGGAAAATAGAAGCGTCAGTC (position 833 à 813, Figure 13).

La réaction est réalisée durant 30 cycles comme décrit dans l'EXEMPLE VIII.3.

Après élimination de l'excès d'amorce sur une colonne S400 et dessalage sur une colonne de P10, 1 µl de l'échantillon est amplifié de nouveau durant 25 cycles dans les mêmes conditions avec le couple d'amorces suivant :
amorce sens : CAGGCCCACTTGCCTGCC (position 25 à 32, Figuré.13)
amorce antisens : CTGTCCCCAAGCTGATGAG (position 506 à 488, Figure 13).

Les produits amplifiés sont soumis à une électrophorèse sur un gel d'agarose 1% (Figure 19-A).

### 2) Digestion par l'enzyme de restriction Styl

Les échantillons sont au préalable dessalés sur une colonne P10 puis digérés par l'enzyme de restriction Styl (BRL 15442-015) dans le tampon de composition suivant :
Tris HCl pH 8. 50 mM, NaCl 100 mM, MgCl₂ 10 mM, à 37°C pendant 30 mn. Les produits de digestion sont analysés par électrophorèse sur un gel d'agarose 1% (tampon TAE). La révélation est réalisée par coloration au bromure d'ethidium (Figure 19-B).

Une bande de 482 paires de bases caractérise l'allèle G⁻³⁰/C⁻²⁰ (Figures 13 et 19). La présence d'une bande de 378 paires de bases et d'une bande de 106 paires de bases caractérisent l'allèle A⁻³⁰/T⁻²⁰ (allèle présentant un site de coupure Styl). Sur l'échantillonnage de 10 personnes, 2 personnes présentent les allèles G⁻³⁰/C⁻²⁰ et A⁻³⁰/T⁻²⁰, les 8 autres personnes étant homozygotes avec l'allèle G⁻³⁰/C⁻²⁰. L'étude d'un nouvel échantillonnage de 9 personnes a mis en évidence 3 personnes hétérozygotes présentant les allèles G⁻³⁰/C⁻²⁰ et A⁻³⁰/T⁻²⁰. les 6 autres personnes étant homozygotes pour l'allèle G⁻³⁰/C⁻²⁰.

### EXEMPLE XII

### Test de réversion de transformation de la lignée SK-N-AS par transfection avec l'ADNc SR-p70.

Le vecteur d'expression utilisé est décrit dans l'EXEMPLE II.5 et schématisé dans la figure 15. La méthode utilisée est celle dite du phosphate de calcium décrite par Graham et al. (Virology 1973, 54, 2, 536-539). La lignée est ensemencée à raison de 5.10⁵ cellules par boite de 6 cm de diamètre dans 5 ml du milieu décrit dans l'exemple I.1.. Les cellules sont mises en culture à 37°C et à 5% de CO₂ pendant une nuit. Le milieu de transfection est préparé de la manière suivante : le mélange suivant est réalisé en ajoutant dans l'ordre 1 ml de tampon HEBS (NaCl 8 mg/ml, KCI 370 µg/ml. Na₂HPO₄-2H₂O 125 µg/ml, Dextrose 1 mg/ml, Hepes pH 7,05 5 mg/ml), 10 µg du plasmide à transfecter et 50 µl CaCl₂ 2,5 M ajouté goutte à goutte. Le milieu de transfection est laissé 30 mn à température ambiante puis ajouté goutte à goutte sur le milieu contenu dans la boite de culture. Les cellules sont incubées 5 à 6 heures à 37° C/5% CO₂. Après aspiration du milieu, 5 ml de milieu frais contenant 2% de sérum du bovin foetal sont ajoutés. Après 48 heures à 37° C/5% CO₂, les cellules sont rincées avec du PBS, décollées par trypsinisation, diluées dans 10 ml de milieu de culture (5% sérum de bovin foetal) et étalées dans une boite de 10 cm de diamètre (la dilution peut être ajustée en fonction de l'efficacité de transfection). Après une nouvelle incubation durant 10 heures (le temps que les cellules adhèrent), les cellules sont passées en sélection par adjonction de G418 à la concentration finale de 600 µg/ml équivalent généticine durant 15 à 21 jours (le milieu est changé tous les jours). Les clones obtenus sont alors rincés au PBS, fixés à l'éthanol 70%, séchés, colorés avec 1 % de cristal violet, puis comptabilisés.

Quatre transfections plasmidiques ont été réalisées en double :
- plasmide pCDNA3 sans insert
- plasmide pCONA3/SR-p70 contenant l'ADNc - SR-P70a humaine
- plasmide pCDNA3/SR-p70 Mut contenant l'ADNc - SR-p70a présentant une mutation à la position 293 AA (R → H) qui est analogue à la mutation 273 (R → H) dans le domaine de fixation à l'ADN de la p53
- témoin sans plasmide.

Le résultat est exprimé en nombre de clones par boite.

| | Expérience 1 | Expérience 2 | Moyenne |
|---|---|---|---|
| pCDNA3 | 172 | 353 | 262 |
| pCDNA3 / SR-p70 | 13 | 8 | 10 |
| pCDNA3 / SR-p70 mut | 92 | 87 | 89 |
| Absence de plasmide | 1 | 3 | 2 |

Le nombre de clones obtenu par transfection avec le plasmide pCDNA3/SR-p70 est de 25 fois inférieur au nombre de clones obtenu avec le témoin pCDNA3 et de 9 fois inférieur au nombre de clones obtenu avec le pcDNA3/SR-p70 Mut, indiquant une mortalité ou un arrêt de division cellulaire des cellules transfectées par l'ADNc SR-p70. Ce résultat n'est pas la conséquence d'une toxicité au vue des clones obtenus avec l'ADNc SR-p70 muté mais probablement d'une apoptose comme cela a été démontré pour la protéine p53 (Koshland et al., Sciences, 1993. 262, 1953-1981).

### EXEMPLE XIII

### Rôle biologique de la protéine SR-p70.

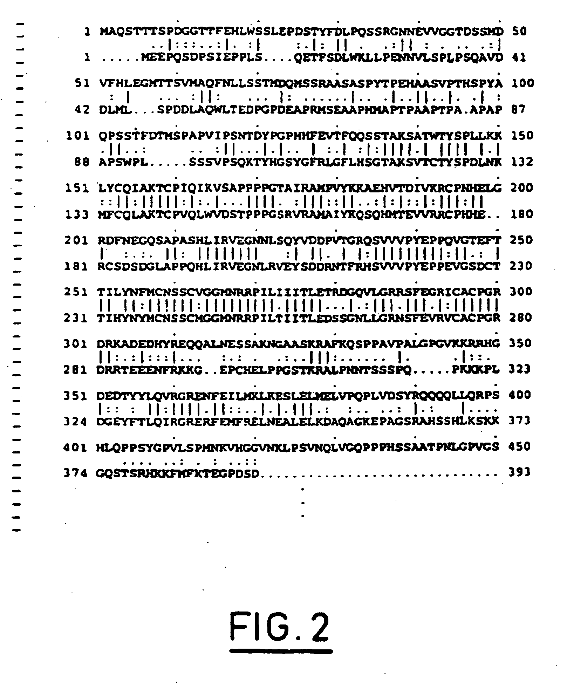

L'homologie de structure entre le domaine de fixation à l'ADN de la p53 et la région centrale de la protéine SR-p70 permet d'inférer que la SR-p70 est un facteur de transcription (cf. Figures 1 et 2). En effet, la p53 (393 acides aminés) est constituée de plusieurs domaines fonctionnels. La région N-terminale (1-91 acides aminés) est impliquée dans l'activation de la transcription, et contient des sites d'interaction à différentes protéines cellulaires et virales. La partie centrale (acides aminés 92 à 292) permet la fixation aux séquences d'ADN spécifiques situés dans les régions promotrices de certains gènes (la majorité des mutations ponctuelles inactivant la p53 sont localisées dans cette région), elle présente également de nombreux sites d'interaction avec des protéines virales qui inhibent son activité. Enfin, les 100 derniers acides aminés de la p53 sont responsables de son oligomérisation ainsi que de la régulation de celle-ci (Hainaut P., Current Opinion in Oncology, 1995, 7, 76-82; Prokocimer M., Blood, 1994, 84 n°8, 2391-2411).

L'homologie de séquence entre p53 et SR-p70 est significative notamment en ce qui concerne les acides aminés impliqués directement dans l'interaction à l'ADN suggérant que la SR-p70 se fixe aux sites p53 sur l'ADN. Ces acides aminés correspondent très exactement à ce qu'on appelle les "hot spot", acides aminés fréquemment mutés dans les tumeurs humaines (SWISS PROT : SW : P53_human et

Prokocimer M., Blood, 1994, 84 n°8. 2391-2411). De cette homologie, on peut déduire que la protéine SR-p70 exerce un contrôle sur l'activité des gènes régulés par la p53, soit indépendamment de celle-ci soit en formant des hétérooligomèrés avec cette dernière.

En conséquence, à l'instar de la p53, les produits du gène SR-p70 doivent être impliqués dans le contrôle et la régulation du cycle cellulaire provoquant des arrêts du cycle (momentanés ou définitifs), et la mise en oeuvre de programmes tels que : la réparation de l'ADN, la différenciation ou la mort cellulaire. L'existence d'activités "p53-like" avait été fortement préssentie avec la mise en évidence chez les souris p53^{-/-}, d'activités de réparation de l'ADN et de mort cellulaire en réponse aux radiations ionisants (Strasser et al., Cell, 1994, 79, 329-339). Les auteurs de la présente invention ont localisé le gène SR-p70 humain dans la région télomérique du bras court du chromosome 1, précisément en 1p36.2-36.3, la plus petite région délétée (SRO) commune à une majorité de neuroblastomes et d'autres types de tumeurs (mélanomes et carcinomes) (White et al., PNAS, 1995, 92, 5520-5524). Cette région de perte d'hétérozygotie (LOH) délimite le locus d'un gène suppresseur de tumeur dont la perte d'activité serait la cause de la formation des tumeurs. II est important de rappeler que cette région est également sujette à "l'empreinte matemelle"; l'allèle maternel est préférentiellement perdu dans les neuroblastomes présentant la délétion 1p36 (sans amplification de N-Myc) (Caron et al., Hum. Mol. Gen., 1995, 4, 535-539). Le gène sauvage SR-p70 introduit et exprimé dans des cellules de neuroblastome permet la réversion de leur transformation. La perte de cette activité anti-oncogénique est donc associée au développement de la tumeur. La région 1p38 présente une homologie syngénique avec le segment distal du chromosome 4 de souris. Dans cette région a été localisé le gène *curly tail (ct)* (Beier et al., Mammalian Genome, 1995, 6, 269-272) impliqué dans les malformations congénitales du tube neural (MTN : *spida bifida,* anencéphalie...). La souris *ct* est le meilleur modèle animal d'étude de ces malformations. II est admis que ces malformations résultent d'anomalies de la prolifération cellulaire. Compte tenu de la nature du gène SR-p70 et de sa localisation chromosomique, une des hypothèses est que le SR-p70 pourrait être l'homologue humain de *ct* et qu'à ce titre, la détection des mutations précoces et les anomalies chromosomiques concernant ce gène devraient permettre par exemple comme application, l'identification des personnes à risques (0,5-1 % des nouveaux-nés atteints par MTN), et la mise en oeuvre de traitements préventifs (Neumann et al., Nature Genetics, 1994, 6, 357-362; Di Vinci et al., Int. J. Cancer, 1994, 59, 422-426; Moll et al., PNAS, 1995, 92, 4407-4411; Chen et al., Development, 1995, 121, 681-691).

### EXEMPLE XIV

### Etude allélique du gène SR-p70.

Les allèles GC et AT sont identifiés aisément par restriction Styl des produits PCR de l'exon 2 (voir exemple XI). On a donc pu déterminer ainsi, chez des individus hétérozygotes GC/AT et porteurs de tumeurs neuroblastomes, l'allète SR-p70 perdu (GC ou AT) et cela malgré la présence de tissu contaminant sain.

D'une façon surprenante, lorsque la même analyse est réalisée sur l'ARN, un seul allèle est mis en évidence indépendamment de présence ou non d'une délétion et plus surprenant encore, malgré la présence de tissu sain. Cela suggère que l'empreinte (expression différentielles des deux allèles) existerait également dans le tissu contaminant.

Pour le vérifier, on a répété la même analyse sur de l'ARN provenant des cellules sanguines d'individus sains hétérozygotes GC/AT. Un seul des deux types de transcrit a été détecté également dans ces cellules. Ce résultat confirme l'observation faite sur les échantillons tumoraux quant à l'existence d'une empreinte génétique généralisée pour le gène SR-p70.

Les implications de cette découverte sont importantes puisqu'elle permet de postuler qu'une seule mutation sporadique inactivant l'allèle actif SR-p70 entraînera une perte d'activité et cela potentiellement dans tous les tissus.

L'absence de données précises sur la fonction biologique SR-p70 ne permet pas de mesurer les conséquences de cette perte d'activité SR-p70 pour la cellule. Néanmoins, sa forte homologie avec la protéine suppresseur de tumeur p53, ainsi que la démonstration que la SR-p70 est un facteur dé transcription capable d'utiliser le promoteur P21^{wsf}, suggère un rôle de cette protéine dans le contrôle du cycle cellulaire et dans la différenciation.

Knudson and Meadows 1980 (New Eng. J. Med. 302:1254-56) considèrent les neuroblastomes IV-S comme une collection de cellules non malignes de la crête neurale portant une mutation qui interfère avec leur différencation normale.

II est concevable que la perte d'activité SR-p70 tout comme la perte du contrôle p53 sur le cycle cellulaire favorise l'apparition d'anomalies cellulaires telles que l'aneuploïdie, l'amplification (décrites dans le cas des neuroblastomes) et d'autres remaniements génétiques pouvant provoquer la transformation cellulaire (Livingstone et al. 1992. Cell 71 :923-25; Yin et al. 1992, Cell 72 :937-48; Cross et al. 1995. Science 267:1353-56; Fukasawa et al. 1996, science 211:1744-47). Les neuroblastomes pourraient donc provenir à leur origine d'une perte d'activité temporaire ou définitive de la SR-p70, favorisant ainsi l'apparition d'événements oncogéniques et donc la progression tumorale.

Dans le cas de la délétion constitutionnelle 1p36 décrite par Biegel et al. 1993 (Am. J. Hum. Genet. 52:176-82), il y a bien apparition de neuroblastome IV-S, et le gène concerné est NBS-1 (SR-p70).

En conclusion, ce qui est décrit pour les neuroblastomes pourrait également s'appliquer à d'autres types de tumeurs notamment ceux associés à des remaniements de l'extrémité du bras court du chromosome 1 (report 2 international workshop on human chr 1 mapping 1995, Cytogenetics and Cell Genet 72 :113-154). Sur un plan thérapeutique, l'implication de la SR-p70 dans l'apparition de tumeurs devrait conduire à éviter l'utilisation d'agents mutagènes en chimiothérapie, compte tenu des risques de transformation cellulaire par ces produits, et leur préférer des substances non mutagènes qui stimulent la différenciation.

D'autre part, la fréquence d'apparition des allèles GC et AT est la suivante : dans la copulation, Fréquence(AT)=0.15 et sur un échantillon de 25 patients (neuroblastomes), F(AT)=0.30. Ces statistiques indiquent que l'allèle AT pourrait être un facteur de prédisposition.

## Revendications

1. Polypeptide purifié, comprenant une séquence d'acides aminés choisie parmi :
a) la séquence SEQ ID n° 2 ;
b) la séquence SEQ ID n° 4 ;
c) la séquence SEQ ID n° 6 ;
d) la séquence SEQ ID n° 8 ;
e) la séquence SEQ ID n° 10 ;
f) la séquence SEQ ID n°13 ;
g) la séquence SEQ ID n° 15 ;
h) la séquence SEQ ID n° 17 ;
i) la séquence SEQ ID n° 19.

2. Polypeptide selon la revendication 1, **caractérisé en ce qu'**il comprend la séquence d'acides aminés choisie parmi SEQ ID n° 6, SEQ ID n° 13, SEQ ID n° 15, SEQ ID n° 17 et SEQ ID n° 19.

3. Polypeptide selon la revendication 1, **caractérisé en ce qu'**il comprend la séquence comprise entre :
- le résidu 110 et le résidu 310 de SEQ ID n° 2 ou 6 ;
- le résidu 60 et le résidu 260 de SEQ ID n° 8.

4. Polypeptide selon la revendication 1, **caractérisé en ce qu'**il résulte d'un épissage alternatif de l'ARN messager du gène correspondant.

5. Polypeptide selon l'une quelconque des revendications précédentes, **caractérisé en ce qu'**il s'agit d'un polypeptide recombinant produit sous la forme d'une protéine de fusion.

6. Séquence d'acides nucléiques isolée codant pour un polypeptide selon l'une quelconque des revendications précédentes.

7. Séquence d'acides nucléiques isolée selon la revendication 6, **caractérisée en ce qu'**elle est choisie parmi :
a) la séquence SEQ ID n° 1 ;
b) la séquence SEQ ID n° 3 ;
c) la séquence SEQ ID n° 5 ;
d) la séquence SEQ ID n°7 ;
e) la séquence SEQ ID n°9 ;
f) la séquence SEQ ID n° 11 ;
g) la séquence SEQ ID n° 12 ;
h) la séquence SEQ ID n° 14 ;
i) la séquence SEQ ID n° 16 ;
j) la séquence SEQ ID n° 18.

8. Séquence nucléotidique selon la revendication 6, **caractérisée en ce qu'**il s'agit d'une séquence choisie parmi SEQ ID n° 5, SEQ ID n° 12, SEQ ID n° 14, SEQ ID n° 16 et SEQ ID n° 18 codant respectivement pour le polypeptide de séquences SEQ ID n° 6, SEQ ID n° 13, SEQ ID n° 15, SEQ ID n° 17 et SEQ ID n° 19.

9. Vecteur de clonage et/ou d'expression contenant une séquence d'acides nucléiques selon l'une quelconque des revendications 6 à 8.

10. Vecteur selon la revendication 9, **caractérisé en ce qu'**il s'agit du plasmide pSE1.

11. Cellule hôte transfectée par un vecteur selon la revendication 9 ou 10.

12. Cellule hôte transfectée selon la revendication 11, **caractérisée en ce qu'**il s'agit de *E. coli* MC 1061.

13. Sonde ou amorce nucléotidique comprenant l'intégralité de la séquence du gène codant pour l'un des polypeptides de la revendication 1.

14. Sonde ou amorce nucléotidique choisie parmi les oligonucléotides suivants ou leurs complémentaires :
SEQ ID n° 20 : GCG AGC TGC CCT CGG AG
SEQ ID n° 21 : GGT TCT GCA GGT GAC TCA G
SEQ ID n° 22 : GCC ATG CCT GTC TAC AAG
SEQ ID n° 23 **:** ACC AGC TGG TTG ACG GAG
SEQ ID n° 24 : GTC AAC CAG CTG GTG GGC CAG
SEQ ID n° 25 : GTG GAT CTC GGC CTC C
SEQ ID n° 26 : AGG CCG GCG TGG GGA AG
SEQ ID n° 27 : CTT GGC GAT CTG GCA GTA G
SEQ ID n° 28 : GCG GCC ACG ACC GTG AC
SEQ ID n° 29 : GGC AGC TTG GGT CTC TGG
SEQ ID n° 30 : CTG TAC GTC GGT GAC CCC
SEQ ID n° 31 : TCA GTG GAT CTC GGC CTC
SEQ ID n° 32 : AGG GGA CGC AGC GAA ACC
SEQ ID n° 33 : CCA TCA GCT CCA GGC TCT C
SEQ ID n° 34 : CCA GGA CAG GCG CAG ATG
SEQ ID n° 35 : GAT GAG GTG GCT GGC TGG A
SEQ ID n° 36 : TGG TCA GGT TCT GCA GGT G
SEQ ID n° 37 : CAC CTA CTC CAG GGA TGC
SEQ ID n° 38 : AGG AAA ATA GAA GCG TCA GTC
SEQ ID n° 39 : CAG GCC CAC TTG CCT GCC
et SEQ ID n° 40 : CTG TCC CCA AGC TGA TGA G

15. Utilisation d'une séquence selon l'une quelconque des revendications 6 à 8 pour la fabrication d'amorces oligonucléotidiques pour des réactions de séquençage ou d'amplification spécifique selon la technique de PCR ou toute variante de celle-ci.

16. Couple d'amorces nucléotidiques, **caractérisé en ce qu'**il comprend les amorces choisies parmi les séquences suivantes :
a) amorce sens : GCG AGC TGC CCT CGG AG (SEQ ID n° 20)
amorce antisens : GGT TCT GCA GGT GAC TCA G (SEQ ID n° 21)
b) amorce sens: GCC ATG CCT GTC TAC AAG (SEQ ID n°22)
amorce antisens : ACC AGC TGG TTG ACG GAG (SEQ ID n° 23)
c) amorce sens : GTC AAC CAG CTG GTG GGC CAG (SEQ ID n° 24) amorce antisens : GTG GAT CTC GGC CTC C (SEQ ID n° 25)
d) amorce sens : AGG CCG GCG TGG GGA AG (SEQ ID n° 26)
amorce antisens : CTT GGC GAT CTG GCA GTA G (SEQ ID n° 27)
e) amorce sens : GCG GCC ACG ACC GTG A (SEQ ID n° 28)
amorce antisens : GGC AGC TTG GGT CTC TGG (SEQ ID n°29)
f) amorce sens : CTG TAC GTC GGT GAC CCC (SEQ ID n°30)
amorce antisens : TCA GTG GAT CTC GGC CTC (SEQ ID n° 31)
g) amorce sens : AGG GGA CGC AGC GAA ACC (SEQ ID n° 32)
amorce antisens : GGC AGC TTG GGT CTC TGG (SEQ ID n° 29)
h) amorce sens : CCCCCCCCCCCCCCN (où N est égal à G, A ou T)
amorce antisens : CCA TCA GCT CCA GGC TCT C (SEQ ID n° 33)
i) amorce sens : CCCCCCCCCCCCCCN (où N est égal à G, A ou T)
amorce antisens : CCA GGA CAG GCG CAG ATG (SEQ ID n° 34)
j) amorce sens : CCCCCCCCCCCCCCCN (où N est égal à G, A ou T)
amorce antisens : CTT GGC GAT CTG GCA GTA G (SEQ ID n° 27)
k) amorce sens : CAC CTA CTC CAG GGA TGC (SEQ ID n° 37)
amorce antisens : AGG AAA ATA GAA GCG TCA GTC (SEQ ID n° 38)
et l) amorce sens : CAG GCC CAC TTG CCT GCC (SEQ ID n° 39)
amorce antisens : CTG TCC CCA AGC TGA TGA G (SEQ ID n° 40).

17. Utilisation d'amorces nucléotidiques selon l'une quelconque des revendications 6 à 8 pour le séquençage.

18. Utilisation d'une sonde ou amorce selon l'une quelconque des revendications 13 à 14, comme outil de diagnostic *in vitro* pour la détection, par des expériences d'hybridation, des séquences d'acides nucléiques codant pour un polypeptide selon l'une quelconque des revendications 1 à 4, dans des échantillons biologiques, ou pour la mise en évidence de synthèses aberrantes ou d'anomalies génétiques.

19. Méthode de diagnostic *in vitro* pour la détection de synthèses aberrantes ou d'anomalies génétiques au niveau des séquences d'acides nucléiques codant pour un polypeptide selon l'une quelconque des revendications 1 à 4, **caractérisée en ce qu'**elle comprend :
- la mise en contact d'une sonde nucléotidique selon l'une quelconque des revendications 13 à 14 avec un échantillon biologique dans des conditions permettant la formation d'un complexe d'hybridation entre ladite sonde et la susdite séquence nucléotidique, éventuellement après une étape préalable d'amplification de la susdite séquence nucléotidique ;
- la détection du complexe d'hybridation éventuellement formé ;
- éventuellement le séquençage de la séquence nucléotidique formant le complexe d'hybridation avec la sonde de l'invention.

20. Utilisation d'une séquence d'acides nucléiques selon l'une quelconque des revendications 6 à 8, pour la production d'un polypeptide recombinant selon l'une quelconque des revendications 1 à 5.

21. Méthode de production d'une protéine recombinante SR-p70, **caractérisée en ce que** l'on cultive des cellules transfectées selon la revendication 10 ou 11 dans des conditions permettant l'expression d'un polypeptide recombinant de séquence SEQ ID n° 2, SEQ ID n° 4, SEQ ID n°6, SEQ ID n° 8, SEQ ID n° 10, SEQ ID n°13, SEQ ID n° 15, SEQ ID n° 17 ou SEQ ID n° 19, et que l'on récupère ledit polypeptide recombinant.

22. Anticorps mono ou polyclonaux ou leurs fragments, anticorps chimériques ou immunoconjugués, **caractérisés en ce qu'**ils sont capables de reconnaître spécifiquement un polypeptide ayant une séquence choisie parmi SEQ ID n° 2, SEQ ID n° 4, SEQ ID n° 6, SEQ ID n° 8, SEQ ID n° 10, SEQ ID n° 13, SEQ ID n° 15, SEQ ID n° 17 et SEQ ID n° 19.

23. Utilisation des anticorps selon la revendication 22, pour la purification ou la détection d'un polypeptide selon l'une quelconque des revendications 1 à 4 dans un échantillon biologique.

24. Procédé de diagnostic *in vitro* de pathologies corrélées à une expression ou une accumulation anormale de protéines SR-p70 comprenant une séquence d'acides aminés choisie parmi les séquences SEQ ID n° 2, SEQ ID n° 4, SEQ ID n°6, SEQ ID n° 8, SEQ ID n° 10, SEQ ID n°13, SEQ ID n° 15, SEQ ID n° 17 ou SEQ ID n° 19, notamment les phénomènes de cancérisation, à partir d'un prélèvement biologique, **caractérisé en ce que** l'on met en contact au moins un anticorps selon la revendication 22 avec ledit prélèvement biologique, dans des conditions permettant la formation éventuelle de complexes immunologiques spécifiques entre une protéine SR-p70 et le ou lesdits anticorps et **en ce que** l'on détecte les complexes immunologiques spécifiques éventuellement formés.

25. Kit pour le diagnostic *in vitro* d'une expression ou une accumulation anormale de protéines SR-p70 comprenant une séquence d'acides aminés choisie parmi les séquences SEQ ID n° 2, SEQ ID n° 4, SEQ ID n°6, SEQ ID n° 8, SEQ ID n° 10, SEQ ID n°13, SEQ ID n° 15, SEQ ID n° 17 ou SEQ ID n° 19 dans un prélèvement biologique et/ou pour la mesure du taux d'expression de celles-ci dans ledit prélèvement comprenant :
- au moins un anticorps selon la revendication 22, éventuellement fixé sur un support,
- des moyens de révélation de la formation de complexes antigènes/anticorps spécifiques entre une protéine SR-p70 et ledit anticorps et/ou des moyens de quantification de ces complexes.

26. Méthode pour le diagnostic précoce de la formation des tumeurs **caractérisée en ce que** l'on met en évidence dans un échantillon de sérum prélevé chez un individu des auto-anticorps dirigés contre une protéine SR-p70 comprenant une séquence d'acides aminés choisie parmi les séquences SEQ ID n° 2, SEQ ID n° 4, SEQ ID n°6, SEQ ID n° 8, SEQ ID n° 10, SEQ ID n°13, SEQ ID n° 15, SEQ ID n° 17 ou SEQ ID n° 19, selon les étapes consistant à mettre en contact un échantillon de sérum prélevé chez un individu avec un polypeptide de l'invention, éventuellement fixé sur un support, dans des conditions permettant la formation de complexes immunologiques spécifiques entre ledit polypeptide et les auto-anticorps éventuellement présents dans l'échantillon de sérum, et **en ce que** l'on détecte les complexes immunologiques spécifiques éventuellement formés.

27. Méthode *in vitro* de détermination d'une variabilité allélique, d'une mutation, d'une délétion, d'une insertion, d'une perte d'hétérozygotie ou d'une anomalie génétique du gène codant une protéine SR-p70 comprenant une séquence d'acides aminés choisie parmi les séquences SEQ ID n° 2, SEQ ID n° 4, SEQ ID n°6, SEQ ID n° 8, SEQ ID n° 10, SEQ ID n°13, SEQ ID n° 15, SEQ ID n° 17 ou SEQ ID n° 19, **caractérisée en ce qu'**elle utilise au moins une séquence nucléotidique selon l'une quelconque des revendications 6 à 8.

28. Méthode de détermination d'une variabilité allélique du gène codant une protéine SR-p70 comprenant une séquence d'acides aminés choisie parmi les séquences SEQ ID n° 2, SEQ ID n° 4, SEQ ID n°6, SEQ ID n° 8, SEQ ID n° 10, SEQ ID n°13, SEQ ID n° 15, SEQ ID n° 17 ou SEQ ID n° 19, ladite variabilité allélique étant située au niveau de la position -30 et -20 par rapport à l'ATG d'initiation de l'exon 2 pouvant être impliquée dans des pathologies et
**caractérisée en ce qu'**elle comprend au moins:
- une étape au cours de laquelle on procède à l'amplification par PCR de l'exon 2 du gène codant une protéine SR-p70 portant la séquence cible à l'aide de couple d'amorces oligonucléotidiques sélectionné dans le groupe constitué de SEQ ID n°32 et SEQ ID n°29, SEQ ID n°37 et SEQ ID n°38, et SEQ ID n°39 et SEQ ID n°40;
- une étape au cours de laquelle on procède au traitement des produits amplifiés par un enzyme de restriction dont le site de coupure correspond à l'allèle recherché;
- une étape au cours de laquelle on procède à la détection ou au dosage d'au moins l'un des produits de la réaction enzymatique.

29. Composition pharmaceutique comprenant, à titre de principe actif, un polypeptide selon l'une quelconque des revendications 1 à 4.

30. Composition pharmaceutique selon la revendication précédente, **caractérisée en ce qu'**elle comprend un polypeptide selon la revendication 2.

## Claims

1. Purified polypeptide comprising an amino acid sequence chosen from:
a) sequence SEQ ID No. 2;
b) sequence SEQ ID No. 4;
c) sequence SEQ ID No. 6;
d) sequence SEQ ID No. 8;
e) sequence SEQ ID No. 10;
f) sequence SEQ ID No. 13;
g) sequence SEQ ID No. 15;
h) sequence SEQ ID No. 17;
i) sequence SEQ ID No. 19.

2. Polypeptide according to claim 1, **characterised in that** it comprises the amino acid sequence chosen from SEQ ID No. 6, SEQ ID No. 13, SEQ ID No. 15, SEQ ID No. 17 and SEQ ID No. 19.

3. Polypeptide according to claim 1, **characterised in that** it comprises the sequence contained between:
• residue 110 and residue 310 of SEQ ID No. 2 or 6;
• residue 60 and residue 260 of SEQ ID No. 8.

4. Polypeptide according to claim 1, **characterised in that** it results from an alternate splicing of the messenger RNA of the corresponding gene.

5. Polypeptide according to any one of the preceding claims, **characterised in that** it is a recombinant polypeptide produced in the form of a fusion protein.

6. Isolated nucleic acid sequence coding for a polypeptide according to any one of the preceding claims.

7. Isolated nucleic acid sequence according to claim 6, **characterised in that** it is chosen from:
a) sequence SEQ ID No. 1;
b) sequence SEQ ID No. 3;
c) sequence SEQ ID No. 5;
d) sequence SEQ ID No. 7;
e) sequence SEQ ID No. 9;
f) sequence SEQ ID No. 11;
g) sequence SEQ ID No. 12;
h) sequence SEQ ID No. 14;
i) sequence SEQ ID No. 16;
j) sequence SEQ ID No. 18.

8. Nucleotide sequence according to claim 6, **characterised in that** it is a sequence chosen from SEQ ID No. 5, SEQ ID No. 12, SEQ ID NO. 14, SEQ ID No. 16 and SEQ ID No. 18 respectively coding for the polypeptide of sequences SEQ ID No. 6, SEQ ID No. 13, SEQ ID NO. 15, SEQ ID No. 17 and SEQ ID No. 19.

9. Cloning and/or expression vector containing a nucleic acid sequence according to any one of claims 6 to 8.

10. Vector according to claim 9, **characterised in that** it is the pSE1 plasmid.

11. Host cell transfected by a vector according to claim 9 or 10.

12. Transfected host cell according to claim 11, **characterised in that** it is *E.coli* MC 1061.

13. Nucleotide probe or primer comprising the whole gene sequence coding for one of the polypeptides of claim 1.

14. Nucleotide probe or primer chosen from the following oligonucleotides or their complements:
SEQ ID No. 20: GCG AGC TGC CCT CGG AG
SEQ ID No. 21: GGT TCT GCA GGT GAC TCA G
SEQ ID No. 22: GCC ATG CCT GTC TAC AAG
SEQ ID No. 23: ACC AGC TGG TTG ACG GAG
SEQ ID No. 24: GTC AAC CAG CTG GTG GGC CAG
SEQ ID No. 25: GTG GAT CTC GGC CTC C
SEQ ID No. 26: AGG CCG GCG TGG GGA AG
SEQ ID No. 27: CTT GGC GAT CTG GCA GTA G
SEQ ID No. 28: GCG GCC ACG ACC GTG AC
SEQ ID No. 29: GGC AGC TTG GGT CTC TGG
SEQ ID No. 30: CTG TAC GTC GGT GAC CCC
SEQ ID No. 31: TCA GTG GAT CTC GGC CTC
SEQ ID No. 32: AGG GGA CGC AGC GAA ACC
SEQ ID No. 33: CCA TCA GCT CCA GGC TCT C
SEQ ID No. 34: CCA GGA CAG GCG CAG ATG
SEQ ID No. 35: GAT GAG GTG GCT GGC TGG A
SEQ ID No. 36: TGG TCA GGT TCT GCA GGT G
SEQ ID No. 37: CAC CTA CTC CAG GGA TGC
SEQ ID No. 38: AGG AAA ATA GAA GCG TCA GTC
SEQ ID No. 39: CAG GCC CAC TTG CCT GCC
and SEQ ID No. 40: CTG TCC CCA AGC TGA TGA G.

15. Use of a sequence according to any one of claims 6 to 8 for the production of oligonucleotide primers for specific sequencing or amplification reactions using the PCR technique or any variant thereof.

16. Nucleotide primer pair, **characterised in that** it comprises primers chosen from the following sequences:
a) sense primer: GCG AGC TGC CCT CGG AG (SEQ ID No. 20)
antisense primer: GGT TCT GCA GGT GAC TCA G (SEQ ID No. 21)
b) sense primer: GCC ATG CCT GTC TAC AAG (SEQ ID No. 22)
antisense primer: ACC AGC TGG TTG ACG GAG (SEQ ID No. 23)
c) sense primer: GTC AAC CAG CTG GTG GGC CAG (SEQ ID No. 24)
antisense primer: GTG GAT CTC GGC CTC C (SEQ ID No. 25)
d) sense primer: AGG CCG GCG TGG GGA AG (SEQ ID No. 26)
antisense primer: CTT GGC GAT CTG GCA GTA G (SEQ ID No. 27)
e) sense primer: GCG GCC ACG ACC GTG A (SEQ ID No. 28)
antisense primer: GGC AGC TTG GGT CTC TGG (SEQ ID No. 29)
f) sense primer: CTG TAC GTC GGT GAC CCC (SEQ ID No. 30)
antisense primer: TCA GTG GAT CTC GGC CTC (SEQ ID No. 31)
g) sense primer: AGG GGA CGC AGC GAA ACC (SEQ ID No. 32)
antisense primer: GGC AGC TTG GGT CTC TGG (SEQ ID No. 29)
h) sense primer: CCCCCCCCCCCCCCN (where N is equal to G, A or T)
antisense primer: CCA TCA GCT CCA GGC TCT C (SEQ ID No. 33)
i) sense primer: CCCCCCCCCCCCCCN (where N is equal to G, A or T)
antisense primer: CCA GGA CAG GCG CAG ATG (SEQ ID No. 34)
j) sense primer: CCCCCCCCCCCCCCN (where N is equal to G, A or T)
antisense primer: CTT GGC GAT CTG GCA GTA G (SEQ ID No. 27)
k) sense primer: CAC CTA CTC CAG GGA TGC (SEQ ID No. 37)
antisense primer: AGG AAA ATA GAA GCG TCA GTC (SEQ ID No. 38)
and 1) sense primer: CAG GCC CAC TTG CCT GCC (SEQ ID No. 39)
antisense primer: CTG TCC CCA AGC TGA TGA G (SEQ ID No. 40).

17. Use of nucleotide primers according to any one of claims 6 to 8 for sequencing.

18. Use of a probe or primer according to any one of claims 13 to 14 as *in vitro* diagnostic tool for the detection, by hybridisation experiments, of nucleic acid sequences coding for a polypeptide according to any one of claims 1 to 4 in biological samples, or for revealing aberrant syntheses or genetic anomalies.

19. *In vitro* diagnostic method for detecting aberrant syntheses or genetic anomalies at the level of nucleic acid sequences coding for a polypeptide according to any one of claims 1 to 4, **characterised in that** it comprises:
• placing a nucleotide probe according to any one of claims 13 to 14 in contact with a biological sample in conditions allowing the formation of a hybridisation complex between said probe and the abovementioned nucleotide sequence, if necessary after an initial step of amplification of the abovementioned nucleotide sequence;
• detecting any hybridisation complex formed;
• if necessary, sequencing the nucleotide sequence forming the hybridisation complex with the probe of the invention.

20. Use of a nucleic acid sequence according to any one of claims 6 to 8 for the production of a recombinant polypeptide according to any one of claims 1 to 5.

21. Method of producing an SR-p70 recombinant protein, **characterised in that** transfected cells according to claim 10 or 11 are cultivated in conditions allowing the expression of a recombinant polypeptide of sequence SEQ ID No. 2, SEQ ID No. 4, SEQ ID No. 6, SEQ ID No. 8, SEQ ID No. 10, SEQ ID No. 13, SEQ ID No. 15, SEQ ID No. 17 or SEQ ID No. 19, and that said recombinant polypeptide is recovered.

22. Mono- or polyclonal antibodies or fragments thereof, chimeric or immunoconjugate antibodies, **characterised in that** they are capable of specifically recognising a polypeptide having a sequence chosen from SEQ ID No. 2, SEQ ID No. 4, SEQ ID No. 6, SEQ ID No. 8, SEQ ID No. 10, SEQ ID No. 13, SEQ ID No. 15, SEQ ID No. 17 and SEQ ID No. 19.

23. Use of the antibodies according to claim 22 for purifying or detecting a polypeptide according to any one of claims 1 to 4 in a biological sample.

24. Process for *in vitro* diagnosis of pathologies closely related to an expression or an abnormal accumulation of SR-p70 proteins comprising an amino acid sequence chosen from sequences SEQ ID No. 2, SEQ ID No. 4, SEQ ID No. 6, SEQ ID No. 8, SEQ ID No. 10, SEQ ID No. 13, SEQ ID No. 15, SEQ ID No. 17 or SEQ ID No. 19, in particular canceration processes, from a biological sample, **characterised in that** at least one antibody according to claim 22 is placed in contact with said biological sample in conditions allowing the possible formation of specific immunological complexes between an SR-p70 protein and said antibody or antibodies, and **in that** any specific immunological complexes formed are detected.

25. Kit for *in vitro* diagnosis of an expression or abnormal accumulation of SR-p70 proteins comprising an amino acid sequence chosen from sequences SEQ ID No. 2, SEQ ID No. 4, SEQ ID No. 6, SEQ ID No. 8, SEQ ID No. 10, SEQ ID No. 13, SEQ ID No. 15, SEQ ID No. 17 or SEQ ID No. 19 in a biological sample and/or for measuring the expression rate thereof in said sample comprising:
• at least one antibody according to claim 22, possibly fixed on a support,
• means for revealing the formation of specific antigen/antibody complexes between an SR-p70 protein and said antibody and/or means for quantifying these complexes.

26. Method for the early diagnosis of the formation of tumours, **characterised in that** autoantibodies directed against an SR-p70 protein comprising an amino acid sequence chosen from sequences SEQ ID No. 2, SEQ ID No. 4, SEQ ID No. 6, SEQ ID No. 8, SEQ ID No. 10, SEQ ID No. 13, SEQ ID No. 15, SEQ ID No. 17 or SEQ ID No. 19 are revealed in a serum sample taken from an individual, using the steps comprising placing a serum sample taken from an individual in contact with a polypeptide of the invention, possibly fixed on a support, in conditions allowing the formation of specific immunological complexes between said polypeptide and any autoantibodies present in the serum sample, and **in that** any specific immunological complexes formed are detected.

27. Method for the *in vitro* determination of an allelic variability, a mutation, a deletion, an insertion, a loss of heterozygosity or a genetic anomaly of the gene coding for an SR-p70 protein comprising an amino acid sequence chosen from sequences SEQ ID No. 2, SEQ ID No. 4, SEQ ID No. 6, SEQ ID No. 8, SEQ ID No. 10, SEQ ID No. 13, SEQ ID No. 15, SEQ ID No. 17 or SEQ ID No. 19, **characterised in that** it uses at least one nucleotide sequence according to any one of claims 6 to 8.

28. Method for determining an allelic variability of the gene coding for an SR-p70 protein comprising an amino acid sequence chosen from sequences SEQ ID No. 2, SEQ ID No. 4, SEQ ID No. 6, SEQ ID No. 8, SEQ ID No. 10, SEQ ID No. 13, SEQ ID No. 15, SEQ ID No. 17 or SEQ ID No. 19, wherein said allelic variability is located at the level of position -30 and -20 in relation to the initiator ATG of exon 2 that can be indicated in pathologies and **characterised in that** it comprises at least:
• a step during which exon 2 of the gene coding for an SR-670 protein bearing the target sequence is amplified by PCR by means of a selected pair of oligonucleotide primers in the group formed by SEQ ID NO. 32 and SEQ ID No. 29, SEQ ID No. 37 and SEQ ID No. 38 and SEQ ID No. 39 and SEQ ID No. 40;
• a step during which the products amplified by a restriction enzyme, the cutting site of which corresponds to the sought allele, are treated;
• a step during which the detection or dosing of at least one of the products of the enzymatic reaction is conducted.

29. Pharmaceutical composition comprising a polypeptide according to any one of claims 1 to 4 as active substance.

30. Pharmaceutical composition according to the preceding claim, **characterised in that** it comprises a polypeptide according to claim 2.

## Patentansprüche

1. Gereinigtes Polypeptid, umfassend eine Aminosäuren - Sequenz, ausgewählt aus :
a) die Sequenz SEQ ID NO. 2 ;
b) die Sequenz SEQ ID NO. 4 ;
c) die Sequenz SEQ ID NO. 6 ;
d) die Sequenz SEQ ID NO. 8 ;
e) die Sequenz SEQ ID NO. 10 ;
f) die Sequenz SEQ ID NO. 13 ;
g) die Sequenz SEQ ID NO. 15 ;
h) die Sequenz SEQ ID NO. 17 ;
i) die Sequenz SEQ ID NO. 19 .

2. Polypeptid nach Patentanspruch 1, **dadurch gekennzeichnet, dass** es die Aminosäuren - Sequenz umfasst, die ausgewählt ist aus der: Sequenz SEQ ID NO. 6 , Sequenz SEQ ID NO. 13 , Sequenz SEQ ID NO. 15 , Sequenz SEQ ID NO. 17 und der Sequenz SEQ ID NO. 19.

3. Polypeptid nach Patentanspruch 1, **dadurch gekennzeichnet, dass** es die Sequenz umfasst zwischen :
- dem Rest 110 und dem Rest 310 der SEQU ID NO. 2 oder 6 ;
- den Rest 60 der SEQU ID NO. 8.

4. Polypeptid nach Patentanspruch 1 , **dadurch gekennzeichnet, dass** es sich ergibt aus einem alternativen Splissen des Messenger - RNAs des entsprechenden Gens.

5. Polypeptid nach irgendeinem der vorangehenden Patentansprüche, **dadurch gekennzeichnet, dass** es sich um ein rekombinantes Polypeptid handelt, das in der Form eines Fusions - Proteins produziert wird.

6. Isolierte Nukleinsäuren - Sequenz , die ein Polypeptid nach irgendeinem der vorangehenden Patentansprüche kodiert.

7. Isolierte Nukleinsäuren - Sequenz nach Patentanspruch 6, **dadurch gekennzeichnet, dass** sie ausgewählt wird aus :
a) die Sequenz SEQ ID NO. 1 ;
b) die Sequenz SEQ ID NO. 3 ;
c) die Sequenz SEQ ID NO. 5 ;
d) die Sequenz SEQ ID NO. 7 ;
e) die Sequenz SEQ ID NO. 9 ;
f) die Sequenz SEQ ID NO. 11 ;
g) die Sequenz SEQ ID NO. 12 ;
h) die Sequenz SEQ ID NO. 14 ;
i) die Sequenz SEQ ID NO. 16 ;
j) die Sequenz SEQ ID NO. 18 .

8. Nucleotid - Sequenz nach Patentanspruch 6, **dadurch gekennzeichnet, dass** es sich um eine Sequenz handelt, die ausgewählt ist aus der SEQ ID NO. 5 , SEQ ID NO. 12 , SEQ ID NO. 14 , SEQ ID NO. 16 und der SEQU ID NO. 18 , die jeweils die Polypeptid - Sequenzen SEQ ID NO.6 , SEQ ID NO. 13, SEQ ID NO.15 , SEQ ID NO. 17 und SEQ ID NO. 19 kodieren.

9. Klonierungs - und / oder Expressions - Vektor, die eine Nukleinsäure - Sequenz nach irgendeinem der Patentansprüche 6 bis 8 enthalten.

10. Vektor nach dem Patentanspruch 9, **dadurch gekennzeichnet, dass** es sich um das Plasmid pSE1 handelt.

11. Durch einen Vektor nach Patentanspruch 9 oder 10 transfektierte Gastzelle.

12. Nach dem Patentanspruch 11 transfektierte Gastzelle, **dadurch gekennzeichnet, dass** es sich um *E. Coli* MC 1061 handelt.

13. Nukleotid - Sonde oder Nukleotid - Primer umfassend die Gesamtheit der Sequenz des Gens , das eines der Polypeptide des Patentanspruchs 1 kodiert.

14. Nukleotid - Sonde oder Nukleotid - Primer, die aus den folgenden Oligonukleotiden oder ihren Komplementen ausgewählt sind:
SEQ ID NO. 20 : GCG AGC TGC CCT CGG AG
SEQ ID NO. 21 : GGT TCT GCA GGT GAC TCA G
SEQ ID NO. 22 : GCC ATG CCT GTC TAC AAG
SEQ ID NO. 23 : ACC AGC TGG TTG ACG GAG
SEQ ID NO. 24 : GTC AAC CAG CTG GTG GGC CAG
SEQ ID NO. 25 : GTG GAT CTC GGC CTC C
SEQ ID NO. 26 : AGG CCG GCG TGG GGA AG
SEQ ID NO. 27 : CTT GGC GAT CTG GCA GTA G
SEQ ID NO. 28 : GCG GCC ACG ACC GTG AC
SEQ ID NO. 29 : GGC AGC TTG GGT CTC TGG
SEQ ID NO. 30 : CTG TAC GTC GGT GAC CCC
SEQ ID NO. 31 : TCA GTG GAT CTC GGC CTC
SEQ ID NO. 32 : AGG GGA CGC AGC GAA ACC
SEQ ID NO. 33 : CCA TCA GCT CCA GGC TCT C
SEQ ID NO. 34: CCA GGA CAG GCG CAG ATG
SEQ ID NO. 35 : GAT GAG GTG GCT GGC TGG A
SEQ ID NO. 36 : TGG TCA GGT TCT GCA GGT G
SEQ ID NO. 37 : CAC CTA CTC CAG GGA TGC
SEQ ID NO. 38 : AGG AAA ATA GAA GCG TCA GTC
SEQ ID NO. 39 : CAG GCC CAC TTG CCT GCC
und SEQ ID NO. 40 : CTG TCC CCA AGC TGA TGA G

15. Verwendung einer Sequenz nach irgendeinem der Patentansprüche 6 bis 8 für die Herstellung von Oligonikleotid - Primern für spezifische Sequenzierungs oder Verstärkungs - Reaktionen nach der PCR - Technik oder jeder beliebigen Variante davon.

16. Ein Paar von Nukleotid - Primern , **dadurch gekennzeichnet, dass** es die Primer umfasst, die aus den folgenden Sequenzen ausgewählt werden:
a ) Sense - Primer: GCG AGC TGC CCT CGG AG ( SEQ ID NO. 20 )
Antisense - Primer: GGT TCT GCA GGT GAC TCA G ( SEQ ID NO. 21 )
b ) Sense - Primer: GCC ATG CCT GTC TAC AAG ( SEQ ID NO. 22 )
Antisense - Primer: ACC AGC TGG TTG ACG GAG ( SEQ ID NO. 23 )
c) Sense - Primer: GTC AAC CAG CTG GTG GGC CAG ( SEQ ID NO. 24 )
Antisense - Primer: GTG GAT CTC GGC CTC C ( SEQ ID NO. 25 )
d ) Sense - Primer: AGG CCG GCG TGG GGA AG ( SEQ ID NO. 26 )
Antisense - Primer: CTT GGC GAT CTG GCA GTA G ( SEQ ID NO. 27 )
e ) Sense - Primer: GCG GCC ACG ACC GTG AC ( SEQ ID NO. 28 )
Antisense - Primer: GCG GCC ACG ACC GTG AC ( SEQ ID NO. 29 )
f ) Sense - Primer: CTG TAC GTC GGT GAC CCC ( SEQ ID NO. 30)
Antisense - Primer: TCA GTG GAT CTC GGC CTC ( SEQ ID NO. 31 )
g ) Sense - Primer: AGG GGA CGC AGC GAA ACC ( SEQ ID NO. 32 )
Antisense - Primer: GGC AGC TTG GGT CTC TGG ( SEQ ID NO. 29 )
h ) Sense - Primer: CCCCCCCCCCCCCCN ( wo N gleich G , A oder T ist)
Antisense - Primer: CCA TCA GCT CCA GGC TCT C ( SEQ ID NO. 33 )
i ) Sense - Primer: CCCCCCCCCCCCCCN ( wo N gleich G , A oder T ist)
Antisense - Primer: CCA GGA CAG GCG CAG ATG ( SEQ ID NO. 34 )
j ) Sense - Primer: CCCCCCCCCCCCCCN ( wo N gleich G , A oder T ist)
Antisense - Primer: CTT GGC GAT CTG GCA GTA G ( SEQ ID NO. 27 )
k) Sense - Primer: CAC CTA CTC CAG GGA TGC ( SEQ ID NO. 37 )
Antisense - Primer: AGG AAA ATA GAA GCG TCA GTC ( SEQ ID NO. 38 )
und
l ) Sense - Primer: CAG GCC CAC TTG CCT GCC ( SEQ ID NO. 39 )
Antisense - Primer: CTG TCC CCA AGC TGA TGA G ( SEQ ID NO. 40 ) .

17. Verwendung von Nukleotid - Primern nach irgendeinem der Patentansprüche 6 bis 8 für die Sequenzierung.

18. Verwendung einer Sonde oder eines Primers nach irgendeinem der Patentansprüche 13 bis 14 , als *in vitro -* Diagnose - Mittel für die Detektion , mit Hilfe von Hybridierungs - Versuchen , von Nukleinsäure - Sequenzen , die für ein Polypeptid nach irgendeinem der Patentansprüche 1 bis 4 kodieren , in biologischen Proben oder für die Feststellung von fehlerhaften Synthesen oder genetischen Anomalien.

19. In vitro - Diagnose - Verfahren für die Detektion von fehlerhaften Synthesen oder genetischen Anomalien bei Nukleinsäure - Sequenzen , die für ein Polypeptid nach irgendeinem der Patentansprüche 1 bis 4 kodieren, **dadurch gekennzeichnet, dass** das Verfahren folgendes umfasst:
- das In - Kontakt - Bringen einer Nukleotid - Sonde nach irgendeinem der Patentansprüche 13 bis 14, mit einer biologischen Probe unter Bedingungen , die die Bildung eines Hybridierungs - Komplexes zwischen dieser Sonde und der oben genannten Nukleotid - Sequenz , eventuell nach einer vorangehenden Verstärkungs - Etappe der oben genannten Nukleotid - Sequenz erlauben ;
- die Detektion des eventuell gebildeten Hybridierungs - Komplexes ;
- eventuell die Sequenzierung der Nukleotid - Sequenz , die den Hybridierungs - Komplex mit der Sonde der Erfindung bildet.

20. Verwendung einer Nukleinsäure - Sequenz nach irgendeinem der Patentansprüche 6 bis 8 , für die Produktion eines rekombinanten Polypeptids nach irgendeinem der Patentansprüche 1 bis 5.

21. Produktions - Verfahren eine rekombinanten Proteins SR - p 70 , **dadurch gekennzeichnet, dass** man transfektierte Zellen nach dem Patentanspruch 10 oder 11 unter Bedingungen züchtet, die die Exprimierung eines rekombinanten Polypeptids folgender Sequenzen ermöglicht: SEQ ID NO. 2 , SEQ ID NO. 4 , SEQ ID NO. 6 , SEQ ID NO. 8 , SEQ ID NO.10 , SEQ ID NO. 13 , SEQ ID NO. 15 , SEQ ID NO. 17 oder SEQ ID NO. 19 und dass man dieses rekombinante Polypeptid zurückgewinnt.

22. Monoklonale oder polyklonale Antikörper oder ihre Fragmente , chimerische oder immunokonjugierte Antikörper, **dadurch gekennzeichnet , dass** sie in der Lage sind , spezifisch ein Polypeptid zu erkennen , das eine Sequenz aufweist , die ausgewählt wird aus : SEQ ID NO. 2 , SEQ ID NO. 4 , SEQ ID NO. 6 , SEQ ID NO. 8 , SEQ ID NO.10 , SEQ ID NO. 13 , SEQ ID NO. 15, SEQ ID NO. 17 oder SEQ ID NO. 19.

23. Verwendung von Antikörpern nach Patenanspruch 22 , für die Reinigung oder Detektion eines Polypeptids nach irgendeinem der Patentansprüche 1 bis 4 in einer biologischen Probe.

24. In - Vitro - Diagnose - Verfahren von Pathologien , die mit einer anormalen Exprimierung oder einer Akkumulierung des Proteins SR - p 70 korreliert sind umfassend eine Aminosäurensequenz , die ausgewählt wird aus den Sequenzen SEQ ID NO. 2 , SEQ ID NO. 4 , SEQ ID NO. 6 , SEQ ID NO. 8 , SEQ ID NO.10 , SEQ ID NO. 13 , SEQ ID NO. 15, SEQ ID NO. 17 oder SEQ ID NO. 19 , insbesondere von Krebsbildungsphänomenen aus einer biologischen Probe , **dadurch gekennzeichnet , dass** man mindestens einen Antikörper nach Patentanspruch 22 unter Bedingungen mit der biologischen Probe in Kontakt bringt, die die eventuelle Bildung von spezifischen immunologischen Komplexen zwischen einem Protein SR - p70 und dem oder den Antikörper (n) erlauben und dass man die eventuell gebildeten spezifischen immunologischen Komplexe detektiert.

25. Bausatz für die In - Vitro - Diagnose einer anormalen Exprimierung oder Akkumulierung der Proteine SR - p70 umfassend eine Aminosäurensequenz ausgewählt aus den Sequenzen SEQ ID NO. 2 , SEQ ID NO. 4 , SEQ ID NO. 6 , SEQ ID NO. 8 , SEQ ID NO.10 , SEQ ID NO. 13 , SEQ ID NO. 15 , SEQ ID NO. 17 oder SEQ ID NO. 19 in einer biologischen Probe und / oder für die Messung des Exprimierungs - Grades dieser Proteine in der Probe umfassend :
- mindestens einen Antikörper nach Patentanspruch 22 , der eventuell auf einem Träger fixiert ist,
- Offenbarungs - Mittel für die Bildung von spezifischen Antigen - / Antikörper - Komplexen zwischen einem Protein SR - p70 und dem Antikörper und / oder den Quantifizierungs - Mitteln dieser Komplexe.

26. Verfahren für die vorzeitige Diagnose der Bildung von Tumoren , **dadurch gekennzeichnet, dass** man in einer einem Individuum entnommenen Serums - Probe Antikörper offenbart , die gegen ein Protein SR - p 70 gerichtet sind , umfassend eine Sequenz von Aminosäuren die ausgewählt sind aus den Sequenzen SEQ ID NO. 2 , SEQ ID NO. 4 , SEQ ID NO. 6 , SEQ ID NO. 8 , SEQ ID NO.10 , SEQ ID NO. 13 , SEQ ID NO. 15, SEQ ID NO. 17 oder SEQ ID NO. 19 , nach den Schritten , die darin bestehen , die bei einem Individuum entnommene Serums - Probe mit einem , eventuell auf einem Träger fixierten Polypeptid der Erfindung in Kontakt unter Bedingungen zu bringen , die die Bildung von spezifischen immunologischen Komplexen zwischen dem Polypeptid und den eventuell in der Serums - Probe vorliegenden Auto - Antikörpern erlauben und dass man die eventuell gebildeten spezifischen immunologischen Komplexe detektiert.

27. In - Vitro - Verfahren zur Bestimmung einer allelischen Variabilität , einer Mutation , einer Deletion , einer Insertion , eines Verlusts der Heterozygotie oder einer genetischen Anomalie des Gens , das ein Protein SR - p70 kodiert, das eine Aminosäuresequenz umfasst, die ausgewählt ist aus den Sequenzen SEQ ID NO. 2 , SEQ ID NO. 4 , SEQ ID NO. 6 , SEQ ID NO. 8 , SEQ ID NO.10 , SEQ ID NO. 13 , SEQ ID NO. 15 , SEQ ID NO. 17 oder SEQ ID NO. 19, **dadurch gekennzeichnet, dass** es mindestens eine Nukleotid - Sequenz nach irgendeinem der Patentansprüche 6 bis 8 verwendet.

28. Verfahren zur Bestimmung einer allelischen Variabilität des Gens , das ein Protein SR - p70 kodiert, das eine Aminosäuresequenz umfasst , die ausgewählt ist aus den Sequenzen SEQ ID NO. 2 , SEQ ID NO. 4 , SEQ ID NO. 6 , SEQ ID NO. 8 , SEQ ID NO.10 , SEQ ID NO. 13 , SEQ ID NO. 15 , SEQ ID NO. 17 oder SEQ ID NO. 19, wobei diese allelische Variabilität auf dem Niveau der Position - 30 bis - 20 in Bezug auf das Initiierungs- ATG des Exons 2 , das in die Pathologien verwickelt sein kann , liegt und **dadurch gekennzeichnet ist, dass** das Verfahren mindestens folgendes umfasst:
- einen Schritt, während dem man ein Verstärkung durch PCR des Exons 2 des Gens vornimmt, das ein Protein SR - p70 kodiert, das die Ziel - Sequenz mit Hilfe des Paares aus Oligonukleotid - Primern trägt, das aus der Gruppe ausgewählt ist, die aus der SEQ ID NO. 32 und der SEQ ID NO. 29 , der SEQ ID NO. 37 und der SEQ ID NO. 38 und der SEQ ID NO. 39 und der SEQ ID NO. 40 besteht,
- einen Schritt, während dem man die Verarbeitung von Produkten vornimmt, die durch ein Restriktions - Enzym verstärkt worden sind , deren Schnitt - Stelle dem gesuchten Allel entspricht;
- einen Schritt, während dem man die Detektion oder die Dosierung mindestens eines Produkts der Enzym - Reaktion vornimmt.

29. Pharmazeutische Zusammensetzung umfassend als aktives Prinzip ein Polypeptid nach irgendeinem der Patentansprüche 1 bis 4.

30. Pharmazeutische Zusammensetzung nach irgendeinem der vorangehenden Ansprüche **, dadurch gekennzeichnet, dass** sie ein Polypeptid nach Patentanspruch 2 umfasst.
